# EUROPEAN PATENT APPLICATION

(11) **EP 4 563 575 A1**
(43) Date of publication of application: **04.06.2025**
(21) Application number: 23845732.9
(22) Date of filing: 31.07.2023
(51) Int. Cl.: C07D 401/06, C07D 401/14, C07D 491/056, A61K 31/4725, A61K 31/4741, A61K 31/404, A61P 25/28, A61P 25/16

(54) **POLYSUBSTITUTED TETRAHYDROISOQUINOLINE COMPOUNDS, METHOD FOR PREPARING SAME, PHARMACEUTICAL COMPOSITION THEREOF, AND USE THEREOF**

(30) Priority: 29.07.2022 CN 202210910074
(71) Applicant: Shanghai Institute of Materia Medica, Chinese Academy of Sciences, Shanghai 201203 (CN); Fudan University, Shanghai 200433 (CN)
(72) Inventor: LIU, Hong, Shanghai 201203 (CN); LI, Yang, Shanghai 200433 (CN); ZHOU, Yu, Shanghai 201203 (CN); YIN, Limin, Shanghai 200433 (CN); ZHOU, Jianhui, Shanghai 201203 (CN); WANG, Ning, Shanghai 200433 (CN); WANG, Mengxue, Shanghai 201203 (CN); LI, Tianyou, Shanghai 200433 (CN); JIANG, Hualiang, Shanghai 201203 (CN); CHEN, Kaixian, Shanghai 201203 (CN)
(74) Representative: dompatent von Kreisler Selting Werner - Partnerschaft von Patent- und Rechtsanwälten mbB
(86) International application number: PCT/CN2023/110145
(87) International publication number: WO 2024/022528

(57) **Abstract**

Disclosed are compounds having general formula I, a method for preparing same, a pharmaceutical composition thereof, and use thereof. Specifically, the present invention provides a compound having a structure represented by general formula I, and a racemate, an R-isomer, an S-isomer and a pharmaceutically acceptable salt thereof, or a mixture thereof. The compound has a good effect on promoting transcription factor EB (TFEB) nuclear translocation and promoting lysosome generation, and can be used for preventing, treating, or assisting in treating various diseases related to lysosome dysfunction and biosynthesis insufficiency, especially neurodegenerative diseases caused by the accumulation of intracerebral pathological proteins (e.g., β-amyloid protein and a-synuclein), such as Alzheimer's disease (AD) and Parkinson's disease (PD).

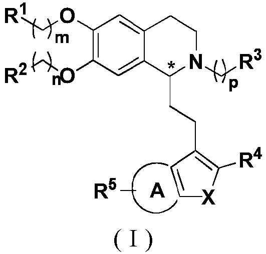

## Description

### Technical Field

The present invention discloses polysubstituted tetrahydroisoquinoline compounds, method for preparing same, pharmaceutical composition thereof, and use thereof. Relating to novel tetrahydroisoquinoline compounds represented by general formula (I), and pharmaceutically acceptable salts, isomers, solvates, metabolites, metabolic precursors thereof, pharmaceutical compositions containing them, and the use of such compounds in preventing and/or treating various diseases related to lysosomal dysfunction and biosynthesis insufficiency, especially neurodegenerative diseases caused by the accumulation of pathological proteins, for example diseases or conditions such as Alzheimer's disease and Parkinson's disease.

### Background

Alzheimer's disease (AD) is a degenerative disease of the central nervous system characterized by cognitive impairment and memory impairment, and has become one of the major diseases affecting human health. The World Alzheimer's Report released by ADI (Alzheimer's Disease International) shows that with the increase of human life expectancy, about one person develops Alzheimer's disease every 3 seconds worldwide. It is expected that by 2050, the global population of AD patients will double every 20 years to 152 million, becoming an urgent medical and social problem that needs to be solved worldwide. China has entered an aging society, with dementia patients account for 25% of global patients. The incidence rate of AD in random population is about 5.6%, which has become the fifth largest cause of death for urban and rural residents in China. The most typical pathological features of AD are senile plaques (SPs) and neurofibrillary tangles (NFTs), which are generated by abnormal accumulation of β-amyloid protein (Aβ) and hyperphosphorylated Tau protein, respectively. In addition, impairment of the autophagy lysosome pathway (ALP) has also been shown to be a major feature of AD. At present, most of the drugs used in clinical practice for AD are acetylcholinesterase (AChE) inhibitors (such as donepezil) and N-methyl-D-aspartate receptor (NMDA) antagonists (such as memantine). Although these drugs can stabilize or slow down the decline of cognitive and behavioral abilities in AD patients, they will not slow down the speed of pathological progression. At present, the development of new anti AD drugs is at a bottleneck, with the vast majority of drugs ending in failure. Although an anti AD antibody drug (aducanumab) has been approved by US FDA recently, its clinical data did not show significant improvement effect. Numerous antibody-based drugs or drugs targeting APP gamma secretase have shown poor clinical trial efficacy, thus there is an urgent need to uncover new targets and drugs for improving AD.

Parkinson's disease (PD), also known as tremor paralysis, is the second most common neurodegenerative disease characterized by static tremors, muscle rigidity, bradykinesia, and abnormal posture and gait, and its pathological features include the degeneration and loss of dopaminergic neurons in the substantia nigra of the midbrain, as well as the accumulation of inclusion (Lewy bodies) containing alpha synuclein (α-syn) in the midbrain. PD is more common in the elderly, according to statistics, the prevalence rate of PD is about 0.3% in the general population, 1% in people over 60 years old, and 3% in people over 80 years old, on average, 1-2 people are diagnosed with PD per 1000 people worldwide. It is estimated that by 2030, PD patients in China will account for half of the global population, and PD will become a "killer" threatening the health of elderly people in China. The research on new PD drugs has always been difficult, because the mechanism that produces dopamine neuronal apoptosis is not well understood. The PD drugs under research and all therapeutic drugs currently on the market only control symptoms and improve motor function, and cannot prevent the progression of the disease itself.

Lysosome is a intracellular organelle (diameter 0.2-0.8 µm) with monolayer membrane, being lipid sphere, and being rich in hydrolytic enzymes (pH 4.5-5.0) and membrane proteins. Lysosomes participate in the intracellular degradation and clearance process, not only digesting the substances entering the cell and recovering intracellular substances, regulating energy metabolism, but also degrading and clearing useless biomacromolecules in the cell, such as low-density lipoprotein, Aβ protein, tau protein, and even killing and degrading pathogens. Lysosome dysfunction and biosynthesis insufficiency are closely related to the occurrence of neurodegenerative diseases. According to research, promoting lysosome biosynthesis and activating the autophagy lysosome pathway (ALP) can clear toxic protein deposits in the mouse brain, thereby improving AD and PD like symptoms.

At present, lysosomal biosynthesis is mainly regulated by transcription factor EB (TFEB). Under normal nutritional conditions, TFEB is located in the cytoplasm; under nutritional deficiencies or stress conditions, dephosphorylated TFEB is activated and enters the nucleus, acting as a transcription factor to initiate lysosome and autophagy related gene expression, thereby promoting lysosome production and enhancing degradation function of lysosome. So far, researchers have developed many drug screening methods for identifying small molecule TFEB activators, among which TFEB-GFP nuclear translocation detection is a typical one; the principle is to construct TFEB protein labeled with GFP (green fluorescent protein), when TFEB is activated, it will be translocated into the nucleus, therefore, the localization changes of TFEB protein in the cell can be observed through fluorescence microscopy to determine whether TFEB protein is activated. Current research has found that mTOR inhibitors, diterpenoid compounds HEP14 and derivatives thereof can induce TFEB nuclear translocation and then promote lysosome production, however, these compounds have limitations, such as single structural type, difficult synthesis, and limited natural product resources. Therefore, there is still an urgent need to search for more types of small molecule compounds that can activate TFEB nuclear translocation and promote lysosome generation, which may be developed into new anti AD or PD drugs targeting new mechanisms of activating TFEB nuclear translocation and promoting lysosome generation.

### Summary of the Invention

The purpose of the present invention is to provide a class of compounds that can promote TFEB nuclear translocation and induce lysosome generation.

In the first aspect of the present invention, provided is a compound with a structure shown in formula (I), and a racemate, an R-isomer, an S-isomer, or a pharmaceutically acceptable salt thereof, 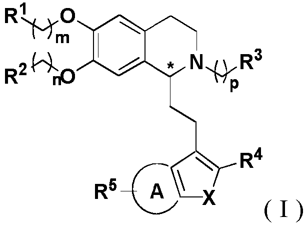 wherein,
X is selected from the group consisting of: O, S, or NR⁶;
m, n and p are each independently selected from the group consisting of: 0, 1, 2, 3, or 4;
the configuration of C* can independently be S configuration, R configuration, or racemate;
R¹ and R² are each independently selected from the group consisting of: hydrogen, deuterium, tritium, halogen, cyano, nitro, hydroxyl, sulfydryl, carboxyl, -S(O)₂OH, substituted or unsubstituted C₁-C₁₀ linear or branched alkyl, substituted or unsubstituted C₁-C₁₀ alkoxy, C₂-C₆ linear or branched alkenyl, substituted or unsubstituted C₂-C₆ alkynyl, substituted or unsubstituted saturated or partially unsaturated C₃-C₁₀ carbocyclyl, substituted or unsubstituted C₆-C₁₀ aryl, substituted or unsubstituted saturated or partially unsaturated 5-12 membered heterocyclyl, substituted or unsubstituted 5-12 membered heteroaryl, substituted or unsubstituted C₂-C₆ acyl, substituted or unsubstituted C₂-C₆ ester group, substituted or unsubstituted C₀-C₆ amino, substituted or unsubstituted C₁-C₆ amido, substituted or unsubstituted C₁-C₆ alkyl-sulfonyl and substituted or unsubstituted C₁-C₆ alkyl-sulfinyl;
or, R¹ and R² together with the atoms to which they are attached form a substituted or unsubstituted 5-12 membered heterocyclic ring or heteroaromatic ring;
R³ is selected from the group consisting of: hydrogen, substituted or unsubstituted (-C₁-C₆ alkyl - C₆-C₁₀ aryl), substituted or unsubstituted (- C₁-C₆ alkyl - 5-12 membered heteroaryl), substituted or unsubstituted (- C₁-C₆ alkyl - C₃-C₈ carbocyclyl), substituted or unsubstituted (- C₁-C₆ alkyl - 3-12 membered heterocyclyl), substituted or unsubstituted (-C₁-C₆ alkyl - 7-20 membered heteropolycyclyl), substituted or unsubstituted C₃-C₈ saturated or partially unsaturated carbocyclyl, substituted or unsubstituted C₆-C₁₀ aryl, substituted or unsubstituted 3-12 membered heterocyclyl, and substituted or unsubstituted 7-20 membered heteropolycyclyl; wherein, the heteropolycyclyl includes fused ring, bridged ring, and spiro ring structures; 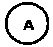
ring is selected from the group consisting of: substituted or unsubstituted 3-12 membered heterocyclyl, substituted or unsubstituted C₆-C₁₀ aryl, and substituted or unsubstituted 5-12 membered heteroaryl;
R⁴ and R⁵ are each independently selected from the group consisting of: hydrogen, deuterium, tritium, halogen, cyano, nitro, amino, C₁-C₆ amino, hydroxyl, hydroxymethyl, carboxyl, C₁-C₆ amido, sulfydryl, -S(O)₂OH, C₁-C₆ alkylsulfonyl, substituted or unsubstituted C₁-C₆ alkyl, substituted or unsubstituted C₁-C₆ alkoxy, substituted or unsubstituted C₃-C₈ cycloalkyl, substituted or unsubstituted C₆-C₁₀ aryl, and substituted or unsubstituted 3-12 membered saturated or partially unsaturated heterocyclyl;
R⁶ is selected from the group consisting of: hydrogen, C₁-C₆ alkylsulfonyl, C₁-C₆ alkyl, C₁-C₆ haloalkyl, C₁-C₆ alkyl substituted with aryl or heteroaryl, C₁-C₆ alkyl substituted with cycloalkane or heterocycloalkane, C₃-C₈ saturated or partially unsaturated carbocyclyl, C₃-C₈ halogenated saturated or partially unsaturated carbocyclyl, C₆-C₁₀ aryl, 3-12 membered heterocyclyl, and -(CH₂)_{q}YR⁷;
wherein, q is selected from the group consisting of: 0, 1, 2, 3, and 4;
Y is selected from the group consisting of: O, S, NR⁸, CO, and SO₂;
R⁷ and R⁸ are each independently selected from the group consisting of: hydrogen, hydroxyl, amino, C₁-C₆ amino, substituted or unsubstituted C₁-C₆ alkoxy, C₁-C₆ alkyl, C₁-C₆ haloalkyl, - C₁-C₆ alkyl - C₆₋₁₀ aryl, - C₁-C₆ alkyl - 5-12 membered heteroaryl, - C₁-C₆ alkyl - C₃₋₈ carbocyclyl, - C₁-C₆ alkyl - 3-12 membered heterocyclyl, C₃-C₈ saturated or partially unsaturated carbocyclyl, C₃-C₈ halogenated saturated or partially unsaturated carbocyclyl, C₆-C₁₀ aryl, 5-12 membered heteroaryl, and 3-12 membered saturated or partially unsaturated heterocyclyl;
wherein, the heteroaryl or heterocyclyl each independently contain 1 to 4 heteroatoms selected from oxygen, sulfur, and nitrogen;
the substituted in substituted or unsubstituted refers to, the group is substituted with 1 to 3 substituents selected from the group consisting of: halogen, cyano, nitro, amino, hydroxyl, hydroxymethyl, carboxyl, sulfydryl, C₁-C₆ alkyl, C₁-C₆ haloalkyl, C₁-C₆ alkoxy, C₁-C₆ alkoxycarbonyl, C₁-C₆ haloalkoxy, C₂-C₆ alkenyl, C₂-C₆ alkynyl, C₁-C₆ alkylsulfonyl, C₃-C₈ saturated or partially unsaturated carbocyclyl, C₆-C₁₀ aryl, 3-12 membered saturated or partially unsaturated heterocyclyl, and 5-12 membered heteroaryl;
the halogen is F, Cl, Br or I.

In another preferred embodiment, R⁴ and R⁵ are each independently selected from the group consisting of: hydrogen, deuterium, tritium, halogen, cyano, nitro, amino, NH₂, C₁-C₆ amino, hydroxyl, hydroxymethyl, carboxyl, C₂-C₆ amide, sulfydryl, -S(O)₂OH, C₁-C₆ alkylsulfonyl, C₁-C₆ alkyl, C₁-C₆ alkyl substituted with halogen, C₁-C₆ alkyl substituted with aryl or heteroaryl, C₁-C₆ alkyl substituted with cycloalkane or heterocycloalkane, C₁-C₆ alkoxy, C₁-C₆ alkoxy substituted with aryl or heteroaryl, C₁-C₆ haloalkoxy, C₃-C₈ cycloalkyl, C₃-C₈ halogenated cycloalkyl, C₆-C₁₀ aryl, 3-12 membered saturated or partially unsaturated heterocyclyl.

In some embodiments, R¹ and R² are independently selected from the group consisting of: substituted or unsubstituted C₁-C₁₀ linear or branched alkyl, substituted or unsubstituted saturated or partially unsaturated C₃-C₁₀ carbocyclyl, and substituted or unsubstituted C₅-C₁₀ aryl.

In another preferred embodiment, R¹ and R² are independently selected from the group consisting of: substituted or unsubstituted C₁-C₆ linear or branched alkyl, substituted or unsubstituted saturated or partially unsaturated C₃-C₆ carbocyclyl, and substituted or unsubstituted phenyl.

In some embodiments, R¹ and R² together with the atoms to which they are attached form a substituted or unsubstituted 5-12 membered heterocyclic ring.

In another preferred embodiment, R¹ and R² together with the atoms to which they are attached form a substituted or unsubstituted 5-7 membered heterocyclic ring.

In some embodiments, R³ is selected from the group consisting of: hydrogen, substituted or unsubstituted C₆-C₁₀ aryl, substituted or unsubstituted 3-12 membered heterocyclyl, or substituted or unsubstituted 7-20 membered heteropolycyclyl; wherein, the heteropolycyclyl includes fused ring, bridged ring, and spiro ring structures.

In some embodiments, 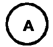 ring is selected from the group consisting of: phenyl, thiophenyl, pyrrolyl, furanyl, pyridinyl, pyrimidinyl, indolyl, or cyclohexyl.

In some embodiments, the compound is selected from the group consisting of:

| Compound Number | Structural Formula | Compound Number | Structural Formula |
|---|---|---|---|
| **A1** | 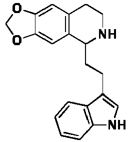 | **(*S*)-A1** | 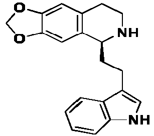 |
| **(*R*)-A1** | 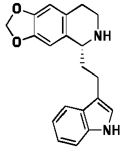 | **A2** | 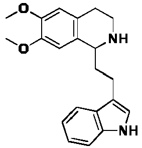 |
| **(*S*)-A2** | 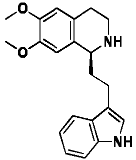 | **(*R*)-A2** | 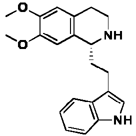 |
| **A3** | 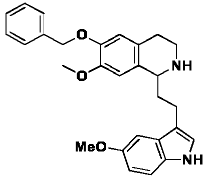 | **A4** | 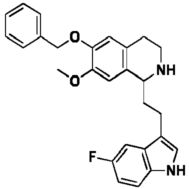 |
| **A5** | 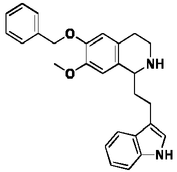 | **A12** | 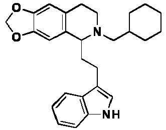 |
| **(S)-A12** | 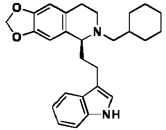 | **(*R*)-A12** | 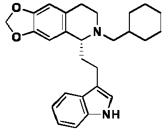 |
| **A13** | 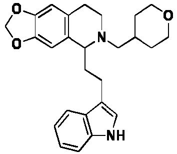 | **(*S*)-A13** | 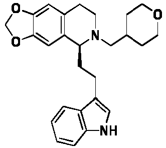 |
| **(*R*)-A13** | 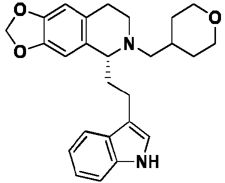 | **A14** | 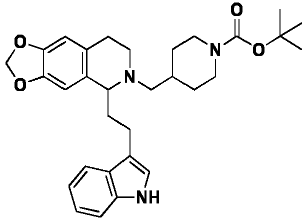 |
| **(*S*)-A14** | 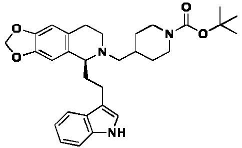 | **(*R*)-A14** | 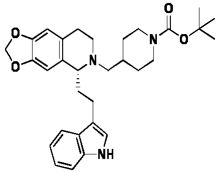 |
| **A15** | 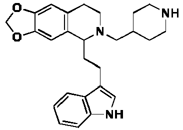 | **(*S*)-A15** | 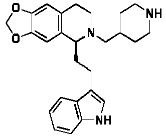 |
| **(*R*)*-*A15** | 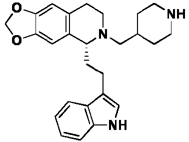 | **A16** | 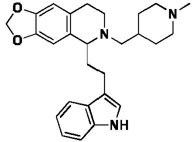 |
| **A17** | 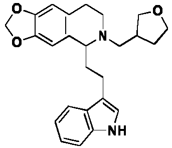 | **A18** | 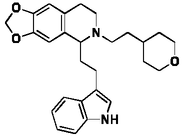 |
| **A23** | 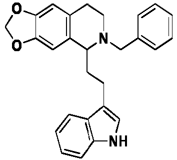 | **A24** | 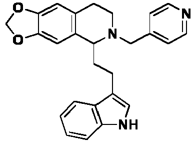 |
| **A25** | 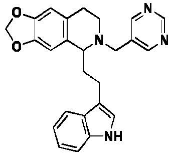 | **A26** | 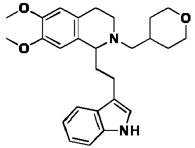 |
| **A27** | 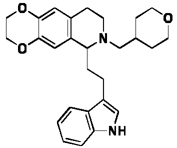 | **A28** | 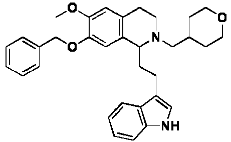 |
| **A29** | 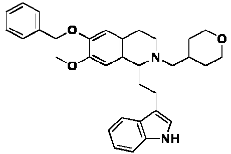 | **A30** | 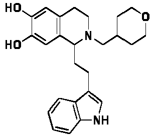 |
| **A31** | 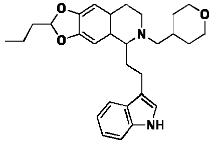 | **A32** | 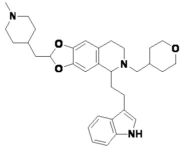 |
| **A33** | 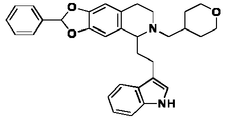 | **A34** | 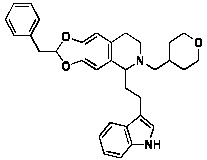 |
| **A35** | 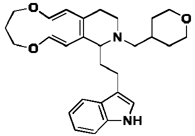 | **A36** | 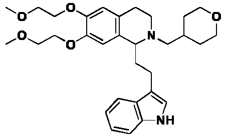 |
| **A37** | 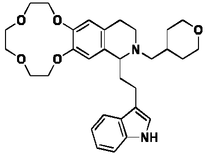 | **A38** | 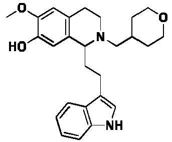 |
| **A39** | 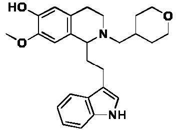 | **A40** | 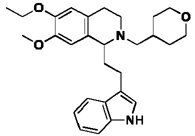 |
| **A41** | 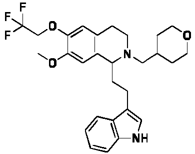 | **A42** | 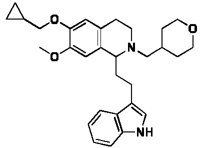 |
| **A43** | 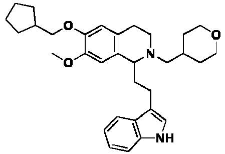 | **A44** | 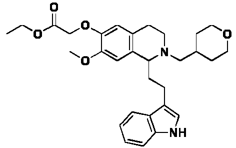 |
| **A45** | 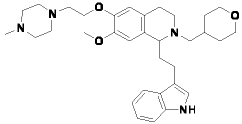 | **A46** | 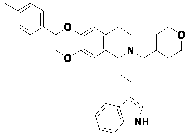 |
| **A47** | 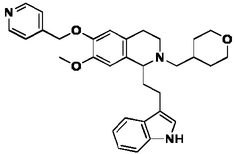 | **A48** | 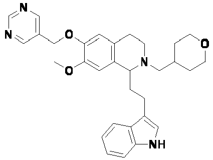 |
| **A49** | 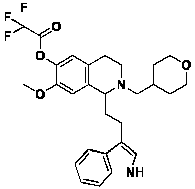 | **A50** | 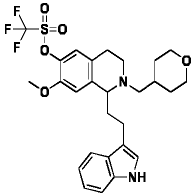 |
| **A51** | 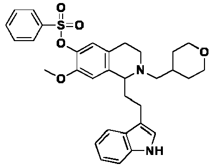 | **A52** | 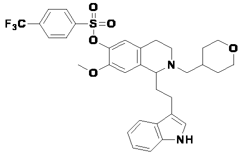 |
| **A53** | 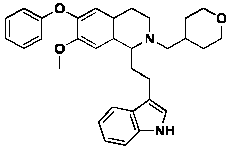 | **A54** | 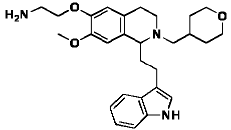 |
| **A55** | 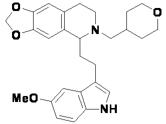 | **A56** | 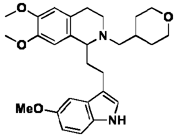 |
| **A57** | 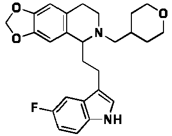 | **A58** | 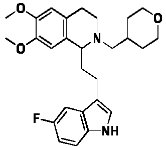 |
| **A59** | 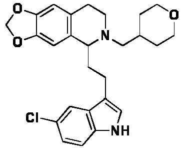 | **A60** | 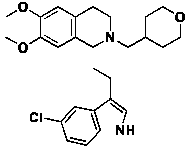 |
| **A61** | 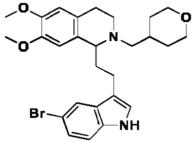 | **A62** | 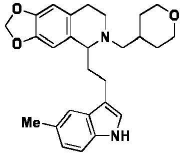 |
| **A63** | 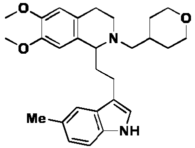 | **A64** | 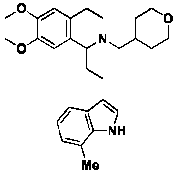 |
| **A65** | 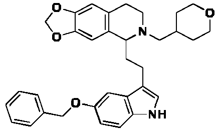 | **A66** | 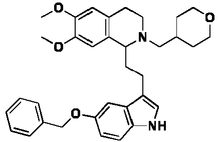 |
| **A67** | 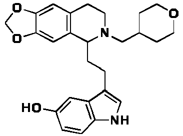 | **A68** | 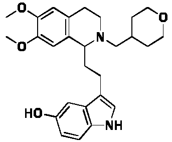 |
| **A69** | 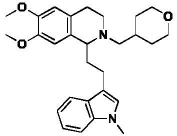 | **A70** | 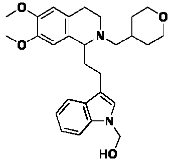 |
| **A71** | 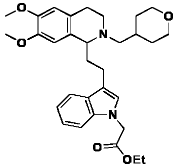 | **A72** | 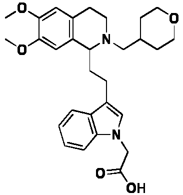 |
| **A73** | 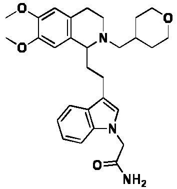 | **A74** | 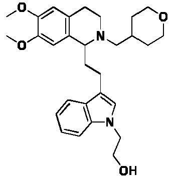 |
| **A75** | 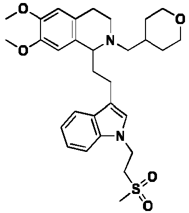 | **A76** | 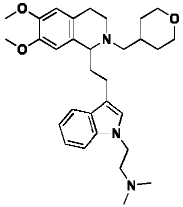 |
| **A77** | 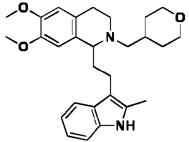 | **A78** | 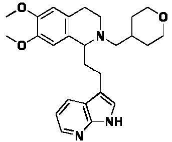 |
| **A79** | 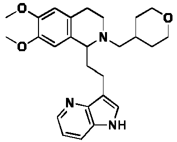 | **A80** | 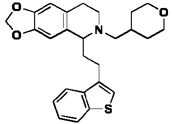 |
| **A81** | 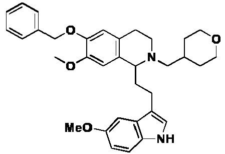 | **A82** | 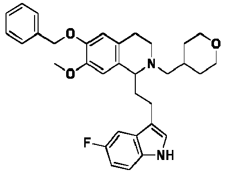 |
| **A83** | 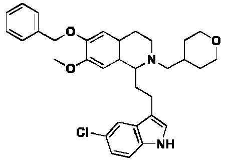 | **A84** | 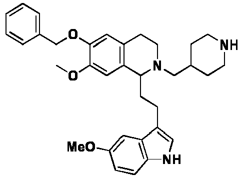 |
| **A85** | 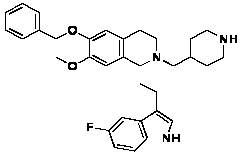 | **A86** | 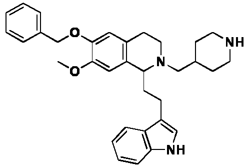 |
| **A87** | 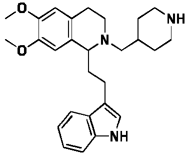 | **A88** | 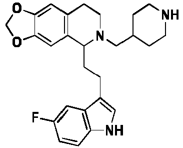 |
| **A89** | 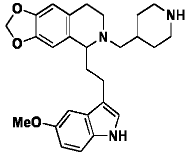 | **A90** | 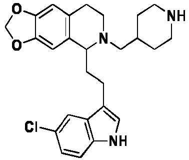 |
| **A91** | 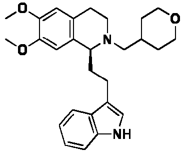 | **A92** | 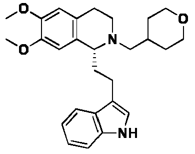 |
| **A93** | 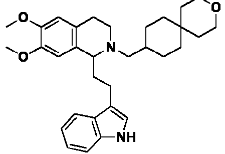 | **A94** | 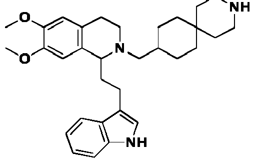 |
| **A95** | 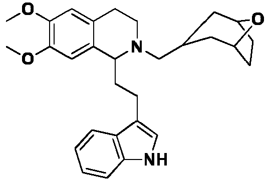 | **A96** | 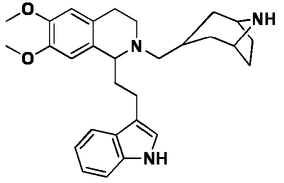 |
| **A97** | 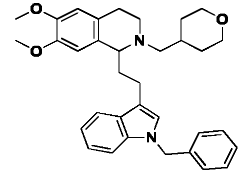 | **A98** | 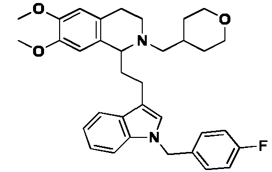 |
| **A99** | 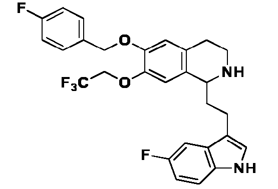 | **A100** | 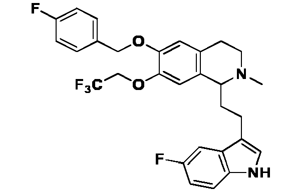 |
| **A101** | 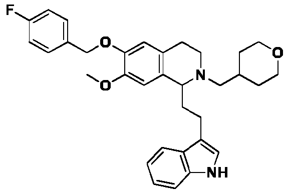 | **A102** | 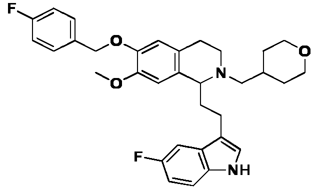 |

In the second aspect of the present invention, provided is a method for preparing a compound shown in formula (I), comprising steps of:
(1) in a mixed solvent of nitromethane and organic acid, subjecting a compound of formula IIₐ to a Henry reaction with nitromethane to obtain an α,β-unsaturated nitro compound II_{b}, then subjecting the compound of formula II_{b} to a reduction reaction to obtain a compound of formula II_{c}; 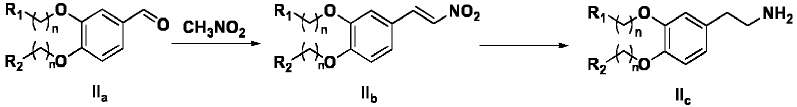
(2) in an basic solvent, in the presence of malonic acid and a base catalyst, subjecting a compound of formula Iₐ to Knoevenagel reaction with malonic acid to obtain a compound of formula I_{b}, then reducing the compound of formula I_{b} by a reducing agent to obtain a compound of formula I_{c}; 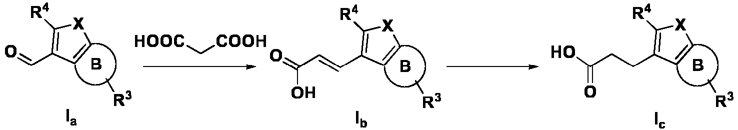
(3) in an inert solvent, in the presence of a condensing agent, reacting the compound of formula II_{c}, and the compound of formula I_{c} to obtain a compound of formula I_{d}; 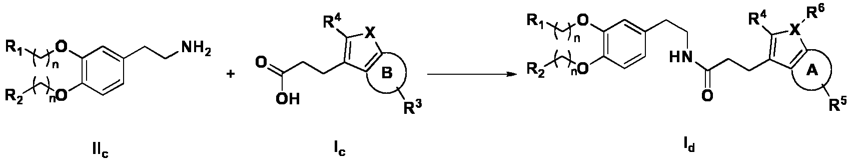
(4) in an inert solvent, performing Bischler Napieralski cyclization reaction using the compound of formula I_{d} to obtain the compound of formula Iₑ; 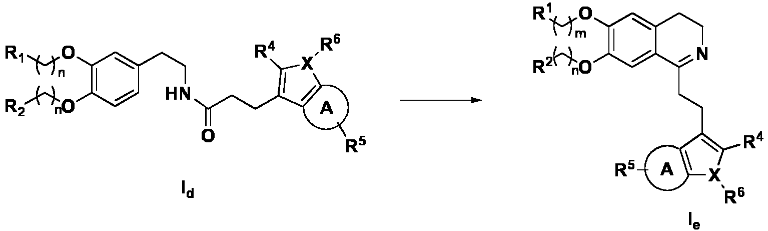
(5) in an inert solvent, performing a reduction reaction using the compound of formula Iₑ to obtain a compound of formula I_{f}; 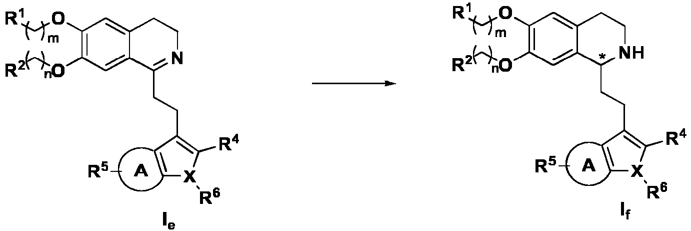
(6) in an inert solvent, performing a substitution reaction using the compound of formula I_{f} and halogenated hydrocarbon I_{g} to obtain a compound of formula (I); 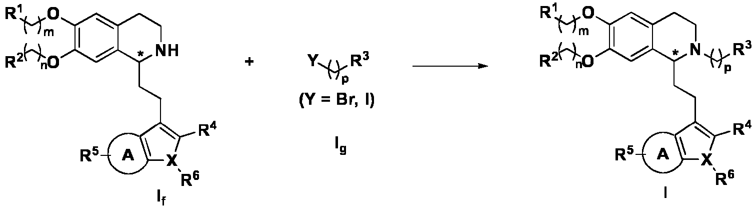 wherein, R¹, R², R³, R⁴, R⁵*,* R⁶, X, m, n, and p are as defined above.

In some embodiments, in step (1), the organic acid is acetic acid or formic acid, and the reducing agent is LiAlH₄, sodium borohydride, or zinc powder.

In another preferred embodiment, in step (2), the basic solvent is pyridine, the base catalyst is piperidine, and the reducing agent is a combination of palladium on carbon hydrogenation catalyst and hydrogen gas, or a combination of palladium on carbon hydrogenation catalyst and ammonium formate.

In another preferred embodiment, in step (3), the condensing agent is HATU or a combination of EDCI and HOBt.

In another preferred embodiment, in step (4), in the cyclization reaction , POCl₃ phosphorus oxychloride is used as the Lewis acid.

In another preferred embodiment, in step (5), in the reduction reaction , borohydride is used as the reducing agent, or Noyori's catalyst is used as the asymmetric reduction catalyst.

In another preferred embodiment, in step (5), in the substitution reaction, potassium carbonate, cesium carbonate, or sodium carbonate is used as the base for catalyzing the reaction.

In the third aspect of the present invention, provided is a pharmaceutical composition comprising: (1) the compound according to the first aspect of the present invention, the racemate, the R-isomer, the S-isomer, or the pharmaceutically acceptable salt thereof; and (2) pharmaceutically acceptable carriers.

In the fourth aspect of the present invention, provided is a use of the compound, according to the first aspect of the present invention, the racemate, the R-isomer, the S-isomer, or the pharmaceutically acceptable salt thereof, or the pharmaceutical composition according to the third aspect of the present invention in preventing and/or treating a disease related to lysosomal dysfunction and insufficient biosynthesis.

In another preferred embodiment, the disease is a neurodegenerative disease caused by the accumulation of pathological protein.

In another preferred embodiment, the disease is selected from the group consisting of: Alzheimer's disease and Parkinson's disease.

It should be understood that within the scope of the present invention, the above-mentioned technical features of the present invention and the specific technical features described in the following (such as example) can be combined with each other to form new or preferred technical solutions. Limited to space, it will not be elaborated here.

### Detailed Description of the Invention

After extensive and in-depth research, the inventor unexpectedly discovered a series of compounds of formula (I) and conducted a series of biological activity tests on them. It was found that compounds have good activity in inducing lysosomal biosynthesis and activating the autophagy lysosome pathway to degrade pathological protein deposition in the brain. On this basis, the present invention has been completed.

### Definition

Unless otherwise defined, all technical and scientific terms used herein have the same meanings as commonly understood by those of ordinary skill in the art to which the present invention belongs.

In this article, unless otherwise specified, the term "substituted" refers to one or more hydrogen atoms in a group are substituted with a substituent selected from the group consisting of: C₁-C₈ alkyl, C₃-C₈ cycloalkyl, C₁-C₈ alkoxy, halogen, hydroxyl, carboxyl, cyano, C₁-C₁₀ aldehyde group, C₂-C₆ acyl, C₂-C₁₀ ester group, amino, and phenyl; the phenyl includes unsubstituted phenyl or substituted phenyl with 1-3 substituents selected from the group consisting of: halogen, C₁-C₁₀ alkyl, cyano, hydroxyl, nitro, C₃-C₈ cycloalkyl, C₁-C₈ alkoxy, and amino.

Unless otherwise specified, each chiral carbon atom in all compounds of the present invention can optionally be in the R or S configuration, or a mixture of R and S configurations.

As used herein, the term "alkyl" includes linear or branched alkyl. For example, a C₁-C₆ alkyl represents a linear or branched alkyl containing 1-6 carbon atoms, such as methyl, ethyl, propyl, isopropyl, butyl, isobutyl, tert-butyl, etc.

As used herein, the term "alkoxy" refers to a group containing a structure of alkyl-oxy. For example, "C₁-C₆ alkoxy" refers to a linear or branched alkoxy containing 1-6 carbon atoms, including methoxy, ethoxy, propoxy, isopropoxy, butoxide, isobutoxy, tert-butoxide, etc.

As used herein, the term "heterocyclyl" or "heterocycloalkyl" refers to a saturated or partially saturated cyclic group with a specific number of ring atoms (such as 3-10 ring atoms), wherein 1-3 atoms are heteroatoms selected from N, S, and O. Which can be in the form of a monocyclic ring, a bicyclic ring, or a polycyclic ring, such as a bridged ring or a spiro ring. Specific examples can include oxetanyl, azetidinyl, tetrahydro-2H-pyranyl, piperidinyl, tetrahydrofuranyl, morpholinyl, and pyrrolidinyl.

As used herein, the term "aryl" refers to a cyclic aromatic group with a specific number of carbon atoms. For example, "C₆-C₁₀ aryl" refers to an aryl containing 6-10 carbon atoms, such as phenyl, naphthyl, or similar groups.

As used herein, the term "aryl" refers to a cyclic aromatic group with a specific number of ring atoms, wherein 1-3 atoms are heteroatoms selected from N, S, and O. Which can be in the form of a monocyclic ring or a fused ring. For example, "5-12 membered aryl" refers to aryl containing 5-12 carbon atoms, such as pyrrolyl, pyridinyl, thiophenyl, furanyl, imidazolyl, pyrimidinyl, benzothiophenyl, indolyl, imidazopyridinyl, quinolinyl, or similar groups.

As used herein, the term "acyl" refers to a -C(=O)R group with a specific number of carbon atoms. For example, C2-6 acyl refers to a -C(=O)R group with 2 to 6 carbon atoms, such as -C(=O)CH₃, -C(=O)C₂H₅, -C(=O)C₃H₇, -C(=O)C₄H₉, or similar groups.

As used herein, the term "ester group" refers to an R-O-C(=O)- group with a specific number of carbon atoms. For example, C2-6 acyl refers to R-O-C(=O)- groups with 2 to 6 carbon atoms, such as -C(=O)OCH₃, -C(=O)OC₂H₅, -C(=O)OC₃H₇, -C(=O)OC₄H₉, or similar groups.

As used herein, the term "alkenyl" refers to an alkenyl with a specific number of carbon atoms, including linear or branched alkynyl. For example, C₂-C₆ alkenyl refers to a linear or branched alkenyl with 2-6 carbon atoms, such as vinyl, allyl, 1-propenyl, isopropenyl, 1-butenyl, 2-butenyl, or similar groups.

As used herein, the term "alkynyl" refers to alkynyl with a specific number of carbon atoms, including linear or branched alkynyl. For example, C₂-C₆ alkynyl refers to linear or branched alkynyl with 2-6 carbon atoms, such as ethynyl, propynyl, butynyl, or similar groups.

As used herein, the term "amino" refers to a -NR¹R² group with 0-6 carbon atoms, such as - NH₂, -NHCH₃, -NHC(=O)CH₃, -NHCH₂CH₃, -N(CH₃)₂, or similar groups. In this article, amino is intended to include amino, namely -NH₂.

As used herein, the term "amido" refers to a -C(=O)NR¹R² group with a specific number of carbon atoms, such as -C(=O)NH₂, -C(=O)NHCH₃, -C(=O)N(CH₃)₂, or similar groups.

As used herein, the term "sulfonamido" refers to a -S(=O)₂NR¹R₂ group with a specific number of carbon atoms. For example, a C₁-C₆ sulfonamido refers to a sulfonamido with 1 to 6 carbon atoms, such as -S(=O)₂NH₂, -S(=O)₂NHCH₃, -S(=O)₂N(CH₃)₂, or similar groups. As used herein, the term "sulfonyl" refers to the -S(=O)₂- group.

As used herein, the term "sulfinyl" refers to the -S(=O)- group.

As used herein, the term "halogen" refers to F, Cl, Br, and I.

Unless otherwise specified, the structural formulas described in the present invention are intended to include all isomeric forms (such as enantiomers, diastereomers, and geometric isomers (or conformational isomers): for example, R, S configurations containing asymmetric centers, (Z), (E) isomers with double bonds, and (Z), (E) conformational isomers. Therefore, individual stereoisomers or mixtures of enantiomers, diastereomers, or geometric isomers (or conformational isomers) of the compounds of the present invention are within the scope of the present invention.

The term "tautomer" refers to structural isomers with different energies that can exceed a low energy barrier and thus transform into each other. For example, proton tautomers (i.e. proton shift) involve interconversion through proton transfer, such as 1H-indazole and 2H-indazole, 1H-benzo[d]imidazole and 3H-benzo[d]imidazole, and valence tautomers involve interconversion through recombination of some bonding electrons.

In this article, forms such as "C₁-C₆" indicate that the group can have 1 to 6 carbon atoms, such as 1, 2, 3, 4, 5, or 6.

### The main advantages of the present invention include:

(1) The compounds of the present invention have good activity in inducing lysosomal biosynthesis and activating the autophagy lysosome pathway to degrade pathological protein deposition in the brain.
(2) The preparation method of the compound of the present invention is simple.
(3) The compound of the present invention has good pharmacokinetic properties and good druggability.

### Specific Examples

The present invention will be further explained in conjunction with specific examples. It should be understood that these examples are only used to illustrate the present invention and not to limit the scope of the present invention. The experimental methods without specific conditions specified in the following examples are usually carried out under conventional conditions or conditions recommended by the manufacturer. Unless otherwise specified, percentages and portions are calculated by weight. The starting materials used in the present invention are all commercially purchased unless otherwise specified.

### Example 1: 5- (2- (1H-indol-3-yl) ethyl) -5,6,7,8-tetrahydro - [1,3] dioxolo [4,5-g] isoquinoline (A1)

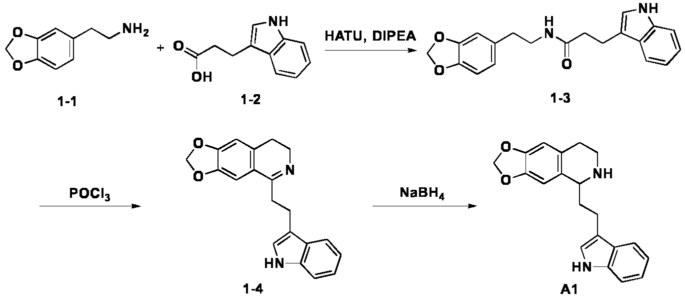

**1.1 Synthesis of Intermediate 1-3:** The starting material **1-2** (3-indole-propionic acid, 2.08 g, 11.0 mmol) was dissolved in 100 mL dichloromethane, and then 2-(7-azobenzotriazole)-*N,N,N*'*,N*'-tetramethylurea hexafluorophosphate (HATU, 5.70 g, 15.0 mmol), *N,N*-diisopropylethylamine (DIPEA, 5.22 mL, 30.0 mmol), and a dichloromethane solution of **1-1** (homopiperony lamine, 1.65 g, 10.0 mmol) were added sequentially, and the mixture was stirred at room temperature for 4 hours. After TLC (Thin Layer Chromatography) monitored the completion of the reaction, the reaction solution was diluted by adding dichloromethane, washed with solutions of 1N diluted hydrochloric acid, saturated sodium bicarbonate, and saturated sodium chloride in sequence, dried over anhydrous sodium sulfate, evaporated to dryness, and purified by column chromatography with petroleum ether/ethyl acetate=1:1 to obtain 3.02 g of yellowish white solid product 1-3, with a yield of 90%. **¹H NMR** (500 MHz, CDCl₃) δ 8.18 (s, 1H), 7.58 (d, *J* = 7.9 Hz, 1H), 7.36 (d, *J =* 8.1 Hz, 1H), 7.20 (ddd, *J =* 8.2, 7.0, 1.2 Hz, 1H), 7.12 (ddd, *J =* 7.9, 7.0, 1.1 Hz, 1H), 6.98 - 6.94 (m, 1H), 6.62 (d, *J* = 7.9 Hz, 1H), 6.53 (d, *J* = 1.7 Hz, 1H), 6.38 (dd, *J* = 7.9, 1.7 Hz, 1H), 5.91 (s, 2H), 5.42 (s, 1H), 3.38 (q, *J* = 6.6 Hz, 2H), 3.09 (t, *J* = 7.3 Hz, 2H), 2.54 (dt, *J* = 17.6, 7.1 Hz, 4H). ¹³C NMR (126 MHz, DMSO) δ 172.7, 147.7, 146.1, 136.3, 132.5, 127.1, 122.0, 121.8, 121.5, 119.3, 118.7, 114.8, 111.2, 109.0, 108.3, 100.8, 40.7, 37.4, 35.2, 21.3. ESI-MS *m*/*z* 336.2 [M+H]⁺.

**1.2 Synthesis of intermediate 1-4:** Intermediate **1-3** (2.70 g, 8.0 mmol) was dissolved in 50 mL anhydrous acetonitrile, and then phosphorus oxychloride (4.46 mL, 48.0 mmol) was added, and the mixture was stirred under reflux for 1.5 hours under argon protection. After TLC monitored the completion of the reaction, the mixture was evaporated to dryness under reduced pressure, adjusted to alkalescence by adding iced saturated sodium bicarbonate, extracted with dichloromethane, washed with saturated sodium chloride solution, dried over anhydrous sodium sulfate, concentrated, and purified by column chromatography with dichloromethane/methanol=20:1 to obtain 2.03 g of yellow solid product **1-4,** with a yield of 80%. ESI-MS *m*/*z* 319.1 [M+H]⁺.

**1.3 Synthesis of Compound A1:** Intermediate **1-4** (2.04 g, 6.4 mmol) was dissolved in 50 mL of a mixed solvent of methanol/dichloromethane (v/v=2:1), and sodium borohydride (1.46 g, 38.4 mmol) was added in batches under ice bath, and the resulting mixture was stirred at room temperature for 4 hours. After TLC monitored the completion of the reaction, the reaction was quenched with saturated ammonium chloride solution, and evaporated to dryness to remove the organic solvent under reduced pressure, and then extracted with dichloromethane, washed with solutions of saturated sodium bicarbonate and saturated sodium chloride sequentially, dried over anhydrous sodium sulfate, concentrated, and purified by column chromatography with dichloromethane/ methanol=15:1 to obtain 1.82 g of white solid product **A1**, with a yield of 89%. **¹H NMR** (500 MHz, CDCl₃) δ 7.87 (s, 1H), 7.61 (d, *J =* 7.9 Hz, 1H), 7.32 (d, *J =* 8.1 Hz, 1H), 7.18 (dd, *J =* 15.0, 7.4 Hz, 2H), 7.11 (t, *J* = 7.5 Hz, 1H), 6.54 (s, 1H), 6.45 (s, 1H), 5.92 - 5.85 (m, 2H), 4.23 (s, 1H), 3.50 (dd, *J =* 6.4, 4.7 Hz, 1H), 3.24 - 3.17 (m, 1H), 2.98 - 2.86 (m, 1H), 2.82 - 2.65 (m, 4H), 2.25 - 2.06 (m, 2H). **¹³C NMR** (150 MHz, CDCl₃) δ 146.3, 145.3, 136.3, 130.9, 127.5, 126.8, 122.0, 121.3, 119.2, 118.7, 116.7, 111.5, 108.4, 106.7, 100.3, 63.0, 47.9, 35.7, 26.0, 21.1. ESI-MS *m*/*z* 321.3 [M+H]⁺.

### Example 2: (S) -5- (2- (1H-indol-3-yl) ethyl) -5,6,7,8-tetrahydro - [1,3] dioxolo [4,5-g] isoquinoline ((S) - A1)

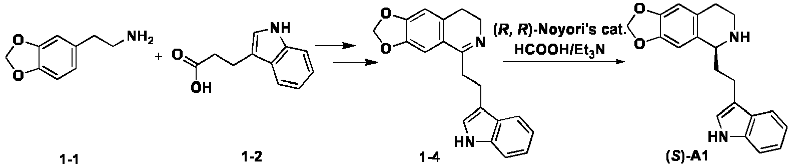

The synthesis of intermediate **1-4** was carried out with reference to Example 1. **1-4** (0.95 g, 3.0 mmol) was dissolved in 10 mL anhydrous DMF, and catalyst RuCl[(*R,R*)-TsDPEN (*p*-cymene)] (57 mg, 0.09 mmol) was added under argon protection at room temperature, and then a mixed solution of formic acid/ triethylamine (v/v=5/2, 1.1 mL) was added, and the mixture was stirred overnight at room temperature. After TLC monitored the completion of the reaction, the reaction solution was neutralized with saturated NaHCO₃ solution until the pH is alkalescence, and then added with 5 times the volume of ice water, and extracted three times with ethyl acetate. The organic layers were combined, washed once with NaHCO₃ solution and once with NaCl solution, and dried over Na₂SO₄. After the organic solvent was evaporated, the resulting residue was purified by column chromatography with dichloromethane/methanol=15:1 to obtain 1.82 g of white solid product **(*S*)-A1,** with a yield of 89%. The optical purity is 96:4 er (chromatographic conditions: chiral IA (4.6 mm × 250 mml), n-hexane/isopropanol/triethylamine=80/20/0.1, 0.8 mL/min, 254 nm UV, room temperature). **¹H NMR** (500 MHz, CDCl₃) δ 7.87 (s, 1H), 7.61 (d, *J =* 7.9 Hz, 1H), 7.32 (d, *J =* 8.1 Hz, 1H), 7.18 (dd, *J =* 15.0, 7.4 Hz, 2H), 7.11 (t, *J* = 7.5 Hz, 1H), 6.54 (s, 1H), 6.45 (s, 1H), 5.92 - 5.85 (m, 2H), 4.23 (s, 1H), 3.50 (dd, *J =* 6.4, 4.7 Hz, 1H), 3.24 - 3.17 (m, 1H), 2.98 - 2.86 (m, 1H), 2.82 - 2.65 (m, 4H), 2.25 - 2.06 (m, 2H). **¹³C NMR** (150 MHz, CDCl₃) δ 146.3, 145.3, 136.3, 130.9, 127.5, 126.8, 122.0, 121.3, 119.2, 118.7, 116.7, 111.5, 108.4, 106.7, 100.3, 63.0, 47.9, 35.7, 26.0, 21.1. ESI-MS *m*/*z* 321.3 [M+H]⁺.

### Example 3: (R) -5- (2- (1H-indol-3-yl) ethyl) -5,6,7,8-tetrahydro - [1,3] dioxolo [4,5-g] isoquinoline ((R) - A1)

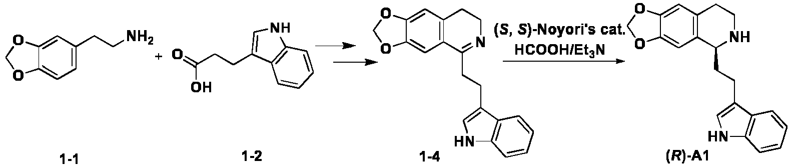

The synthesis of intermediate **1-4** was carried our with reference to Example 1. **1-4** (0.95 g, 3.0 mmol) was dissolved in 10 mL anhydrous D MF, and catalyst RuCl[(R,R)-TsDPEN(*p*-cymene)] (57 mg, 0.09 mmol) was added under argon protection at room temperature, and then a mixed solution of formic acid/triethylamine (v/v=5/2, 1.1 mL) was added, and the resulting mixture was stirred overnight at room temperature. After TLC monitored the completion of the reaction, the reaction solution was neutralized with saturated NaHCO₃ solution until the pH is alkalescence, and then added with 5 times the volume of ice water, and extracted three times with ethyl acetate. The organic layers was combined, washed once with NaHCO₃ solution and once with NaCl solution, and dried over Na₂SO₄. After the organic solvent was evaporated, the residue was purified by column chromatography with dichloromethane/methanol=15:1 to obtain 1.78 g of white solid product **(*R*)-A1,** with a yield of 87%. The optical purity is 95:5 er (chromatographic conditions: chiral IA (4.6 mm × 250 mml), n-hexane/isopropanol/triethylamine=80/20/0.1, 0.8 mL/min, 254 nm UV, room temperature). **¹H NMR** (500 MHz, CDCl₃) δ 7.87 (s, 1H), 7.61 (d, *J* = 7.9 Hz, 1H), 7.32 (d, *J* = 8.1 Hz, 1H), 7.18 (dd, *J =* 15.0, 7.4 Hz, 2H), 7.11 (t, *J =* 7.5 Hz, 1H), 6.54 (s, 1H), 6.45 (s, 1H), 5.92 - 5.85 (m, 2H), 4.23 (s, 1H), 3.50 (dd, *J =* 6.4, 4.7 Hz, 1H), 3.24 - 3.17 (m, 1H), 2.98 - 2.86 (m, 1H), 2.82 - 2.65 (m, 4H), 2.25 - 2.06 (m, 2H). **¹³C NMR** (150 MHz, CDCl₃) δ 146.3, 145.3, 136.3, 130.9, 127.5, 126.8, 122.0, 121.3, 119.2, 118.7, 116.7, 111.5, 108.4, 106.7, 100.3, 63.0, 47.9, 35.7, 26.0, 21.1. ESI-MS *m*/*z* 321.3 [M+H]⁺.

### Example 4: 1- (2- (1H-indol-3-yl) ethyl) -6,7- dimethoxy - 1,2,3,4 - tetrahydroisoquinoline (A2)

Referring to the synthesis route of **A1,** compound **A2** was obtained by replacing raw material **1-1** in Example 1 with 3,4-dimethoxyphenylethylamine (CAS: 120-20-7). The yield is 89%. ¹H NMR (400 MHz, DMSO-d₆) δ 10.91 (s, 1H), 7.63 (d, *J =* 7.8 Hz, 1H), 7.35 (d, *J* = 8.1 Hz, 1H), 7.24 (d, *J =* 2.3 Hz, 1H), 7.07 (t, *J =* 7.5 Hz, 1H), 6.98 (t, *J =* 7.6 Hz, 1H), 6.77 (s, 1H), 6.67 (s, 1H), 4.36 (t, *J* = 6.1 Hz, 1H), 3.72 (s, 3H), 3.63 (s, 3H), 3.47 - 3.36 (m, 1H), 3.26 - 3.15 (m, 1H), 3.07 - 2.91 (m, 3H), 2.96 - 2.81 (m, 1H), 2.32 (q, *J* = 7.8, 7.1 Hz, 2H). 13C NMR (100 MHz, DMSO-d₆) δ 148.4, 148.0, 136.8, 127.4, 125.7, 124.9, 123.1, 121.4, 119.0, 118.6, 113.7, 112.2, 111.9, 110.1, 55.98, 55.95, 54.3, 49.1, 34.9, 25.4, 21.7. ESI-MS *m*/*z* 337.2 [M+H]⁺.

### Example 5: (S) -1- (2- (1H-indol-3-yl) ethyl) -6,7- dimethoxy - 1,2,3,4 - tetrahydroisoquinoline ((S) - A2)

Referring to the synthesis route of **(*S*)-A1,** compound **(*S*)-A2** was obtained by replacing raw material **1-1** in Example 2 with 3,4-dimethoxyphenylethylamine (CAS: 120-20-7). ¹H NMR (400 MHz, DMSO-d₆) δ 10.91 (s, 1H), 7.63 (d, *J =* 7.8 Hz, 1H), 7.35 (d, *J =* 8.1 Hz, 1H), 7.24 (d, *J* = 2.3 Hz, 1H), 7.07 (t, *J* = 7.5 Hz, 1H), 6.98 (t, *J =* 7.6 Hz, 1H), 6.77 (s, 1H), 6.67 (s, 1H), 4.36 (t, *J* = 6.1 Hz, 1H), 3.72 (s, 3H), 3.63 (s, 3H), 3.47 - 3.36 (m, 1H), 3.26 - 3.15 (m, 1H), 3.07 - 2.91 (m, 3H), 2.96 - 2.81 (m, 1H), 2.32 (q, *J* = 7.8, 7.1 Hz, 2H). ¹³C NMR (100 MHz, DMSO-d₆) δ 148.4, 148.0, 136.8, 127.4, 125.7, 124.9, 123.1, 121.4, 119.0, 118.6, 113.7, 112.2, 111.9, 110.1, 55.98, 55.95, 54.3, 49.1, 34.9, 25.4, 21.7. ESI-MS *m*/*z* 337.2 [M+H]⁺.

### Example 6: (R) -1- (2- (1H-indol-3-yl) ethyl) -6,7- dimethoxy - 1,2,3,4 - tetrahydroisoquinoline ((R) - A2)

Referring to the synthesis route of **(*R*)-A1,** compound **(*R*)-A2** was obtained by replacing raw material **1-1** in Example 3 with 3,4-dimethoxyphenylethylamine (CAS: 120-20-7). ¹H NMR (400 MHz, DMSO-d₆) δ 10.91 (s, 1H), 7.63 (d, *J =* 7.8 Hz, 1H), 7.35 (d, *J =* 8.1 Hz, 1H), 7.24 (d, *J* = 2.3 Hz, 1H), 7.07 (t, *J* = 7.5 Hz, 1H), 6.98 (t, *J =* 7.6 Hz, 1H), 6.77 (s, 1H), 6.67 (s, 1H), 4.36 (t, *J* = 6.1 Hz, 1H), 3.72 (s, 3H), 3.63 (s, 3H), 3.47 - 3.36 (m, 1H), 3.26 - 3.15 (m, 1H), 3.07 - 2.91 (m, 3H), 2.96 - 2.81 (m, 1H), 2.32 (q, *J* = 7.8, 7.1 Hz, 2H). ¹³C NMR (100 MHz, DMSO-d₆) δ 148.4, 148.0, 136.8, 127.4, 125.7, 124.9, 123.1, 121.4, 119.0, 118.6, 113.7, 112.2, 111.9, 110.1, 55.98, 55.95, 54.3, 49.1, 34.9, 25.4, 21.7. ESI-MS *m*/*z* 337.2 [M+H]⁺.

### Example 7: 1- (2- (1H-indol-3-yl) ethyl) -6- (benzyloxy) -7- methoxy - 1,2,3,4 - tetrahydroisoquinoline (A3)

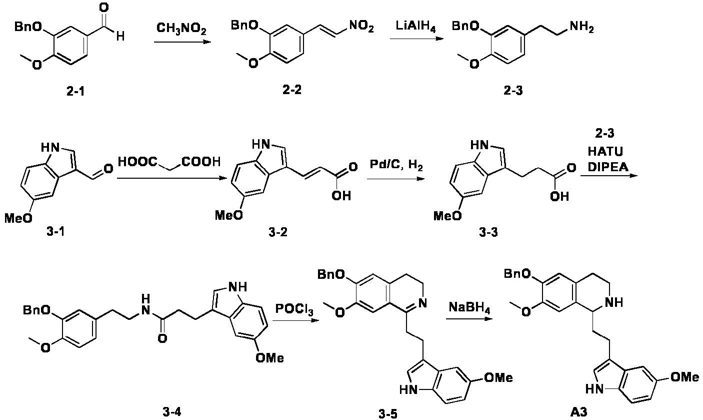

**Synthesis of Intermediate 2-3:** The starting material **2-1** (4.84 g, 20.0 mmol) was dissolved in nitromethane (21.5 mL, 400 mmol), and ammonium acetate (3.08 g, 40.0 mmol) and glacial acetic acid (11.4 mL, 200 mmol) were added in sequence, and then the resulting mixture was heated up to 80 °C and stirred for 2 hours. After TLC monitored the completion of reaction, the organic solvent was evaporated, and then the reaction was added with the saturated sodium bicarbonate slowly and stirred at room temperature to adjust the pH to neutral, and then the precipitated solid was filtered, washed once with saturated sodium bicarbonate and water in sequence, dried to obtain 4.62 g of bright yellow intermediate **2-2,** with a yield of 81%, which was directly used for the next step. **2-2** (4.56 g, 16.0 mmol) was dissolved in an ultra dry tetrahydrofuran solution for later use. In a -5 °C ice bath, 50 mL ultra dry tetrahydrofuran was added to a 250 mL two necked round bottom flask, and then lithium aluminum hydride (1.82 g, 48.0 mmol) was added in three batches. Under ice bath and argon protection, **2-2** solution was added dropwise into the reaction flask using a dropper funnel. After addition was complete, the dropper funnel was remove, the reaction flask was installed with a condenser tube, and protected with argon gas, and transferred into an oil bath and heated under reflux for 2 hours. After TLC monitored the completion of the reaction, the reaction flask was moved to a -5 °C ice bath and stirred, the ice water was added dropwise to quench the reaction until no bubbles were present, and the insoluble solid was filtered off, and washed with ethyl acetate, the filtrate was collected, and spin-dried to remover organic solvent, extracted with water and ethyl acetate, the organic layer was washed with saturated sodium chloride, dried over anhydrous sodium sulfate, concentrated, and purified by column chromatography with dichloromethane/methanol/ammonia solution = 10:1:0.1 to obtain 2.88 g of brown liquid product **2-3,** with a yield of 70%. **¹H NMR** (400 MHz, DMSO-d₆) δ 7.44-7.33 (m, 5H), 6.89 (br. d, *J* = 8.8Hz, 1H), 6.76 (dd, *J* = 8.4, 2.0 Hz, 1H), 5.04 (s, 2H), 3.72 (s, 3H), 2.73 t, *J =* 6.8Hz, 2H), 2.53 t, *J =* 6.8Hz, 2H). ESI-MS *m*/*z* 258.1 [M+H]⁺.

**Synthesis of Intermediate 3-3:** Raw material **3-1** (5-methoxyindole-3-carbaldehyde, 3.50 g, 20 mmol) and malonic acid (4.08 g, 40 mmol) were dissolved in 50 mL pyridine, and the mixture was added with piperidine (340 mg, 4 mmol) under argon protection and reacted at 100 °C for 3 hours. After the solvent was evaporated off, the reaction was transferred into a 0 °C ice bath, and added with 20 mL 6N hydrochloric acid dropwise. After being stirred for 30 minutes, the reaction was filtered and washed with water to obtain 3.12 g of white solid **3-2,** with a yield of 72%, which was directly used in the next step. **3-2** (2.17 g, 10 mmol) was dissolved in 40 mL methanol, and 217 mg palladium carbon hydrogenation catalyst (containing 10% water) was added, and the mixture was heated up to 55 °C and reacted overnight under 1-2 bar of hydrogen protection. After TLC monitored the completion of the reaction, the reaction was filtered, and the filtrate was collected, concentrated, and purified by column chromatography with dichloromethane/methanol=30:1 to obtain 1.93 g of white solid product **3-3,** with a yield of 88%. ¹H NMR (400 MHz, DMSO-d₆) δ 12.08 (s, 1H), 10.62 (s, 1H), 7.22 (d, *J =* 8.5 Hz, 1H), 7.07 ( d, *J =* 2.0 Hz, 1H), 6.99 (d, *J =* 2.5 Hz, 1H), 6.71 (dd, *J* = 8.5, 2.5 Hz, 1H), 3.76 (s, 3H), 2.89 (2H, t, *J* = 7.5 Hz), 2.58 (2H, t, *J* = 7.5 Hz). ESI-MS *m*/*z* 218.0 [M-H]-.

**Synthesis of intermediate 3-4:** Referring to the synthesis route of **A1**, white solid product **3-4** was obtained by replacing raw material **1-1** in Example 1 with **2-3** and replacing raw material **1-2** in Example 1 with **3-3. ¹H NMR** (500 MHz, CDCl₃) δ 8.18 (s, 1H), 7.57 (d, *J* = 7.9 Hz, 1H), 7.45-7.33 (m, 5H), 7.37 (d, *J =* 8.1 Hz, 1H), 7.22 (ddd, *J* = 8.2, 7.0, 1.2 Hz, 1H), 7.12 (ddd, *J =* 7.9, 7.0, 1.1 Hz, 1H), 6.62 (d, *J* = 7.9 Hz, 1H), 6.53 (d, *J =* 1.7 Hz, 1H), 6.38 (dd, *J =* 7.9, 1.7 Hz, 1H), 5.44 (s, 1H), 5.10 (s, 2H), 3.80 (s, 3H), 3.37 (q, *J =* 6.6 Hz, 2H), 3.09 (t, *J =* 7.1 Hz, 2H), 2.54 (dt, *J =* 17.5, 7.0 Hz, 4H). ESI-MS *m*/*z* 459.2 [M+H]⁺.

**Synthesis of Intermediate 3-5:** Intermediate **3-4** (3.66 g, 8.0 mmol) was dissolved in 50 mL anhydrous acetonitrile, and phosphorus oxychloride (4.46 mL, 48.0 mmol) was added and the resulting mixture was stirred under reflux for 1.5 hours under argon protection. After TLC monitored the completion of the reaction, the reaction was evaporated to dryness under reduced pressure, adjusted to alkalescence by adding iced saturated sodium bicarbonate, extracted with dichloromethane, and washed with saturated sodium chloride solution, then the organic phase was dried over anhydrous sodium sulfate, concentrated, and purified by column chromatography with dichloromethane/methanol=20:1 to obtain 2.71 g of yellow solid product **3-5,** with a yield of 77%. ESI-MS *m*/*z* 441.2 [M+H]⁺.

**Synthesis of Compound A3:** Intermediate **3-5** (2.64 g, 6.0 mmol) was dissolved in 50 mL of a mixed solvent of methanol/dichloromethane (v/v=2:1), and sodium borohydride (1.37 g, 36.0 mmol) was added in batches under ice bath and the resulting mixture was stirred at room temperature for 4 hours. After TLC monitored the completion of the reaction, the reaction was quenched with saturated ammonium chloride solution, the organic solvent was evaporated under reduced pressure and then the residue was extracted with dichloromethane, and the organic layer was washed with saturated sodium bicarbonate and saturated sodium chloride solution sequentially, dried over anhydrous sodium sulfate, concentrated, and purified by column chromatography with dichloromethane/methanol=15:1 to obtain 2.22 g of white solid product **A1,** with a yield of 84%. **¹H NMR** (500 MHz, CDCl₃) δ 7.88 (s, 1H), 7.60 (d, *J* = 7.9 Hz, 1H), 7.48-7.36 (m, 5H), 7.32 (d, *J* = 8.1 Hz, 1H), 7.16 (dd, *J* = 15.0, 7.4 Hz, 2H), 7.11 (t, *J =* 7.5 Hz, 1H), 6.52 (s, 1H), 5.21 (s, 2H), 4.23 (s, 1H), 3.50 (s, 3H), 3.24 - 3.17 (m, 1H), 2.82 - 2.65 (m, 4H), 2.58 - 2.31 (m, 2H), 2.25 - 2.06 (m, 2H). **¹³C NMR** (150 MHz, CDCl₃) δ 146.3, 145.3, 136.3, 130.9, 127.5, 126.8, 122.0, 121.3, 119.2, 118.7, 116.7, 111.5, 108.4, 106.7, 100.3, 63.0, 47.9, 35.7, 26.0, 21.1. ESI-MS *m*/*z* 443.3 [M+H]⁺.

### Example 8: 6- (benzyloxy) -1- (2- (5-fluoro-1H-indol-3-yl) ethyl) -7-methoxy-1,2,3,4- tetrahydroisoquinoline (A4)

Referring to the synthesis route of **A3,** compound **A4** was obtained by replacing raw material **3-1** in Example 7 with 5-fluoroindole-3-carboxaldehyde (CAS: 2338-71-8). ¹H NMR (500 MHz, CDCl₃) δ 7.92 (s, 1H), 7.65 (d, *J* = 7.9 Hz, 1H), 7.47-7.36 (m, 5H), 7.30 (d, *J* = 8.0 Hz, 1H), 7.15 (dd, *J* = 15.0, 7.4 Hz, 2H), 7.10 (t, *J* = 7.5 Hz, 1H), 6.52 (s, 1H), 5.21 (s, 2H), 4.23 (s, 1H), 3.24 - 3.17 (m, 1H), 2.82 - 2.65 (m, 4H), 2.55 - 2.31 (m, 2H), 2.25 - 2.08 (m, 2H). **¹³C NMR** (150 MHz, CDCl₃) δ 146.3, 145.3, 136.3, 130.9, 127.5, 126.8, 122.0, 121.3, 119.2, 118.7, 116.7, 111.5, 108.4, 106.7, 100.3, 47.9, 35.7, 26.0, 21.1. ESI-MS *m*/*z* 431.2 [M+H]⁺.

### Example 9: 1- (2- (1H-indol-3-yl) ethyl) -6- (benzyloxy) -7-methoxy-1,2,3,4-tetrahydroisoquinoline (A5)

Referring to the synthesis route of **A3,** compound **A5** was obtained by replacing intermediate **3-3** in Example 7 with 3-indole-propionic acid (CAS: 830-96-6). **¹H NMR** (500 MHz, CDCl₃) δ 8.05 (s, 1H), 7.75 (d, *J* = 7.9 Hz, 1H), 7.46-7.36 (m, 5H), 7.30 (d, *J* = 8.0 Hz, 1H), 7.13 (dd, *J* = 15.0, 7.4 Hz, 2H), 7.11 (t, *J =* 7.5 Hz, 1H), 6.72 (s, 1H), 6.50 (s, 1H), 5.21 (s, 2H), 4.23 (s, 1H), 3.24 - 3.17 (m, 1H), 2.82 - 2.65 (m, 4H), 2.55 - 2.31 (m, 2H), 2.25 - 2.08 (m, 2H). **¹³C NMR** (150 MHz, CDCl₃) δ 146.8, 145.3, 137.3, 130.9, 127.3, 126.8, 122.1, 121.3, 119.0, 118.7, 116.7, 111.5, 107.4, 106.5, 100.3, 48.0, 35.7, 26.2, 20.8. ESI-MS *m*/*z* 413.2 [M+H]⁺.

### Example 10: 5- (2- (1H-indol-3-yl) ethyl) -6- (cyclohexylmethyl) -5,6,7,8-tetrahydro - [1,3] dioxolo [4,5-g] isoquinoline (A12)

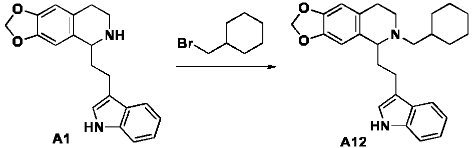

Product **A1** in Example 1 was used as the starting material for this reaction, **A1** (1.60 g, 5.0 mmol) and bromomethylcyclohexane (CAS: 2550-36-9) (1.32 g, 7.5 mmol) were dissolved in 50 mL acetonitrile, and potassium carbonate (3.45 g, 25.0 mmol) was added and the resulting mixture was stirred under reflux for 24 hours. After TLC monitored the completion of the reaction, the solvent was evaporated, and the residue was purified by column chromatography with dichloromethane /methanol=20:1 to obtain 1.35 g of yellow solid **A12,** with a yield of 65%. **¹H NMR** (500 MHz, CDCl₃) δ 8.10 (s, 1H), 7.62 (d, *J =* 7.9 Hz, 1H), 7.32 (d, *J* = 7.9 Hz, 1H), 7.25 - 7.11 (m, 2H), 6.95 (d, *J* = 2.1 Hz, 1H), 6.55 (d, *J* = 9.3 Hz, 2H), 5.88 (s, 2H), 3.96 - 3.90 (m, 2H), 3.61 (dd, *J* = 8.0, 4.4 Hz, 1H), 3.35 (m, 2H), 3.32 - 3.21 (m,1H), 2.45 - 2.25 (m, 2H), 2.23 - 2.00 (m, 5H), 1.69 - 1.46 (m, 5H), 1.41 - 1.24 (m, 4H). **¹³C NMR** (125 MHz, CDCl₃) δ 145.6, 145.5, 136.3, 131.7, 127.3, 127.3, 121.6, 121.0, 119.0, 118.8, 116.4, 111.0, 108.3, 107.4, 100.3, 68.0, 59.9, 50.1, 43.1, 36.3, 35.0, 33.1, 32.8, 24.0, 22.2. ESI/MS *m*/*z* 417.3 [M + H]⁺.

### Example 11: (S) -5- (2- (1H-indol-3-yl) ethyl) -6- (cyclohexylmethyl) -5,6,7,8-tetrahydro - [1,3] dioxazolo [4,5-g] isoquinoline ((S) - A12)

Referring to the synthesis route of **A12,** a yellow solid product **(*S*)-A12** was obtained by replacing the raw material **A1** in Example 1 with **(*S*)-A1.** ¹H NMR (500 MHz, CDCl₃) δ 8.10 (s, 1H), 7.62 (d, *J =* 7.9 Hz, 1H), 7.32 (d, *J =* 7.9 Hz, 1H), 7.25 - 7.11 (m, 2H), 6.95 (d, *J =* 2.1 Hz, 1H), 6.55 (d, *J* = 9.3 Hz, 2H), 5.88 (s, 2H), 3.96 - 3.90 (m, 2H), 3.61 (dd, *J* = 8.0, 4.4 Hz, 1H), 3.35 (m, 2H), 3.32 - 3.21 (m,1H), 2.45 - 2.25 (m, 2H), 2.23 - 2.00 (m, 5H), 1.69 - 1.46 (m, 5H), 1.41 - 1.24 (m, 4H). **¹³C NMR** (125 MHz, CDCl₃) δ 145.6, 145.5, 136.3, 131.7, 127.3, 127.3, 121.6, 121.0, 119.0, 118.8, 116.4, 111.0, 108.3, 107.4, 100.3, 68.0, 59.9, 50.1, 43.1, 36.3, 35.0, 33.1, 32.8, 24.0, 22.2. ESI/MS *m*/*z* 417.3 [M + H]⁺.

### Example 12: (R)-5- (2- (1H-indol-3-yl) ethyl) -6- (cyclohexylmethyl) -5,6,7,8-tetrahydro - [1,3] dioxolo [4,5-g] isoquinoline ((R) - A12)

Referring to the synthesis route of A12, a yellow solid product (R) - A12 was obtained by replacing the raw material **A1** in Example 1 with **(*R*)** - **A1. ^{1H}NMR** (500 MHz, CDCl₃) δ 8.10 (s, 1H), 7.62 (d, *J* = 7.9 Hz, 1H), 7.32 (d, *J =* 7.9 Hz, 1H), 7.25 - 7.11 (m, 2H), 6.95 (d, *J* = 2.1 Hz, 1H), 6.55 (d, *J* = 9.3 Hz, 2H), 5.88 (s, 2H), 3.96 - 3.90 (m, 2H), 3.61 (dd, *J =* 8.0, 4.4 Hz, 1H), 3.35 (m, 2H), 3.32 - 3.21 (m,1H), 2.45 - 2.25 (m, 2H), 2.23 - 2.00 (m, 5H), 1.69 - 1.46 (m, 5H), 1.41 - 1.24 (m, 4H). **¹³C NMR** (125 MHz, CDCl₃) δ 145.6, 145.5, 136.3, 131.7, 127.3, 127.3, 121.6, 121.0, 119.0, 118.8, 116.4, 111.0, 108.3, 107.4, 100.3, 68.0, 59.9, 50.1, 43.1, 36.3, 35.0, 33.1, 32.8, 24.0, 22.2. ESI/MS *m*/*z* 417.2 [M + H]⁺.

### Example 13: 5- (2- (1H-indol-3-yl) ethyl) -6- ((tetrahydro-2H-pyran-4-yl) methyl) -5,6,7,8-tetrahydro - [1,3] dioxolo [4,5-g] isoquinoline (A13)

Referring to the synthesis route of **A12,** 4-bromomethyltetrahydropyran (CAS: 125552-89-8) was used to replace the reaction reagent cyclohexylmethyl bromide in Example 10, and subjected to a substitution reaction with compound **A1** to obtain **A13. ¹H NMR** (500 MHz, CDCl₃) δ 8.11 (s, 1H), 7.65 (d, *J =* 8.0 Hz, 1H), 7.35 (d, *J =* 8.0 Hz, 1H), 7.23 - 7.09 (m, 2H), 6.96 (d, *J =* 2.2 Hz, 1H), 6.54 (d, *J =* 9.4 Hz, 2H), 5.88 (s, 2H), 3.98 - 3.92 (m, 2H), 3.59 (dd, *J =* 8.3, 4.5 Hz, 1H), 3.37 (m, 2H), 3.30 - 3.21 (m,1H), 2.98 - 2.79 (m, 4H), 2.49 (m, 1H), 2.23 - 2.00 (m, 2H), 1.69 - 1.46 (m, 5H), 1.41 - 1.24 (m, 2H). **¹³C NMR** (125 MHz, CDCl₃) δ 145.6, 145.5, 136.3, 131.7, 127.3, 127.3, 121.6, 121.0, 119.0, 118.8, 116.4, 111.0, 108.3, 107.4, 100.3, 68.0, 59.9, 50.1, 43.1, 36.3, 35.0, 33.1, 32.8, 24.0, 22.2. ESI/MS *m*/*z* 419.1 [M + H]⁺.

### Example 14: (S) -5- (2- (1H-indol-3-yl) ethyl) -6- (tetrahydro-2H-pyran-4-yl) methyl) -5,6,7,8-tetrahydro - [1,3] dioxolo [4,5-g] isoquinoline ((S) - A13)

Referring to the synthesis route of ***(S)*** - **A12,** 4-bromomethyltetrahydropyran (CAS: 125552-89-8) was used to replace cyclohexylmethyl bromide in Example 11, and subjected to a substitution reaction with compound **(*S*)** - **A1** to obtain **(*S*)** - **A13. ¹H NMR** (500 MHz, CDCl₃) δ 8.11 (s, 1H), 7.65 (d, *J* = 8.0 Hz, 1H), 7.35 (d, *J =* 8.0 Hz, 1H), 7.23 - 7.09 (m, 2H), 6.96 (d, *J* = 2.2 Hz, 1H), 6.54 (d, *J =* 9.4 Hz, 2H), 5.88 (s, 2H), 3.98 - 3.92 (m, 2H), 3.59 (dd, *J =* 8.3, 4.5 Hz, 1H), 3.37 (m, 2H), 3.30 - 3.21 (m,1H), 2.98 - 2.79 (m, 4H), 2.49 (m, 1H), 2.23 - 2.00 (m, 2H), 1.69 - 1.46 (m, 5H), 1.41 - 1.24 (m, 2H). **¹³C NMR** (125 MHz, CDCl₃) δ 145.6, 145.5, 136.3, 131.7, 127.3, 127.3, 121.6, 121.0, 119.0, 118.8, 116.4, 111.0, 108.3, 107.4, 100.3, 68.0, 59.9, 50.1, 43.1, 36.3, 35.0, 33.1, 32.8, 24.0, 22.2. ESI/MS *m*/*z* 419.1 [M + H]⁺.

### Example 15: (R) -5- (2- (1H-indol-3-yl) ethyl) -6- ((tetrahydro-2H-pyran-4-yl) methyl) -5,6,7,8-tetrahydro - [1,3] dioxolo [4,5-g] isoquinoline ((R) - A13)

Referring to the synthesis route of ***(R)*** - **A12,** 4-bromomethyltetrahydropyran (CAS: 125552-89-8) was used to replace cyclohexylmethyl bromide in Example 12, and subjected to a substitution reaction with compound **(*R*)** - **A1** to obtain **(*R*)** - **A13. ¹H NMR** (500 MHz, CDCl₃) δ 8.11 (s, 1H), 7.65 (d, *J* = 8.0 Hz, 1H), 7.35 (d, *J* = 8.0 Hz, 1H), 7.23 - 7.09 (m, 2H), 6.96 (d, *J* = 2.2 Hz, 1H), 6.54 (d, *J =* 9.4 Hz, 2H), 5.88 (s, 2H), 3.98 - 3.92 (m, 2H), 3.59 (dd, *J =* 8.3, 4.5 Hz, 1H), 3.37 (m, 2H), 3.30 - 3.21 (m,1H), 2.98 - 2.79 (m, 4H), 2.49 (m, 1H), 2.23 - 2.00 (m, 2H), 1.69 - 1.46 (m, 5H), 1.41 - 1.24 (m, 2H). **¹³C NMR** (125 MHz, CDCl₃) δ 145.6, 145.5, 136.3, 131.7, 127.3, 127.3, 121.6, 121.0, 119.0, 118.8, 116.4, 111.0, 108.3, 107.4, 100.3, 68.0, 59.9, 50.1, 43.1, 36.3, 35.0, 33.1, 32.8, 24.0, 22.2. ESI/MS *m*/*z* 419.1 [M + H]⁺.

### Example 16: tert Butyl-4- ((5- (2- (1H-indol-3-yl) ethyl) -7,8-dihydro - [1,3] dioxolo [4,5-g] isoquinolin-6 (5H) - yl) methyl) piperidine-1-carboxylate (A14)

Referring to the synthesis route of **A12,** 1-Boc-4-bromomethylpiperidine (CAS: 158407-04-6) was used to replace the reaction reagent cyclohexylmethyl bromide in Example 10, and subjected to a substitution reaction with compound **A1** to obtain **A14. ¹H NMR** (500 MHz, CDCl₃) δ 8.18 (s, 1H), 7.60 (s, 1H), 7.36 (d, *J =* 8.1 Hz, 1H), 7.21 - 7.12 (m, 2H), 7.05 (t, *J =* 7.4 Hz, 1H), 6.55 (d, *J =* 9.4 Hz, 2H), 5.46 (s, 2H), 4.09 (s, 2H), 3.52 (dd, *J =* 8.9, 4.1 Hz, 1H), 3.31 - 3.23 (m, 1H), 2.99 - 2.64 (m, 6H), 2.52 - 2.34 (m, 3H), 1.82 (m, 2H), 1.74 - 1.60 (m, 5H), 1.46 (s, 9H). **¹³C NMR** (125 MHz, CDCl₃) δ 161.5, 146.1, 145.8, 136.6, 132.0, 127.6, 127.2, 121.6, 121.0, 119.0, 118.8, 116.8, 111.1, 108.3, 107.5, 101.3, 78.8, 68.0, 59.9, 50.1, 45.1, 38.3, 36.1, 33.1, 32.5, 26.0, 25.4. ESI/MS *m*/*z* 518.3 [M + H]⁺.

### Example 17: (S) - tert butyl-4- ((5- (2- (1H-indol-3-yl) ethyl) -7,8-dihydro - [1,3] dioxolo [4,5-g] isoquinolin-6 (5H) - yl) methyl) piperidine-1- carboxylate ((S) - A14)

Referring to the synthesis route of ***(S)*** - **A12,** 1-Boc-4-bromomethylpiperidine (CAS: 158407-04-6) was used to replace cyclohexylmethyl bromide in Example 11, and subjected to a substitution reaction with compound **(*S*)** - **A1** to obtain **(*S*)** - **A14. ¹H NMR** (500 MHz, CDCl₃) δ 8.18 (s, 1H), 7.60 (s, 1H), 7.36 (d, *J =* 8.1 Hz, 1H), 7.21 - 7.12 (m, 2H), 7.05 (t, *J* = 7.4 Hz, 1H), 6.55 (d, *J=* 9.4 Hz, 2H), 5.46 (s, 2H), 4.09 (s, 2H), 3.52 (dd, *J* = 8.9, 4.1 Hz, 1H), 3.31 - 3.23 (m, 1H), 2.99 - 2.64 (m, 6H), 2.52 - 2.34 (m, 3H), 1.82 (m, 2H), 1.74 - 1.60 (m, 5H), 1.46 (s, 9H). **¹³C NMR** (125 MHz, CDCl₃) δ 161.5, 146.1, 145.8, 136.6, 132.0, 127.6, 127.2, 121.6, 121.0, 119.0, 118.8, 116.8, 111.1, 108.3, 107.5, 101.3, 78.8, 68.0, 59.9, 50.1, 45.1, 38.3, 36.1, 33.1, 32.5, 26.0, 25.4. ESI/MS *m*/*z* 518.3 [M + H]⁺.

### Example 18: (R) - tert butyl-4- ((5- (2- (1H-indol-3-yl) ethyl) -7,8-dihydro - [1,3] dioxolo [4,5-g] isoquinolin-6 (5H) - yl) methyl) piperidine-1-carboxylate ((R) - A14)

Referring to the synthesis route of ***(R)*** - **A12,** 1-Boc-4-bromomethylpiperidine (CAS: 158407-04-6) was used to replace cyclohexylmethyl bromide in Example 12, and subjected to a substitution reaction with compound **(*R*)** - A1 to obtain **(*R*)** - **A14. ¹H NMR** (500 MHz, CDCl₃) δ 8.18 (s, 1H), 7.60 (s, 1H), 7.36 (d, *J =* 8.1 Hz, 1H), 7.21 - 7.12 (m, 2H), 7.05 (t, *J* = 7.4 Hz, 1H), 6.55 (d, *J=* 9.4 Hz, 2H), 5.46 (s, 2H), 4.09 (s, 2H), 3.52 (dd, *J* = 8.9, 4.1 Hz, 1H), 3.31 - 3.23 (m, 1H), 2.99 - 2.64 (m, 6H), 2.52 - 2.34 (m, 3H), 1.82 (m, 2H), 1.74 - 1.60 (m, 5H), 1.46 (s, 9H). **¹³C NMR** (125 MHz, CDCl₃) δ 161.5, 146.1, 145.8, 136.6, 132.0, 127.6, 127.2, 121.6, 121.0, 119.0, 118.8, 116.8, 111.1, 108.3, 107.5, 101.3, 78.8, 68.0, 59.9, 50.1, 45.1, 38.3, 36.1, 33.1, 32.5, 26.0, 25.4. ESI/MS *m*/*z* 518.3 [M + H]⁺.

### Example 19: 5- (2- (1H-indol-3-yl) ethyl) -6- (piperidin-4-ylmethyl) -5,6,7,8-tetrahydro - [1,3] dioxolo [4,5-g] isoquinoline (A15)

1.0 **A14** was dissolved in 20 mL of a mixed solvent of dichloromethane/trifluoroacetic acid (v/v=5:1), and the mixture was stirred at room temperature for 1 hour. TLC monitoring showed that the raw materials were completely consumed. Then the reaction solution was added with saturated sodium carbonate solution and the pH of the reaction solution was adjusted to 8-9. The resulting solution was extracted with ethyl acetate, and the organic phases were combined, washed with saturated sodium chloride, dried over anhydrous sodium sulfate, evaporated to remove the solvent, and purified by column chromatography using dichloromethane/methanol=10:1 to obtain a yellow solid **A15. ¹H NMR** (500 MHz, CDCl₃) δ 8.18 (s, 1H), 7.60 (s, 1H), 7.36 (d, *J* = 8.1 Hz, 1H), 7.21 - 7.12 (m, 2H), 7.05 (t, *J* = 7.4 Hz, 1H), 6.55 (d, *J* = 9.4 Hz, 2H), 5.46 (s, 2H), 4.09 (s, 2H), 3.52 (dd, *J* = 8.9, 4.1 Hz, 1H), 3.31 - 3.23 (m, 1H), 2.99 - 2.64 (m, 6H), 2.52 - 2.34 (m, 3H), 1.82 (m, 2H), 1.74 - 1.60 (m, 5H), 1.46 (s, 9H). **¹³C NMR** (125 MHz, CDCl₃) δ 146.0, 145.5, 136.1, 132.3, 127.5, 127.1, 121.5, 121.1, 119.2, 118.7, 116.5, 111.0, 108.2, 107.4, 101.3, 78.8, 68.0, 59.8, 50.1, 45.1, 36.3, 33.1, 32.5, 25.4. ESI/MS *m*/*z* 418.2 [M + H]⁺.

### Example 20: (S) -5- (2- (1H-indol-3-yl) ethyl) -6- (piperidin-4-ylmethyl) -5,6,7,8-tetrahydro - [1,3] dioxolo [4,5-g] isoquinoline (S) - A15)

1.0 **(*S*)** - **A14** was dissolved in 20 mL of a mixed solvent of dichloromethane/trifluoroacetic acid (v/v=5:1), and the mixture was stirred at room temperature for 1 hour. TLC monitoring showed that the raw materials were completely consumed. Then the reaction solution was added with saturated sodium carbonate solution and the pH of the reaction solution was adjusted to 8-9. The resulting solution was extracted with ethyl acetate, and the organic phases were combined, washed with saturated sodium chloride, dried with anhydrous sodium sulfate, evaporated to remove the solvent, and purified by column chromatography using dichloromethane/methanol=10:1 to obtain a yellow solid **(*S*)** - **A15. ¹H NMR** (500 MHz, CDCl₃) δ 8.18 (s, 1H), 7.60 (s, 1H), 7.36 (d, *J* = 8.1 Hz, 1H), 7.21 - 7.12 (m, 2H), 7.05 (t, *J =* 7.4 Hz, 1H), 6.55 (d, *J =* 9.4 Hz, 2H), 5.46 (s, 2H), 4.09 (s, 2H), 3.52 (dd, *J =* 8.9, 4.1 Hz, 1H), 3.31 - 3.23 (m, 1H), 2.99 - 2.64 (m, 6H), 2.52 - 2.34 (m, 3H), 1.82 (m, 2H), 1.74 - 1.60 (m, 5H), 1.46 (s, 9H). **¹³C NMR** (125 MHz, CDCl₃) δ 146.0, 145.5, 136.1, 132.3, 127.5, 127.1, 121.5, 121.1, 119.2, 118.7, 116.5, 111.0, 108.2, 107.4, 101.3, 78.8, 68.0, 59.8, 50.1, 45.1, 36.3, 33.1, 32.5, 25.4. ESI/MS *m*/*z* 418.2 [M + H]⁺.

### Example 21: (R) -5- (2- (1H-indol-3-yl) ethyl) -6- (piperidin-4-ylmethyl) -5,6,7,8-tetrahydro - [1,3] dioxolo [4,5-g] isoquinoline ((R) - A15)

*1.0* **(*R*)** - **A14** was dissolved in 20 mL of a mixed solvent of dichloromethane/trifluoroacetic acid (v/v=5:1), and the mixture was stirred at room temperature for 1 hour. TLC monitoring showed that the raw materials were completely consumed. Then the reaction solution was added with saturated sodium carbonate solution and the pH of the reaction solution was adjusted to 8-9. The resulting solution was extracted with ethyl acetate, and the organic phases were combined, washed with saturated sodium chloride, dried over anhydrous sodium sulfate, evaporated to remove the solvent, and purified by column chromatography using dichloromethane/methanol=10:1 to obtain a yellow solid **(*R*)** - **A15. ¹H NMR** (500 MHz, CDCl₃) δ 8.18 (s, 1H), 7.60 (s, 1H), 7.36 (d, *J* = 8.1 Hz, 1H), 7.21 - 7.12 (m, 2H), 7.05 (t, *J =* 7.4 Hz, 1H), 6.55 (d, *J =* 9.4 Hz, 2H), 5.46 (s, 2H), 4.09 (s, 2H), 3.52 (dd, *J =* 8.9, 4.1 Hz, 1H), 3.31 - 3.23 (m, 1H), 2.99 - 2.64 (m, 6H), 2.52 - 2.34 (m, 3H), 1.82 (m, 2H), 1.74 - 1.60 (m, 5H), 1.46 (s, 9H). **¹³C NMR** (125 MHz, CDCl₃) δ 146.0, 145.5, 136.1, 132.3, 127.5, 127.1, 121.5, 121.1, 119.2, 118.7, 116.5, 111.0, 108.2, 107.4, 101.3, 78.8, 68.0, 59.8, 50.1, 45.1, 36.3, 33.1, 32.5, 25.4. ESI/MS *m*/*z* 418.2 [M + H]⁺.

### Example 22: 5- (2- (1H-indol-3-yl) ethyl) -6- ((1-methylpiperidin-4-yl) methyl) -5,6,7,8-tetrahydro - [1,3] dioxolo [4,5-g] isoquinoline (A16)

1.0 **A15** was dissolved in 20 mL of acetonitrile, and 510 mg of iodomethane and 1.65 g of potassium carbonate were added in sequence. The mixture was reacted under reflux for 24 hours. TLC monitoring showed that the raw materials were completely consumed. The reaction solution was spin-dried to remove the organic solvent, extracted by adding with water and ethyl acetate. The organic phases were combined, washed with saturated sodium chloride, dried over anhydrous sodium sulfate, evaporated to remove the solvent, and purified by column chromatography using dichloromethane/methanol=15:1 to obtain a yellow solid **A16.** ESI-MS *m*/*z* 432.2 [M+H]⁺.

### Example 23: 5- (2- (1H-indol-3-yl) ethyl) -6- ((1-methylpiperidin-4-yl) methyl) -5,6,7,8-tetrahydro - [1,3] dioxolo [4,5-g] isoquinoline (A17)

Referring to the synthesis route of **A12,** 3-bromomethyltetrahydrofuran (CAS: 165253-29-2) was used to replace the reaction reagent cyclohexylmethyl bromide in Example 10, and subjected to a substitution reaction with compound **A1** to obtain **A17.** ESI-MS *m*/*z* 405.1 [M+H]⁺

### Example 24: 5- (2- (1H-indol-3-yl) ethyl) -6- (2- (tetrahydro-2H-pyran-4-yl) ethyl) -5,6,7,8-tetrahydro - [1,3] dioxolo [4,5-g] isoquinoline (A18)

Referring to the synthesis route of **A12,** 4-bromoethyltetrahydropyran (CAS: 4677-20-7) was used to replace the reaction reagent cyclohexylmethyl bromide in Example 10, and subjected to a substitution reaction with compound **A1** to obtain **A18. ¹H NMR** (400 MHz, CDCl₃) δ 8.08 (s, 1H), 7.61 (d, *J* = 7.8 Hz, 1H), 7.35 (d, *J* = 8.1 Hz, 1H), 7.25 - 7.17 (m, 1H), 7.15 - 7.10 (m, 1H), 7.02 (s, 1H), 6.59 (s, 1H), 6.49 (s, 1H), 4.07 - 3.97 (m, 2H), 3.87 (s, 3H), 3.79 (s, 3H), 3.57 (s, 1H), 3.41 (td, *J =* 11.4, 4.4 Hz, 2H), 3.32 (dd, *J* = 19.4, 10.0 Hz, 1H), 3.02 - 2.77 (m, 4H), 2.59 - 2.37 (m, 2H), 2.04 - 2.17 (m, 2H), 1.76 - 1.85 (m, 2H), 1.36 - 1.26 (m, 2H). **¹³C NMR** (125 MHz, CDCl₃): δ 147.4, 136.5, 130.9, 127.0, 125.6, 122.0, 121.0, 119.1, 119.0, 117.0, 111.4, 111.1, 110.7, 68.1, 61.4, 60.1, 55.9, 55.8, 44.0, 36.8, 32.8, 32.0, 29.7, 24.7, 23.8, 22.5. ESI/MS *m*/*z* 433.1 [M + H]⁺.

### Example 25: 5- (2- (1H-indol-3-yl) ethyl) -6-phenylmethyl-5,6,7,8-tetrahydro-1,3-dioxolo [4,5-g] isoquinoline (A23)

Referring to the synthesis route of **A12,** bromobenzyl was used to replace the reaction reagent cyclohexylmethyl bromide in Example 10, and subjected to a substitution reaction with compound **A1** to obtain **A23.** ESI/MS *m*/*z* 411.2 [M + H]⁺.

### Example 26: 5- (2- (1H-indol-3-yl) ethyl) -6- (pyridin-4-ylmethyl) -5,6,7,8-tetrahydro - [1,3] dioxolo [4,5-g] isoquinoline (A24)

Referring to the synthesis route of **A12,** 4-bromomethylpyridine (CAS: 54751-01-8) was used to replace the reaction reagent cyclohexylmethyl bromide in Example 10, and subjected to a substitution reaction with compound **A1** to obtain **A24.** ESI/MS *m*/*z* 412.2 [M + H]⁺.

### Example 27: 5- (2- (1H-indol-3-yl) ethyl) -6- (pyrimidin-5-ylmethyl) -5,6,7,8-tetrahydro - [1,3] dioxolo [4,5-g] isoquinoline (A25)

Referring to the synthesis route of **A12,** 5- (bromomethyl) pyrimidine (CAS: 25198-96-3) was used to replace the reaction reagent cyclohexylmethyl bromide in Example 10, and subjected to a substitution reaction with compound **A1** to obtain **A25.** ESI/MS *m*/*z* 413.2 [M + H]⁺.

### Example 28:1- (2- (1H-indol-3-yl) ethyl) -6,7-dimethoxy-2-((tetrahydro-2H-pyran-4-yl) methyl) -1,2,3,4-tetrahydroisoquinoline (A26)

Referring to the synthesis routes of **A3** and **A12, A26** was obtained by replacing **2-3** and **3-3** in Example 7 with 3,4-dimethoxyphenylethylamine and 3-indole-propionic acid, respectively, and replacing the reaction reagent cyclohexylmethyl bromide in Example 10 with 4-bromomethyltetrahydropyran (CAS: 125552-89-8). ESI/MS *m*/*z* 413.2 [M+H]⁺. As light yellow solid, **¹H NMR** (400 MHz, CDCl₃) δ 8.06 (brs, 1H), 7.63 (d, J=7.8 Hz, 1H), 7.38 (d, J=8.1 Hz, 1H), 7.24-7.18 (m, 1H), 7.16-7.10 (m, 1H), 7.02 (s, 1H), 6.59 (s, 1H), 6.49 (s, 1H), 4.07-3.97 (m, 2H), 3.87 (s, 3H), 3.79 (s, 3H), 3.57 (s, 1H), 3.41 (td). J=11.4, 4.4 Hz, 2H), 3.32 (dd, J=19.4, 10.0 Hz, 1H), 3.02-2.77 (m, 4H), 2.59-2.37 (m, 3H), 2.04-2.17 (m, 2H), 1.76-1.85 (m, 3H), 1.36-1.26 (m, 2H). **¹³C NMR** (125 MHz, CDCl₃): δ 147.2, 136.5, 130.9, 127.6, 126.6, 122.0, 121.0, 119.1, 119.0, 117.0, 111.4, 111.1, 110.7, 68.1, 61.4, 60.1, 55.9, 55.8, 44.1 0, 36.8, 33.8, 32.0, 31.7, 29.7, 23.7, 22.1. ESI/MS *m*/*z* 435.0

### Example 29:5- (2- (1H-indol-3-yl) ethyl) -7- ((tetrahydro-2H-pyran-4-yl) methyl) -2,3,6,7,8,9-hexahydro - [1,4] dioxino [2,3-g] isoquinoline (A27)

Referring to the synthesis routes of **A3** and **A12, A27** was obtained by replacing **2-1** and **3-3** in Example 7 with 1,4-benzodioxan-6-carboxaldehyde(CAS: 29668-44-8) and 3-indole-propionic acid, respectively, and replacing the reaction reagent cyclohexylmethyl bromide in Example 10 with 4-bromomethyltetrahydropyran (CAS: 125552-89-8) as a white solid. **¹H NMR** (400 MHz, CDCl₃) δ 8.24 (s, 1H), 7.35 (d, *J =* 8.3 Hz, 1H), 7.25 - 7.11 (m, 1H), 7.09 (s, 1H), 6.44 (s, 1H), 6.50 (s, 1H), 6.44 (s, 1H), 6.36 - 6.16 (m, 2H), 4.77 (s, 2H), 4.51 (t, *J* = 13.7 Hz, 2H), 3.33 - 3.05(m, 1H), 3.00 - 2.61 (m, 4H), 4.12 (s, 3H), 2.32 - 2.17 (m, 2H). **¹³C NMR** (125 MHz, CDCl₃) δ 160.9, 150.8, 147.3, 135.8, 128.1, 126.5, 125.4, 124.4, 121.8, 122.7, 121.8, 119.0, 118.3, 115.1, 114.6, 108.9, 92.7, 70.9, 53.7, 43.2, 33.5, 25.4. ESI-MS *m*/*z* 433.2.

### Example 30: 1- (2- (1H-indol-3-yl) ethyl) -7- (benzyloxy) -6-methoxy-2-((tetrahydro-2H-pyran-4-yl) methyl) -1,2,3,4-tetrahydroisoquinoline (A28)

Referring to the synthesis routes of **A3** and **A12, A28** was obtained by replacing **2-1** and **3-3** in Example 7 with 4-benzyloxy-3-methoxybenzaldehyde (CAS: 2426-87-1) and 3-indole-propionic acid, respectively, and replacing the reaction reagent cyclohexylmethyl bromide in Example 10 with 4-bromomethyltetrahydropyran (CAS: 125552-89-8). ESI-MS *m*/*z* 511.3.

### Example 31: 1- (2- (1H-indol-3-yl) ethyl) -6- (benzyloxy) -7-methoxy-2-((tetrahydro-2H-pyran-4-yl) methyl) -1,2,3,4-tetrahydroisoquinoline (A29)

Referring to the synthesis routes of **A3** and **A12, A29** was obtained by replacing **2-1** and **3-3** in Example 7 with 3-benzyloxy-4-methoxybenzaldehyde (CAS: 6346-05-0) and 3-indole-propionic acid, respectively, and replacing the reaction reagent cyclohexylmethyl bromide in Example 10 with 4-bromomethyltetrahydropyran. ESI-MS *m*/*z 511.3.*

### Example 32: 1- (2- (1H-indol-3-yl) ethyl) -2- ((tetrahydro-2H-pyran-4-yl) methyl) -1,2,3,4-tetrahydroisoquinoline-6,7-diol (A30)

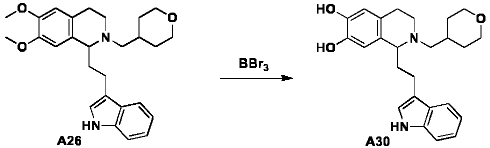

Compound **A26** (1.30 g, 3.0 mmol) was dissolved in ultra-dry dichloromethane and 4N boron tribromide in dichloromethane solution (7.5 mL, 30 mmol) was added dropwise at -15 °C. The mixture was then transferred to room temperature and stirred for 4 hours. After TLC monitoring showed that the reaction was complete, excess water was added to quench the reaction, and the resulting mixture was extracted with ethyl acetate, and the organic phase was washed once with saturated sodium bicarbonate and once with saturated sodium chloride solution, then concentrated and purified by column chromatography using dichloromethane/methanol=15:1 to obtain 0.91 g of **A30** with a yield of 75% as a yellow solid. **¹H NMR** (400 MHz, CDCl₃) δ 8.06 (brs, 1H), 7.68 (d, *J =* 7.8 Hz, 1H), 7.28 (d, *J =* 8.1 Hz, 1H), 7.16 - 7.11 (m, 1H), 7.05 - 7.01 (m, 1H), 6.95 (s, 1H), 6.49 (s, 1H), 6.30 (s, 1H), 4.08 - 3.97 (m, 2H), 3.57 (s, 1H), 3.41 (td, *J* = 11.4, 4.4 Hz, 2H), 3.32 (dd, *J =* 19.4, 10.0 Hz, 1H), 3.02 - 2.77 (m, 4H), 2.59 - 2.37 (m, 3H), 2.04 - 2.17 (m, 2H), 1.76 - 1.85 (m, 3H), 1.36 - 1.26 (m, 2H). **¹³C NMR** (125 MHz, CDCl₃): δ 145.2, 137.5, 131.9, 125.6, 123.5, 122.0, 121.0, 119.1, 119.0, 117.0, 111.4, 111.1, 110.7, 62.1, 55.9, 55.8, 42.0, 36.8, 33.8, 32.0, 31.7, 29.7, 23.7, 22.1. ESI/MS *m*/*z* 405.1 [M - H]⁻.

### Example 33:5- (2- (1H-indol-3-yl) ethyl) -2-propyl-6- ((tetrahydro-2H-pyran-4-yl) methyl) -5,6,7,8-tetrahydro - [1,3] dioxolo [4,5-g] isoquinoline (A31)

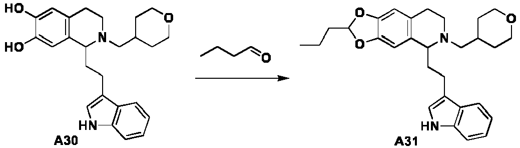

Compound **A30** (0.81 g, 2.0 mmol), n-butanal (173 mg, 2.4 mmol), and p-toluenesulfonic acid (34 mg, 0.2 mmol) were dissolved in 10 mL of toluene and the mixture was heated at 110 ° C under reflux for 24 hours. After TLC monitoring showed that the reaction was complete, the mixture was extracted with water and ethyl acetate. The organic phase was collected, washed once with saturated sodium chloride solution, concentrated and purified by column chromatography using dichloromethane/methanol=40:1 to obtain 0.62 g of **A31** with a yield of 67% as a light yellow solid. ESI/MS *m*/*z* 461.2 [M + H]⁺.

### Example 34: 5- (2- (1H-indol-3-yl) ethyl) -2- ((1-methylpiperidin-4-yl) methyl) -6-((tetrahydro-2H-pyran-4-yl) methyl) -5,6,7,8-tetrahydro - [1,3] dioxolo [4,5-g] isoquinoline (A32)

Referring to the synthesis route of **A31, A32** was obtained by replacing the reaction reagent n-butanal in Example 33 with 1-methylpiperidine-4-carbaldehyde (CAS: 50675-21-3). ESI/MS *m*/*z* 530.3 [M + H]⁺.

### Example 35: 5- (2- (1H-indol-3-yl) ethyl) -2-phenyl-6- ((tetrahydro-2H-pyran-4-yl) methyl) -5,6,7,8-tetrahydro - [1,3] dioxolo [4,5-g] isoquinoline (A33)

Referring to the synthesis route of **A31, A33** was obtained by replacing the reaction reagent n-butanal in Example 33 with benzaldehyde. ESI/MS *m*/*z* 495.0 [M + H]⁺.

### Example 36:5- (2- (1H-indol-3-yl) ethyl) -2-phenylmethyl-6-((tetrahydro-2H-pyran-4-yl) methyl) -5,6,7,8-tetrahydro - [1,3] dioxolo [4,5-g] isoquinoline (A34)

Referring to the synthesis route of **A31, A34** was obtained by replacing the reaction reagent n-butanal in Example 43 with phenylacetaldehyde. ESI/MS *m*/*z* 509.2 [M + H]⁺.

### Example 37:7- (2- (1H-indol-3-yl) ethyl) -8- ((tetrahydro-2H-pyran-4-yl) methyl) -3,4,7,8,9,10-hexahydro-2H - [1,4] dioxepino [2,3-g] isoquinoline (A35)

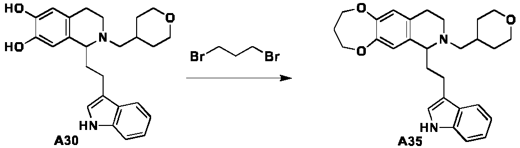

Compound **A30** (0.81 g, 2.0 mmol) and 1,3-dibromopropane (0.81 g, 4 mmol) were dissolved in 20 mL of acetonitrile, and then potassium carbonate (2.2 g, 16 mmol) was added. The mixture was heated under reflux at 85 ° C for 48 hours. After TLC monitoring showed that the reaction was complete, the reaction solution was filtered, and the filter cake was washed with ethyl acetate. The filtrate was collected, concentrated, and then purified by column chromatography using dichloromethane/methanol=40:1 to obtain 0.52 g of **A35** with a yield of 58% as a light yellow solid. ESI/MS *m*/*z* 447.1 [M + H]⁺.

### Example 38: 1- (2- (1H-indol-3-yl) ethyl) -6,7-di (2-methoxyethoxy) -2-((tetrahydro-2H-pyran-4-yl) methyl) -1,2,3,4-tetrahydroisoquinoline (A36)

Referring to the synthesis route of **A35, A36** was obtained by replacing the reaction reagent 1,3-dibromopropane in Example 37 with 2-bromoethyl methyl ether (CAS: 6482-24-2). ESI/MS *m*/*z* 523.3 [M + H]⁺.

### Example 39: 12- (2- (1H-indol-3-yl) ethyl) -13- (tetrahydro-2H-pyran-4-yl) methyl) -2,3,5,6,8,9,12,13,14,15-decahydro-[1,4,7,10] tetraoxacyclododeca [2,3-g] isoquinoline (A37)

Referring to the synthesis route of **A35, A37** was obtained by replacing the reaction reagent 1,3-dibromopropane in Example 37 with 1,14-Dibromo-3,6,9,12-tetraoxatetradecane (CAS: 57602-02-5). ESI/MS *m*/*z* 521.2 [M + H]⁺.

### Example 40: 1- (2- (1H-indol-3-yl) ethyl) -6-methoxy-2-((tetrahydro-2H-pyran-4-yl) methyl) -1,2,3,4-tetrahydroisoquinoline-7-ol (A38)

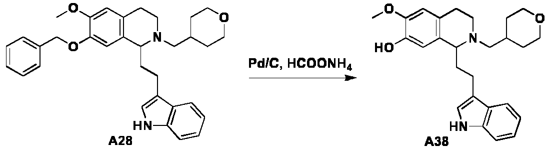

Compound **A28** (1.0 g, 2.0 mmol) was dissolved in 20 mL of methanol, and then 100 mg of Pd/C catalyst (containing 10% water) and ammonium formate (1.26 g, 20 mmol) were added. The mixture was heated and stirred at 55 °C for 12 hours. After TLC monitoring showed that the reaction was complete, the reaction solution was filtered, and the filtrate was collected, concentrated to remove the solvent, and purified by column chromatography using dichloromethane/methanol=20:1 to obtain 0.70 g of **A38** with a yield of 83% as a yellow solid. ESI/MS *m*/*z* 420.2 [M + H]⁺.

### Example 41: 1- (2- (1H-indol-3-yl) ethyl) -7-methoxy-2-((tetrahydro-2H-pyran-4-yl) methyl) -1,2,3,4-tetrahydroisoquinoline-6-phenol (A39)

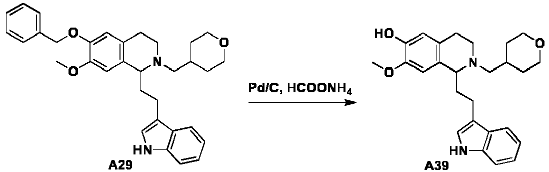

Compound **A29** (1.0 g, 2.0 mmol) was dissolved in 20 mL of methanol, and then 100 mg of Pd/C catalyst (containing 10% water) and ammonium formate (1.26 g, 20 mmol) were added. The mixture was heated and stirred at 55 °C for 12 hours. After TLC monitoring showed that the reaction was complete, the reaction solution was filtered, and the filtrate was collected, concentrated to remove the solvent, and purified by column chromatography using dichloromethane/methanol=20:1 to obtain 0.71 g of **A39** with a yield of 85% as a yellow solid. ESI/MS *m*/*z* 420.2 [M + H]⁺.

### Example 42:1- (2- (1H-indol-3-yl) ethyl) -6-ethoxy-7-methoxy-2-((tetrahydro-2H-pyran-4-yl) methyl) -1,2,3,4-tetrahydroisoquinoline (A40)

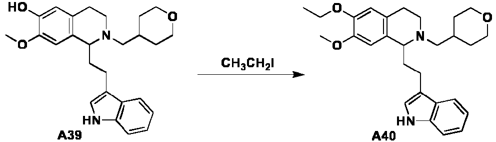

Compound **A39** (1.05 g, 2.5 mmol) was dissolved in 20 mL of acetonitrile, and then ethyl iodide(0.58g, 3.75mmol) and potassium carbonate (1.04 g, 7.5 mmol) were added. The mixture was stirred under reflux at 85 °C for 12 hours. After TLC monitoring showed that the reaction was complete, the reaction solution was filtered, and the filtrate was collected, concentrated to remove the solvent, and then purified by column chromatography using dichloromethane/methanol=30:1 to obtain 0.87 g of **A40** with a yield of 78% as a yellow solid. ESI/MS *m*/*z* 449.2 [M + H]⁺.

### Example 43:1- (2- (1H-indol-3-yl) ethyl) -7-methoxy-2- ((tetrahydro-2H-pyran-4-yl) methyl) -6- (2,2,2-trifluoroethoxy) -1,2,3,4-tetrahydroisoquinoline (A41)

Referring to the synthesis route of **A40, A41** was obtained by replacing the reaction reagent ethyl iodide in Example 52 with 2-iodo-1,1,1-trifluoroethane (CAS: 353-83-3). ESI/MS *m*/*z* 503.2 [M + H]⁺.

### Example 44: 1- (2- (1H-indol-3-yl) ethyl) -6- (cyclopropylmethoxy) -7-methoxy-2-((tetrahydro-2H-pyran-4-yl) methyl) -1,2,3,4-tetrahydroisoquinoline (A42)

Referring to the synthesis route of **A40, A42** was obtained by replacing the reaction reagent ethyl iodide in Example 42 with iodomethyl-cyclopropane (CAS: 33574-02-6). ESI/MS *m*/*z* 475.3 [M + H]⁺.

### Example 45:1- (2- (1H-indol-3-yl) ethyl) -6- (cyclopentylmethoxy) -7-methoxy-2-((tetrahydro-2H-pyran-4-yl) methyl) -1,2,3,4-tetrahydroisoquinoline (A43)

Referring to the synthesis route of **A40, A43** was obtained by replacing the reaction reagent ethyl iodide in Example 42 with iodomethyl-cyclopropane (CAS: 27935-87-1). ESI/MS *m*/*z* 503.2 [M + H]⁺.

### Example 46: Ethyl 2- ((1- (2- (1H-indol-3-yl) ethyl) -7-methoxy-2-((tetrahydro-2H-pyran-4-yl) methyl) -1,2,3,4-tetrahydroisoquinolin-6-yl) oxo) acetate (A44)

Referring to the synthesis route of **A40, A44** was obtained by replacing the reaction reagent ethyl iodide in Example 42 with ethyl bromoacetate. ESI/MS *m*/*z* 507.2 [M + H]⁺.

### Example 47:1- (2- (1H-indol-3-yl) ethyl) -7-methoxy-6- (2- (4-methylpiperazin-1-yl) ethoxy) -2- (tetrahydro-2H-pyran-4-yl) methyl) -1,2,3,4-tetrahydroisoquinoline (A45)

Referring to the synthesis route of **A40, A45** was obtained by replacing the reaction reagent ethyl iodide in Example 42 with 1-(2-bromoethyl)-4-methylpiperazine(CAS: 801152-34-1). ESI/MS *m*/*z* 547.4 [M + H]⁺.

### Example 48:1- (2- (1H-indol-3-yl) ethyl) -7-methoxy-6- ((4-methylbenzyl) oxo) -2-((tetrahydro-2H-pyran-4-yl) methyl) -1,2,3,4-tetrahydroisoquinoline (A46)

Referring to the synthesis route of **A40, A46** was obtained by replacing the reaction reagent ethyl iodide in Example 42 with 4-methylbenzyl bromide (CAS: 104-81-4). ESI/MS *m*/*z* 525.4 [M + H]⁺.

### Example 49:1- (2- (1H-indol-3-yl) ethyl) -7-methoxy-6- (pyridin-4-ylmethoxy) -2-((tetrahydro-2H-pyran-4-yl) methyl) -1,2,3,4-tetrahydroisoquinoline (A47)

Referring to the synthesis route of **A40, A47** was obtained by replacing the reaction reagent ethyl iodide in Example 42 with 4-methylbenzyl bromide (CAS: 104-81-4). ESI/MS *m*/*z* 525.4 [M + H]⁺.

### Example 50: 1- (2- (1H-indol-3-yl) ethyl) -7-methoxy-6- (pyrimidin-5-ylmethoxy) -2- ((tetrahydro-2H-pyran-4-yl) methyl) -1,2,3,4-tetrahydroisoquinoline (A48)

Referring to the synthesis route of **A40, A48** was obtained by replacing the reaction reagent ethyl iodide in Example 42 with 5-(bromomethyl) pyrimidine (CAS: 25198-96-3). ESI/MS *m*/*z* 513.3 [M + H]⁺.

### Example 51: 1- (2- (1H-indol-3-yl) ethyl) -7-methoxy-2-((tetrahydro-2H-pyran-4-yl) methyl) -1,2,3,4-tetrahydroisoquinolin-6-yl 2,2,2-trifluoroacetate (A49)

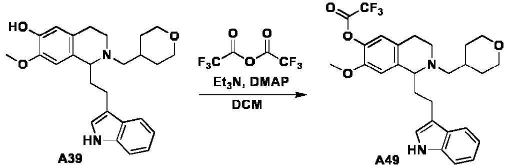

Compound **A39** (1.05 g, 2.5 mmol) was dissolved in 20 mL of dichloromethane at 0 °C, then trifluoroacetic anhydride (0.79 g, 3.75 mmol), triethylamine (0.45 g, 4.5 mmol), and N, N-dimethylaminopyridine (DMAP, 30.5 mg, 0.25 mmol) were added in sequence. The mixture was then reacted under stirring at room temperature for 6 hours. After TLC monitoring showed that the reaction was complete, the reaction solution was extracted with water and ethyl acetate; the organic phase was collected, concentrated, and then purified by column chromatography using dichloromethane/methanol=30:1 to obtain 1.04 g of **A49** with a yield of 81% as a yellow solid. ESI/MS *m*/*z* 517.2 [M + H]⁺.

### Example 52: 1- (2- (1H-indol-3-yl) ethyl) -7-methoxy-2-((tetrahydro-2H-pyran-4-yl) methyl) -1,2,3,4-tetrahydroisoquinolin-6-yl trifluoromethanesulfonic acid (A50)

Referring to the synthesis route of **A49, A50** was obtained by replacing trifluoroacetic anhydride in Example 51 with trifluoromethanesulfonic anhydride. ESI/MS *m*/*z* 553.2 [M + H]⁺.

### Example 53: 1- (2- (1H-indol-3-yl) ethyl) -7-methoxy-2-((tetrahydro-2H-pyran-4-yl) methyl) -1,2,3,4-tetrahydroisoquinolin-6-ylbenzenesulfonate (A51)

Referring to the synthesis route of **A49, A51** was obtained by replacing trifluoroacetic anhydride in Example 51 with benzenesulfonyl chloride. ESI/MS *m*/*z* 561.2 [M + H]⁺.

### Example 54:1- (2- (1H-indol-3-yl) ethyl) -7-methoxy-2- ((tetrahydro-2H-pyran-4-yl) methyl) -1,2,3,4-tetrahydroisoquinolin-6-yl 4- (trifluoromethyl) benzenesulfonate (A52)

Referring to the synthesis route of **A49, A52** was obtained by replacing trifluoroacetic anhydride in Example 51 with 4-trifluoromethylbenzenesulfonyl chloride (CAS: 2991-42-6). ESI/MS *m*/*z* 629.2 [M + H]⁺.

### Example 55: 1- (2- (1H-indol-3-yl) ethyl) -7-methoxy-6-phenoxy-2-((tetrahydro-2H-pyran-4-yl) methyl) -1,2,3,4-tetrahydroisoquinoline (A53)

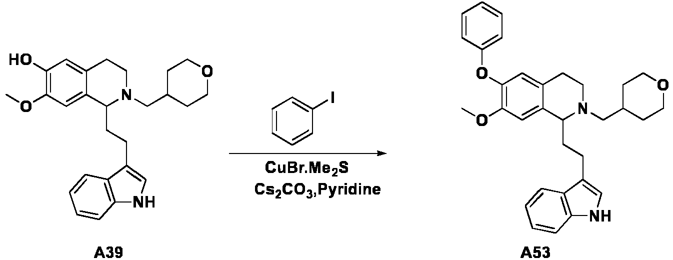

Compound **A39** (0.63 g, 1.5 mmol), iodobenzene (0.37 g, 1.8 mmol), copper(I) bromide-dimethyl sulfide (0.31 g, 1.5 mmol), and cesium carbonate (1.47 g, 4.5 mmol) were dissolved in 15 mL of pyridine, and the mixture was heated at 120 °C under argon protection for 24 hours. After TLC monitoring showed that the reaction was complete, the reaction solution was filtered, and washed with ethyl acetate; the filtrate was collected, and washed three times with water having an equal volume of the organic phase. The organic phase was concentrated, and purified by column chromatography using dichloromethane/methanol=40:1 to obtain 0.32 g of **A53** with a yield of 43% as a yellow solid. ESI/MS *m*/*z* 497.3 [M + H]⁺.

### Example 56: 2- ((1- (2- (1H-indol-3-yl) ethyl) -7-methoxy-2- ((tetrahydro-2H-pyran-4-yl) methyl) -1,2,3,4-tetrahydroisoquinolin-6-yl) oxo) ethan-1-amine (A54)

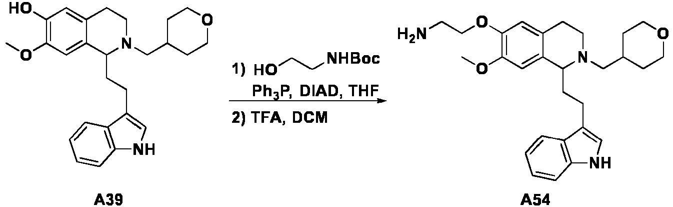

As shown in the figure above, compound **A39** (0.63 g, 1.5 mmol), N-Boc-ethanolamine (0.48 g, 3 mmol), and triphenylphosphine (1.18 g, 4.5 mmol) were dissolved in 30 mL of tetrahydrofuran. Diisopropyl azodicarboxylate (DIAD, 1.04 g, 7.5 mmol) was added dropwise while stirring at 0 °C. After addition, the mixture was transferred to room temperature and stirred for 6 hours. After TLC monitoring showed that the reaction was complete, the reaction solution was extracted by adding water and ethyl acetate; the organic phase was collected, concentrated, and then dissolved in 10 mL of dichloromethane/trifluoroacetic acid(v/v=5:1) and stirred at room temperature for 2 hours. The reaction solution was added with saturated sodium bicarbonate solution to neutralize trifluoroacetic acid, and the pH of the reaction solution was adjusted to 8. Then the reaction solution was extracted with dichloromethane; the organic phase was collected, concentrated and then purified by column chromatography using dichloromethane/methanol=10:1 to obtain 0.32 g **A54,** with a yield of 47% over two steps as a yellow oil. ESI/MS *m*/*z* 464.3 [M + H]⁺.

### Example 57: 5- (2- (5-methoxy-1H-indol-3-yl) ethyl) -6- (tetrahydro-2H-pyran-4-yl) methyl) -5,6,7,8-tetrahydro - [1,3] dioxolo [4,5-g] isoquinoline (A55)

Referring to the synthesis routes of **A3** and **A12, A55** was obtained by replacing **2-3** and **3-1** in Example 7 with homopiperonylamine and 5-methoxyindole-3-carboxaldehyde, respectively, and replacing the reaction reagent cyclohexylmethyl bromide in Example 10 with 4-bromomethyltetrahydropyran. ESI/MS *m*/*z* 449.3 [M + H]⁺.

### Example 58: 6,7-dimethoxy-1- (2- (5-methoxy-1H-indol-3-yl) ethyl) -2-(tetrahydro- 2H-pyran-4-yl) methyl) -1,2,3,4-tetrahydroisoquinoline (A56)

Referring to the synthesis routes of **A3** and **A12, A56** was obtained by replacing **2-3** and **3-1** in Example 7 with 3,4-dimethoxyphenylethylamine and 5-methoxyindole-3-carboxaldehyde, respectively, and replacing the reaction reagent cyclohexylmethyl bromide in Example 10 with 4-bromomethyltetrahydropyran. **¹H NMR** (500 MHz, CDCl₃) δ 7.94 (s, 1H), 7.28 (s, 1H), 7.09 - 6.97 (m, 2H), 6.87 (dd, *J* = 8.7, 2.5 Hz, 1H), 6.59 (s, 1H), 6.48 (s, 1H), 3.99 (dd, *J* = 10.5, 4.2 Hz, 2H), 3.87 (d, *J =* 2.4 Hz, 6H), 3.57 (s, 3H), 3.57 (s, 1H), 3.36 (dd, *J* = 30.6, 19.6 Hz, 3H), 2.96 - 2.75 (m, 4H), 2.47 (s, 3H), 2.26 - 1.98 (m, 2H), 1.79 (dd, *J* = 32.5, 13.5 Hz, 5H). **¹³C NMR** (125 MHz, CDCl₃) δ 153.8, 146.7, 131.7, 127.9, 122.0, 111.9, 111.8, 111.4, 110.6, 101.1, 68.1, 61.3, 56.0, 55.9, 55.8, 43.9, 31.9, 31.7, 22.2. ESI/MS *m*/*z* 465.2 [M + H]⁺.

### Example 59:5- (2- (5-fluoro-1H-indol-3-yl) ethyl) -6- ((tetrahydro-2H-pyran-4-yl) methyl) -5,6,7,8-tetrahydro - [1,3] dioxolo [4,5-g] isoquinoline (A57)

Referring to the synthesis routes of **A3** and **A12, A57** was obtained by replacing **2-3** and **3-1** in Example 7 with homopiperonylamine and 5-fluoroindole-3-carboxaldehyde, respectively, and replacing the reaction reagent cyclohexylmethyl bromide in Example 10 with 4-bromomethyltetrahydropyran. **¹H NMR** (500 MHz, CDCl₃) δ 7.99 (s, 1H), 7.26 (d, *J* = 3.9 Hz, 1H), 7.20 (dd, *J* = 9.7, 2.5 Hz, 1H), 7.03 (s, 1H), 6.93 (td, *J* = 9.0, 2.5 Hz, 1H), 6.53 (d, *J* = 21.4 Hz, 2H), 5.88 (s, 2H), 3.99 - 3.93 (m, 2H), 3.51 (s, 1H), 3.41 - 3.19 (m,3H), 2.83 (t, *J* = 7.9 Hz, 4H), 2.41 (d, *J* = 25.7 Hz, 3H), 2.13 - 1.93 (m, 2H), 1.81 - 1.63 (m, 5H). ESI/MS *m*/*z* 437.1 [M + H]⁺.

### Example 60: 1-1- (2- (5-fluoro-1H-indol-3-yl) ethyl) -6,7-dimethoxy-2-((tetrahydro- 2H-pyran-4-yl) methyl) -1,2,3,4-tetrahydroisoquinoline (A58)

Referring to the synthesis routes of **A3** and **A12, A58** was obtained by replacing **2-3** and **3-1** in Example 7 with 3,4-dimethoxyphenethylamine and 5-fluoroindole-3-carboxaldehyde, respectively, and replacing the reaction reagent cyclohexylmethyl bromide in Example 10 with 4-bromomethyltetrahydropyran. ESI/MS *m*/*z* 437.1 [M + H]⁺.

### Example 61: 5- (2- (5-Chloro-1H-indol-3-yl) ethyl) -6- ((tetrahydro-2H-pyran-4-yl) methyl) -5,6,7,8-tetrahydro - [1,3] dioxolo [4,5-g] isoquinoline (A59)

Referring to the synthesis routes of **A3** and **A12, A59** was obtained by replacing **2-3** and **3-1** in Example 7 with homopiperonylamine and 5-chloroindole-3-carboxaldehyde, respectively, and replacing the reaction reagent cyclohexylmethyl bromide in Example 10 with 4-bromomethyltetrahydropyran. ESI/MS *m*/*z* 453.2 [M + H]⁺.

### Example 62: 1- (2- (5-Chloro-1H-indol-3-yl) ethyl) -6,7-dimethoxy-2- ((tetrahydro-2H-pyran-4-yl) methyl) -1,2,3,4-tetrahydroisoquinoline (A60)

Referring to the synthesis routes of **A3** and **A12, A60** was obtained by replacing **2-3** and **3-1** in Example 7 with 3,4- dimethoxyphenethylamine and 5-chloroindole-3-carboxaldehyde, respectively, and replacing the reaction reagent cyclohexylmethyl bromide in Example 10 with 4-bromomethyltetrahydropyran. ESI/MS *m*/*z* 469.1 [M + H]⁺.

### Example 63: 1- (2- (5-bromo-1H-indol-3-yl) ethyl) -6- ((tetrahydro-2H-pyran-4-yl) methyl) -5,6,7,8-tetrahydro - [1,3] dioxolo [4,5-g] isoquinoline (A61)

Referring to the synthesis routes of **A3** and **A12, A61** was obtained by replacing **2-3** and **3-1** in Example 7 with 3,4 - dimethoxyphenethylamine and 5-bromoindole-3-carboxaldehyde, respectively, and replacing the reaction reagent cyclohexylmethyl bromide in Example 10 with 4-bromomethyltetrahydropyran. ESI/MS *m*/*z* 513.1 [M + H]⁺.

### Example 64: 5- (2- (5-methyl-1H-indol-3-yl) ethyl) -6- (tetrahydro-2H-pyran-4-yl) methyl) -5,6,7,8-tetrahydro - [1,3] dioxolo [4,5-g] isoquinoline (A62)

Referring to the synthesis routes of **A3** and **A12, A62** was obtained by replacing **2-3** and **3-1** in Example 7 with homopiperonylamine and 5-methylindole-3-carboxaldehyde, respectively, and replacing the reaction reagent cyclohexylmethyl bromide in Example 10 with 4-bromomethyltetrahydropyran. ESI/MS *m*/*z* 449.1 [M + H]⁺.

### Example 65: 6,7-dimethoxy-1- (2- (5-methyl-1H-indol-3-yl) ethyl) -2- ((tetrahydro-2H-pyran-4-yl) methyl) -1,2,3,4-tetrahydroisoquinoline (A63)

Referring to the synthesis routes of **A3** and **A12, A63** was obtained by replacing **2-3** and **3-1** in Example 7 with 3,4 - dimethoxyphenethylamine and 5-methylindole-3-carboxaldehyde, respectively, and replacing the reaction reagent cyclohexylmethyl bromide in Example 10 with 4-bromomethyltetrahydropyran. ESI/MS *m*/*z* 449.1 [M + H]⁺.

### Example 66: 6,7-dimethoxy-1- (2- (7-methyl-1H-indol-3-yl) ethyl) -2- ((tetrahydro-2H-pyran-4-yl) methyl) -1,2,3,4-tetrahydroisoquinoline (A64)

Referring to the synthesis routes of **A3** and **A12, A64** was obtained by replacing **2-3** and **3-1** in Example 7 with 3,4 - dimethoxyphenethylamine and 7-methylindole-3-carboxaldehyde, respectively, and replacing the reaction reagent cyclohexylmethyl bromide in Example 10 with 4-bromomethyltetrahydropyran. ESI/MS *m*/*z* 449.1 [M + H]⁺.

### Example 67: 5- (2- (5- (benzyloxy) -1H-indol-3-yl) ethyl) -6- ((tetrahydro-2H-pyran-4-yl) methyl) -5,6,7,8-tetrahydro - [1,3] dioxolo [4,5-g] isoquinoline (A65)

Referring to the synthesis routes of **A3** and **A12, A65** was obtained by replacing **2-3** and **3-1** in Example 7 with homopiperonylamine and 5-(phenylmethoxy)-1H-indole -3-carbaldehyde, respectively, and replacing the reaction reagent cyclohexylmethyl bromide in Example 10 with 4-bromomethyltetrahydropyran. ESI/MS *m*/*z* 525.2 [M + H]⁺.

### Example 68: 1- (2- (5- (benzyloxy) -1H-indol-3-yl) ethyl) -6,7-dimethoxy-2-((tetrahydro-2H-pyran-4-yl) methyl) -1,2,3,4-tetrahydroisoquinoline (A66)

Referring to the synthesis routes of **A3** and **A12, A66** was obtained by replacing **2-3** and **3-1** in Example 7 with 3,4-dimethoxyphenylethylamine and 5-(phenylmethoxy)-1H-indole -3-carbaldehyde, respectively, and replacing the reaction reagent cyclohexylmethyl bromide in Example 10 with 4-bromomethyltetrahydropyran. ESI/MS *m*/*z* 541.3 [M + H]⁺.

### Example 69: 3- (2- (6- ((tetrahydro-2H-pyran-4-yl) methyl) -5,6,7,8-tetrahydro - [1,3] dioxolo [4,5-g] isoquinolin-5-yl) ethyl) -1H-indole-5-phenol (A67)

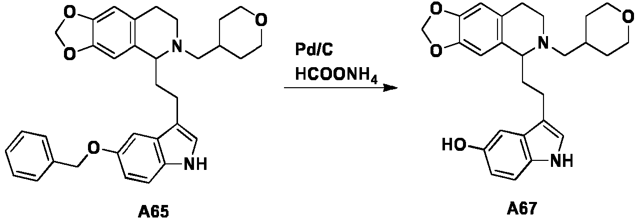

**A65** (262 mg, 0.5 mmol) was dissolved in 10 mL of methanol, and then 26 mg of palladium on carbon catalyst (containing 10% water) and ammonium formate (315 mg, 5 mmol) were added. The mixture was heated at 55 °C overnight, and then filtered. The filtrate was collected, concentrated, and then purified by column chromatography using dichloromethane/methanol=20:1 to obtain **A67** as a yellow solid. ESI/MS *m*/*z* 435.2 [M + H]⁺.

### Example 70: 3- (2- (6,7-dimethoxy-2- ((tetrahydro-2H-pyran-4-yl) methyl) -1,2,3,4-tetrahydroisoquinolin-1-yl) ethyl) -1H-indole-5-phenol (A68)

Referring to the synthesis route of **A67, A68** was obtained by replacing the raw material **A65** in Example 69 with **A66.** ESI/MS *m*/*z* 450.1 [M + H]⁺.ESI/MS *m*/*z* 451.1 [M + H]⁺.

### Example 71: 6,7-dimethoxy-1- (2- (1-methyl-1H-indol-3-yl) ethyl) -2- ((tetrahydro-2H-pyran-4-yl) methyl) -1,2,3,4-tetrahydroisoquinoline (A69)

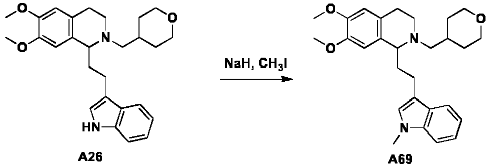

Compound **A26** (1.30 g, 3.0 mmol) was dissolved in ultra-dry DMF at 0 ° C under an ice bath, and sodium hydride (1.5 equivalents) was added. The mixture continued stirring for 15 minutes in the ice bath, and then the reaction system was added with iodine methane in tetrahydrofuran solution dropwise. After addition, the reaction was protected with argon gas, and then transferred to room temperature and stirred for 4 hours.

After TLC monitoring showed that the reaction was complete, the reaction solution was quenched by adding excess water and extracted with ethyl acetate; the organic phase was washed once with saturated sodium bicarbonate solution and onece with saturated sodium chloride solution, concentrated and then purified by column chromatography using dichloromethane/methanol=20:1 to obtain 1.13 g **A69** with a yield of 85% as a yellow solid. **¹H NMR** (400 MHz, CDCl₃) δ 7.58 (d, *J* = 7.8 Hz, 1H), 7.18 (d, *J* = 8.1 Hz, 1H), 7.15- 7.11 (m, 1H), 7.05 - 7.01 (m, 1H), 6.95 (s, 1H), 6.49 (s, 1H), 6.30 (s, 1H), 4.08 - 3.97 (m, 2H), 3.57 (s, 1H), 3.41 (td, *J =* 11.4, 4.4 Hz, 2H), 3.32 (dd, *J =* 19.4, 10.0 Hz, 1H), 3.35 (s, 3H), 3.02 - 2.77 (m, 4H), 2.59 - 2.37 (m, 3H), 2.04 - 2.17 (m, 2H), 1.76 - 1.85 (m, 3H), 1.36 - 1.26 (m, 2H). **¹³C NMR** (125 MHz, CDCl₃): δ 145.2, 137.5, 131.9, 125.6, 123.5, 122.0, 121.0, 119.1, 119.0, 117.0, 111.4, 111.1, 110.7, 62.1, 55.9, 55.8, 47.5, 42.0, 36.8, 33.8, 32.0, 31.7, 29.7, 23.7, 22.1. ESI/MS *m*/*z* 449.3 [M + H]⁺.

### Example 72: (2- (6,7-dimethoxy-2- ((tetrahydro-2H-pyran-4-yl) methyl) -1,2,3,4-tetrahydroisoquinolin-1-yl) ethyl) -1H-indol-1-yl) methanol (A70)

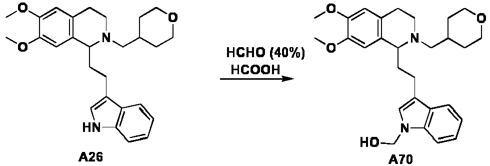

Compound **A21** (130 mg, 0.3 mmol) was dissolved in 5 mL of a mixed solvent of formic acid/40% formaldehyde aqueous solution (v/v=3:2) under an ice bath, and the mixture was heated and stirred at 90 °C for 2 hours under argon protection. After TLC monitoring showed that the reaction was complete, the reaction was quenched by adding excess saturated sodium bicarbonate solution, and extracted with ethyl acetate. The organic phase was concentrated and then purified by column chromatography using dichloromethane/methanol=20:1 to afford 1.13 g of **A70** with a yield of 73% as light yellow solid. **¹H NMR** (400 MHz, CDCl₃) δ 7.58 (d, *J =* 7.8 Hz, 1H), 7.18 (d, *J =* 8.1 Hz, 1H), 7.16-7.10 (m, 1H), 7.05 - 7.01 (m, 1H), 6.95 (s, 1H), 6.49 (s, 1H), 6.30 (s, 1H), 4.18 - 3.97 (m, 2H), 3.55 (s, 1H), 3.40 (td, *J =* 11.4, 4.4 Hz, 2H), 3.37 (d, 2H), 3.32 (dd, *J* = 19.4, 10.0 Hz, 1H), 3.02 - 2.77 (m, 4H), 2.59 - 2.37 (m, 3H), 2.04 - 2.17 (m, 2H), 1.76 - 1.85 (m, 3H), 1.36 - 1.26 (m, 2H). **¹³C NMR** (125 MHz, CDCl₃): δ 145.2, 137.5, 131.9, 125.6, 123.5, 122.0, 121.0, 119.1, 119.0, 117.0, 111.4, 111.1, 110.7, 62.1, 55.9, 55.8, 47.5, 42.0, 36.8, 33.8, 32.0, 31.7, 29.7, 23.7, 22.1. ESI/MS *m*/*z* 465.1 [M + H]⁺.

### Example 73: Ethyl 2- (3- (2- (6,7-dimethoxy-2- ((tetrahydro-2H-pyran-4-yl) methyl) -1,2,3,4-tetrahydroisoquinolin-1-yl) ethyl) -1H-indol-1-yl) acetate (A71)

Referring to the synthesis route of **A69, A71** was obtained by replacing iodomethane in Example 71 with ethyl 2-bromoacetate. ESI/MS *m*/*z* 521.1 [M + H]⁺.

### Example 74: 2- (3- (2- (6,7-dimethoxy-2- ((tetrahydro-2H-pyran-4-yl) methyl) -1,2,3,4-tetrahydroisoquinolin-1-yl) ethyl) -1H-indol-1-yl) acetic acid (A72)

100 mg of compound **A71** was dissolved in 10 mL of a mixed solution of EtOH/2N NaOH (v/v=1:1), and the resulting mixture was stirred at room temperature for 1 hour. After TLC monitoring showed that the raw material was consumed, the reaction was spin-dried to remove the organic solvent, and adjusted to pH 4-5 with 1N HCl, extracted with dichloromethane, concentrated, and recrystallized with ethyl acetate and n-hexane to obtain **A72.**

### Example 75: 2- (3- (2- (6,7-dimethoxy-2- ((tetrahydro-2H-pyran-4-yl) methyl) -1,2,3,4-tetrahydroisoquinolin-1-yl) ethyl) -1H-indol-1-yl) acetamide (A73)

100 mg of compound **A72** was dissolved in 10 mL of 1,4-dioxane, and the mixture was added with 1 mL of pyridine (5 equiv.), 1.2 mL of tert butyl dicarbonate (2 equiv.), and 2.1 g of ammonium bicarbonate (10 equiv.), and heated at 50 ° C for 12 hours. After TLC monitoring showed that the raw material was consumed, the reaction solution was added with 50 mL of water, and extracted three times with ethyl acetate. The organic phases were combined, washed with water and saturated sodium chloride in sequence, dried over anhydrous sodium sulfate, evaporated to remove the solvent, and purified by column chromatography using dichloromethane/methanol=20:1 to obtain **A73.**

### Example 76: 2- (3- (2- (6,7-dimethoxy-2- ((tetrahydro-2H-pyran-4-yl) methyl) -1,2,3,4-tetrahydroisoquinolin-1-yl) ethyl) -1H-indol-1-yl) ethan-1-ol (A74)

Referring to the synthesis route of **A69, A74** was obtained by replacing iodomethane in Example 71 with 2-iodoethanol (CAS: 624-76-0). ESI/MS *m*/*z* 521.1 [M + H]⁺.

Referring to the synthesis route of **A65, A74** was obtained by replacing iodomethane in Example 65 with 2-iodoethanol (CAS: 624-76-0) and purified by column chromatography using dichloromethane/methanol=20:1 to afford a white solid. ESI/MS *m*/*z* 479.3 [M + H]⁺.

### Example 77: 6,7-dimethoxy-1- (2- (1- (2- (methylsulfonyl) ethyl) -1H-indol-3-yl) ethyl) -2- (tetrahydro-2H-pyran-4-yl) methyl) -1,2,3,4-tetrahydroisoquinoline (A75)

Referring to the synthesis route of **A69, A75** was obtained by replacing iodomethane in Example 71 with 1-bromo-2-(methylsulfonyl)-ethane (CAS: 16523-02-7). ESI/MS *m*/*z* 541.3 [M + H]⁺.

### Example 78:2- (3- (2- (6,7-dimethoxy-2- ((tetrahydro-2H-pyran-4-yl) methyl) -1,2,3,4-tetrahydroisoquinolin-1-yl) ethyl) -1H-indol-1-yl) - N, N-dimethylethan-1-amine (A76)

Referring to the synthesis route of **A69, A76** was obtained by replacing iodomethane in Example 71 with (2-bromoethyl)dimethylamine (CAS:5459-68-7). ESI/MS *m*/*z* 506.1 [M + H]⁺.

### Example 79: 6,7-dimethoxy-1- (2- (2-methyl-1H-indol-3-yl) ethyl) -2- ((tetrahydro-2H-pyran-4-yl) methyl) -1,2,3,4-tetrahydroisoquinoline (A77)

Referring to the synthesis routes of **A3** and **A12, A77** was obtained by replacing **2-3** and **3-1** in Example 7 with 3,4-dimethoxyphenethylamine (CAS: 120-20-7) and 2-methylindole-3-carboxaldehyde (CAS: 5416-80-8), respectively, and replacing the reaction reagent cyclohexylmethyl bromide in Example 10 with 4-bromomethyltetrahydropyran (CAS: 125552-89-8). ESI/MS *m*/*z* 449.1 [M + H]⁺.

### Example 80: 1- (2- (1H-pyrrolo [2,3-b] pyridin-3-yl) ethyl) -6,7-dimethoxy-2-((tetrahydro-2H-pyran-4-yl) methyl) -1,2,3,4-tetrahydroisoquinoline (A78)

Referring to the synthesis route of **A3** and **A12, A78** was obtained by replacing **2-3** and **3-1** in Example 7 with 3,4-dimethoxyphenethylamine (CAS: 120-20-7) and 7-azaindole-3-carboxaldehyde (CAS: 4649-09-6), respectively, and replacing the reaction reagent cyclohexylmethyl bromide in Example 10 with 4-bromomethyltetrahydropyran (CAS: 125552-89-8). ESI/MS *m*/*z* 436.2 [M + H]⁺.

### Example 81: 1- (2- (1H-pyrrolo [3,2-b] pyridin-3-yl) ethyl) -6,7-dimethoxy-2-((tetrahydro-2H-pyran-4-yl) methyl) -1,2,3,4-tetrahydroisoquinoline (A79)

Referring to the synthesis route of **A3** and **A12, A79** was obtained by replacing **2-3** and **3-1** in Example 7 with 3,4-dimethoxyphenethylamine (CAS: 120-20-7) and 4-azaindole-3-carboxaldehyde (CAS: 276862-85-2), respectively, and replacing the reaction reagent cyclohexylmethyl bromide in Example 10 with 4-bromomethyltetrahydropyran (CAS: 125552-89-8). ESI/MS *m*/*z* 436.2 [M + H]⁺.

### Example 82: 5- (2- (benzo [b] thiophen-3-yl) ethyl) -6- ((tetrahydro-2H-pyran-4-yl) methyl) -5,6,7,8-tetrahydro - [1,3] dioxolo [4,5-g] isoquinoline (A80)

Referring to the synthesis route of **A3** and **A12, A80** was obtained by replacing **2-3** and **3-1** in Example 7 with homopiperonylamine (CAS: 1484-85-1) and 2-thiophenecarboxaldehyde (CAS: 98-03-3), respectively, and replacing the reaction reagent cyclohexylmethyl bromide in Example 10 with 4-bromomethyltetrahydropyran (CAS: 125552-89-8). ESI/MS *m*/*z* 436.1 [M + H]⁺.

### Example 83: 6- (benzyloxy) -7-methoxy-1- (2- (5-methoxy-1H-indol-3-yl) ethyl) -2-((tetrahydro-2H-pyran-4-yl) methyl) -1,2,3,4-tetrahydroisoquinoline (A81)

Referring to the synthesis route of **A3** and **A12, A81** was obtained by replacing the reaction reagent cyclohexylmethyl bromide in Example 10 with 4-bromomethyltetrahydropyran (CAS: 125552-89-8) and undergoing a substitution reaction with **A3.** ESI/MS *m*/*z* 541.1 [M + H]⁺.

### Example 84: 6- (benzyloxy) -1- (2- (5-fluoro-1H-indol-3-yl) ethyl) -7-methoxy-2-((tetrahydro-2H-pyran-4-yl) methyl) -1,2,3,4-tetrahydroisoquinoline (A82)

Referring to the synthesis routes of **A3** and **A12, A82** was obtained by replacing **3-1** in example 7 with 5-fluoroindole-3-carboxaldehyde, and replacing the reaction reagent cyclohexylmethyl bromide in Example 10 with 4-bromomethyltetrahydropyran (CAS: 125552-89-8) for the last substitution reaction. ESI/MS *m*/*z* 529.2 [M + H]⁺.

### Example 85: 6- (benzyloxy) -1- (2- (5-chloro-1H-indol-3-yl) ethyl) -7-methoxy-2-((tetrahydro-2H-pyran-4-yl) methyl) -1,2,3,4-tetrahydroisoquinoline (A83)

Referring to the synthesis routes of **A3** and **A12, A83** was obtained by replacing **3-1** in example 7 with 5-chloroindole-3-carboxaldehyde, and replacing the reaction reagent cyclohexylmethyl bromide in Example 10 with 4-bromomethyltetrahydropyran (CAS: 125552-89-8) for the last substitution reaction. ESI/MS *m*/*z* 545.3 [M + H]⁺.

### Example 86: 6- (benzyloxy) -7-methoxy-1- (2- (5-methoxy-1H-indol-3-yl) ethyl) -2-(piperidin-4-ylmethyl) -1,2,3,4-tetrahydroisoquinoline (A84)

Referring to the synthesis routes of **A3** and **A12, A84** was obtained by replacing **3-1** in example 7 with 5-methoxyindole-3-carboxaldehyde, and replacing the reaction reagent cyclohexylmethyl bromide in Example 10 with 1-Boc-4-bromomethylpiperidine (CAS: 125552-89-8) for the substitution reaction and undergoing the last hydrolysis reaction. ESI/MS *m*/*z* 540.3 [M + H]⁺.

### Example 87: 6- (benzyloxy) -1- (2- (5-fluoro-1H-indol-3-yl) ethyl) -7-methoxy-2-(piperidin-4-ylmethyl) -1,2,3,4-tetrahydroisoquinoline (A85)

Referring to the synthesis routes of **A3** and **A12, A85** was obtained by replacing **3-1** in example 7 with 5-fluoroindole-3-carboxaldehyde, and replacing the reaction reagent cyclohexylmethyl bromide in Example 10 with 1-Boc-4-bromomethylpiperidine (CAS: 125552-89-8) for substitution reaction and undergoing the last hydrolysis reaction. ESI/MS *m*/*z* 528.1 [M + H]⁺.

### Example 88: 1- (2- (1H-indol-3-yl) ethyl) -6- (benzyloxy) -7-methoxy-2-(piperidin-4-ylmethyl) -1,2,3,4-tetrahydroisoquinoline (A86)

Referring to the synthesis routes of **A3** and **A12, A86** was obtained by replacing **3-3** in example **7** with 3-indolepropionic acid, and replacing the reaction reagent cyclohexylmethyl bromide in Example 10 with 1-Boc-4-bromomethylpiperidine (CAS: 125552-89-8) for substitution reaction and undergoing the last hydrolysis reaction. ESI/MS *m*/*z* 510.0 [M + H]⁺.

### Example 89: 1- (2- (1H-indol-3-yl) ethyl) -6,7-dimethoxy-2- (piperidin-4-ylmethyl) -1,2,3,4-tetrahydroisoquinoline (A87)

Referring to the synthesis routes of **A3** and **A12, A87** was obtained by replacing **2-3** and **3-3** in example 7 with 3,4-dimethoxyphenethylamine(CAS: 120-20-7) and 3-indolepropionic acid, and replacing the reaction reagent cyclohexylmethyl bromide in Example 10 with 1-Boc-4-bromomethylpiperidine (CAS: 125552-89-8) for substitution reaction and undergoing the last hydrolysis reaction. ESI/MS *m*/*z* 434.4 [M + H]⁺.

### Example 90: 5- (2- (5-fluoro-1H-indol-3-yl) ethyl) -6- (piperidin-4-ylmethyl) -5,6,7,8-tetrahydro - [1,3] dioxolo [4,5-g] isoquinoline (A88)

Referring to the synthesis routes of **A3** and **A12, A88** was obtained by replacing **2-3** and **3-1** in example 7 with homopiperonylamine (CAS: 120-20-7) and 5-fluoroindole-3-carboxaldehyde, and replacing the reaction reagent cyclohexylmethyl bromide in Example 10 with 1-Boc-4-bromomethylpiperidine (CAS: 125552-89-8) for substitution reaction and undergoing the last hydrolysis reaction. ESI/MS *m*/*z* 436.1 [M + H]⁺.

### Example 91: 5- (2- (5-methoxy-1H-indol-3-yl) ethyl) -6- (piperidin-4-ylmethyl) -5,6,7,8-tetrahydro - [1,3] dioxolo [4,5-g] isoquinoline (A89)

Referring to the synthesis routes of **A3** and **A12, A89** was obtained by replacing **2-3** and **3-1** in example 7 with homopiperonylamine (CAS: 120-20-7) and 5-methoxyindole-3-carboxaldehyde, and replacing the reaction reagent cyclohexylmethyl bromide in Example 10 with 1-Boc-4-bromomethylpiperidine (CAS: 125552-89-8) for substitution reaction and undergoing the last hydrolysis reaction. ESI/MS *m*/*z* 448.2 [M + H]⁺.

### Example 92: 5- (2- (5-Chloro-1H-Indole-3-yl) ethyl) -6- (piperidin-4-ylmethyl) -5,6,7,8-tetrahydro-1,3-dioxolo [4,5-g] isoquinoline (A90)

Referring to the synthesis routes of **A3** and **A12, A90** was obtained by replacing **2-3** and **3-1** in example 7 with homopiperonylamine (CAS: 120-20-7) and 5-chloroindole-3-carboxaldehyde, and replacing the reaction reagent cyclohexylmethyl bromide in Example 10 with 1-Boc-4-bromomethylpiperidine (CAS: 125552-89-8) for substitution reaction and undergoing the last hydrolysis reaction. ESI/MS *m*/*z* 452.2 [M + H]⁺.

### Example 93: (S) -1- (2- (1H-indol-3-yl) ethyl) -6,7-dimethoxy-2-((tetrahydro-2H-pyran-4-yl) methyl) -1,2,3,4-tetrahydroisoquinoline ((S) - A26)

Referring to the synthesis route of **A12,** taking **(*S*)** - **A2** as the raw material, **(*S*)** - **A26** was obtained as a light yellow solid by replacing cyclohexylmethyl bromide in Example 11 with 4-(bromomethyl)tetrahydropyran (CAS: 125552-89-8) and undergoing a substitution reaction with **(*S*)** - **A2. ¹H NMR** (400 MHz, CDCl₃) δ 8.06 (brs, 1H), 7.63 (d, J=7.8 Hz, 1H), 7.38 (d, J=8.1 Hz, 1H), 7.24-7.18 (m, 1H), 7.16-7.10 (m, 1H), 7.02 (s, 1H), 6.59 (s, 1H), 6.49 (s, 1H), 4.07-3.97 (m, 2H), 3.87 (s, 3H), 3.79 (s, 3H), 3.57 (s, 1H), 3.41 (td, J=11.4, 4.4 Hz, 2H), 3.32 (s, 1H) dd, J=19.4, 10.0 Hz, 1H), 3.02-2.77 (m, 4H), 2.59-2.37 (m, 3H), 2.04-2.17 (m, 2H), 1.76-1.85 (m, 3H), 1.36-1.26 (m, 2H). **¹³C NMR** (125 MHz, CDCl₃): δ 147.2, 136.5, 130.9, 127.6, 126.6, 122.0, 121.0, 119.1, 119.0, 117.0, 111.4, 111.1, 110.7, 68.1, 61.4, 60.1, 55.9, 55.8, 44.0, 36.8, 33.8, 32.0, 31.7. 29.7, 23.7, 22.1. ESI/MS *m*/*z* 435.3 [M+H]⁺

### Example 94: (R) -1- (2- (1H-indol-3-yl) ethyl) -6,7-dimethoxy-2-((tetrahydro-2H-pyran-4-yl) methyl) -1,2,3,4-tetrahydroisoquinoline ((R) - A26)

Referring to the synthesis route of **A12,** taking **(*R*)** - **A2** as the raw material, **(*R*)** - **A26** was obtained as a light yellow solid by replacing cyclohexylmethyl bromide in Example 12 with 4-(bromomethyl)tetrahydropyran (CAS: 125552-89-8) and undergoing a substitution reaction with **(*R*)** - **A2. ¹H NMR** (400 MHz, CDCl₃) δ 8.06 (brs, 1H), 7.63 (d, J=7.8 Hz, 1H), 7.38 (d, J=8.1 Hz, 1H), 7.24-7.18 (m, 1H), 7.16-7.10 (m, 1H), 7.02 (s, 1H), 6.59 (s, 1H), 6.49 (s, 1H), 4.07-3.97 (m, 2H), 3.87 (s, 3H), 3.79 (s, 3H), 3.57 (s, 1H), 3.41 (td, J=11.4, 4.4 Hz, 2H), 3.32 (s, 1H) dd, J=19.4, 10.0 Hz, 1H), 3.02-2.77 (m, 4H), 2.59-2.37 (m, 3H), 2.04-2.17 (m, 2H), 1.76-1.85 (m, 3H), 1.36-1.26 (m, 2H). **¹³C NMR** (125 MHz, CDCl₃): δ 147.2, 136.5, 130.9, 127.6, 126.6, 122.0, 121.0, 119.1, 119.0, 117.0, 111.4, 111.1, 110.7, 68.1, 61.4, 60.1, 55.9, 55.8, 44.0, 36.8, 33.8, 32.0, 31.7. 29.7, 23.7, 22.1. ESI/MS *m*/*z* 435.3 [M+H]⁺

### Example 95: 1- (2- (1H-indol-3-yl) ethyl) -2- ((3-oxaspiro [5.5] undecane-9-yl) methyl) -6,7-dimethoxy-1,2,3,4-tetrahydroisoquinoline (A93)

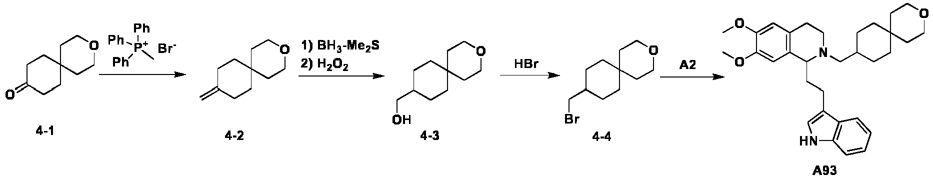

Referring to the synthesis route of **A12,** taking 3-oxaspiro[5.5]undecan-9-one(4-1, CAS: 1159280-53-1) and **A2** as the raw materials. Methyltriphenylphosphonium bromide (12 mmoL) was dissolved in 50 mL of tetrahydrofuran and the mixture was evacuated and protected with argon gas. Potassium tert butoxide solution (1 mol/L) was slowly added dropwise at 0 ° C. After addition, the mixture was stirred at 0 ° C for 1 hour, and then slowly added dropwise with a tetrahydrofuran solution of 3-oxaspiro[5.5]undecan-9-one. After dripping, the mixture was transferred to room temperature and reacted overnight. After TLC monitoring showed that the reaction was complete, the reaction was quenched by adding saturated ammonium chloride solution dropwise at 0 °C, filtered through diatomaceous earth, extracted by adding ethyl acetate, dried over anhydrous sodium sulfate, concentrated, and subjected to column chromatography using PE/EA (10/1) to obtain intermediate **4-2.** Intermediate **4-2** (6 mmol) was dissolved in tetrahydrofuran, and the resulting mixture was slowly added with borane-methyl sulfide solution (9 mmol) dropwise at 0 °C and under argon protection, and then transferred to room temperature and reacted for 4 hours. The reaction solution was then added with NaOH solution dropwise to quench the reaction until no bubbles appeared and stirred for 15 minutes, then added with 30% H₂O₂, and continued to react at room temperature for 2 hours. After TLC monitoring showed that the reaction was complete, the reaction solution was extracted with ethyl acetate, dried over anhydrous sodium sulfate, concentrated, and subjected to column chromatography using PE/EA (5/1) to afford intermediate **4-3.** Intermediate **4-3** (5 mmol) was dissolved in 20 mL of a mixed solution of 48% hydrogen bromide/ethanol (v/v=1:1), and the resulting mixture was heated at 90 ° C for 2 hours. After TLC monitoring showed that the reaction was complete, the mixture was neutralized with saturated sodium bicarbonate, extracted with ethyl acetate, dried over anhydrous sodium sulfate, concentrated, and subjected to column chromatography using PE/EA (10/1) to afford intermediate **4-4. A2** (3 mol) was dissolved in 15 mL of acetonitrile, added with **4-4** (4.5 mol) and potassium carbonate (15 mol), the mixture was heated and refluxed for 24 hours. After TLC monitoring showed that the reaction was complete, the mixture was filtered and the filtrate was collected, concentrated, and subjected to column chromatography using DCM/MeOH (30/1) to afford compound **A93.** ESI/MS *m*/*z* 503.4 [M + H]⁺.

### Example 96: 9- ((1- (2- (1H-indol-3-yl) ethyl) -6,7-dimethoxy-3,4-dihydroisoquinolin -2 (1H) - yl) methyl) -3-azaspiro [5.5] undecane (A94)

Referring to the synthesis route of **A93, A94** was obtained by taking 3-azaspiro[5.5]undecan-9-one (CAS: 1056629-32-3) and **A2** as starting materials. ESI/MS *m*/*z* 502.4 [M + H]⁺.

### Example 97: 1- (2- (1H-indol-3-yl) ethyl) -2- (((1R, 5S) -8-oxabicyclo [3.2.1] octan-3-yl) methyl) -6,7-dimethoxy-1,2,3,4-tetrahydroisoquinoline (A95)

Referring to the synthesis route of **A93, A95** was obtained by taking 8-oxabicyclo[3.2.1]octan-3-one (CAS: 77745-32-5) and **A2** as starting materials. ESI/MS *m*/*z* 461.3 [M + H]⁺.

### Example 98: 1- (2- (1H-indol-3-yl) ethyl) -2- (((1R, 5S) -8-oxabicyclo [3.2.1] octan-3-yl) methyl) -6,7-dimethoxy-1,2,3,4-tetrahydroisoquinoline (A96)

Referring to the synthesis route of **A93, A96** was obtained by taking nortropinone (CAS: 5632-84-8) and **A2** as starting materials. ESI/MS *m*/*z* 460.4 [M + H]⁺.

### Example 99: 1- (2- (1-phenylmethyl-1H-indol-3-yl) ethyl) -6,7-dimethoxy-2-((tetrahydro-2H-pyran-4-yl) methyl) -1,2,3,4-tetrahydroisoquinoline (A97)

Using compound **A26** as the synthetic intermediate and following the synthetic route of **A69, A97** was obtained by replacing methyl iodide with benzyl bromide and undergoing a substitution reaction at position 1 of indole. ESI/MS *m*/*z* 525.3 [M + H]⁺.

### Example 100: 1- (2- (1- (4-fluorobenzyl) -1H-indol-3-yl) ethyl) -6,7-dimethoxy-2-((tetrahydro-2H-pyran-4-yl) methyl) -1,2,3,4-tetrahydroisoquinoline (A98)

Using compound **A26** as the synthetic intermediate and following the synthetic route of **A69, A98** was obtained by replacing methyl iodide with 4-fluorobenzyl bromide and undergoing a substitution reaction at position 1 of indole. ESI/MS *m*/*z* 543.3 [M + H]⁺.

### Example 101: 1- (2- (5-fluoro-1H-indol-3-yl) ethyl) -6- ((4-fluorobenzyl) oxo) -7-(2,2,2-trifluoroethoxy) -1,2,3,4-tetrahydroisoquinoline (A99)

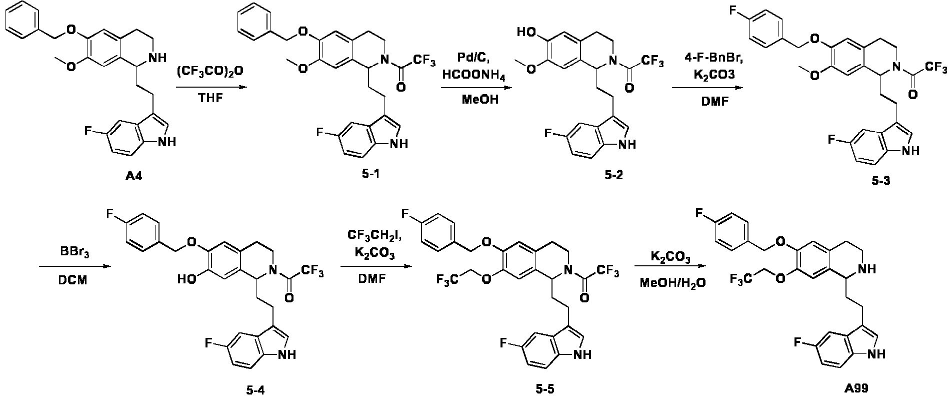

Taking compound **A4** as the synthetic intermediate. **A4** (430 mg, 1.0 mmol) was dissolved in 50 mL of tetrahydrofuran. The mixture was added slowly dropwise with trifluoroacetic anhydride (420 mg, 2.0 mmol) at room temperature. After the dropwise addition was complete, the mixture was transferred to 60 °C and stirred for 2 hours. After TLC monitoring showed that the reaction was complete, a saturated solution of sodium bicarbonate was slowly added dropwise to the above solution until no bubbles were generated. After dripping, the mixture was transferred to a separatory funnel, extracted twice with ethyl acetate and water, and washed once with saturated saline solution, dried over anhydrous sodium sulfate, and concentrated to afford compound **5-1** without passing through a column (500mg, yield 95%).

**5-1** (500 mg, 0.95 mmol) was dissolved in 50 mL of methanol, and 50 mg of palladium carbon (containing 10% water) and ammonium formate (599 mg, 9.5 mmol) were added. The mixture was heated and reacted at 60 ° C under argon protection for 4 hours. After TLC monitoring showed that the reaction was complete, the mixture was filtered to remove the palladium carbon, then extracted twice by adding water and ethyl acetate, washed once with saturated saline solution, dried over anhydrous sodium sulfate, and concentrated to afford compound **5-2** without passing through a column (348 mg, yield 84%).

**5-2** (348 mg, 0.8 mmol) was dissolved in 40 mL DMF, and 4-fluorobenzyl bromide (181 mg, 0.96 mmol) and potassium carbonate (552 mg, 4.0 mmol) were added. The mixture was heated and reacted at 60 ° C for 2 hours. After TLC monitoring showed that the reaction was complete, the reaction solution was extracted twice by adding water and ethyl acetate, washed once with saturated saline, dried over anhydrous sodium sulfate, concentrated, and recrystallized with ethyl acetate/petroleum ether (1/2, v/v) to obtain a yellow compound **5-3** (381 mg, yield 88%).

**5-3** (381 mg, 0.7 mmol) was dissolved in 40 mL of ultra-dry dichloromethane, and 1.0 M BBr₃ solution (4.2 mL, 4.2 mmol) was added slowly dropwise at -20 °C. The reaction was stirred at the same temperature for 2 hours. After TLC monitoring showed that the reaction was complete, the reaction was quenched by slowly adding water, extracted twice by adding water and dichloromethane, washed once with saturated saline solution, dried over anhydrous sodium sulfate, concentrated, and separated by column chromatography using ethyl acetate/petroleum ether (1/3, v/v) to obtain a yellow compound **5-4** (267 mg, yield 72%).

**5-4** (267 mg, 0.5 mmol) was dissolved in 30 mL DMF, and 2-iodo-1,1,1-trifluoroethane (158 mg, 0.75 mmol) and potassium carbonate (345 mg, 2.5 mmol) were added. The mixture was heated and reacted at 60 ° C for 2 hours. After TLC monitoring showed that the reaction was complete, the reaction was extracted twice by adding water and ethyl acetate, washed once with saturated saline water, dried over anhydrous sodium sulfate, concentrated, and recrystallized with ethyl acetate/petroleum ether (1/2, v/v) to obtain a yellow compound **5-5** (257 mg, yield 84%).

**5-5** (257 mg, 0.42 mmol) was dissolved in 15 mL of MeOH and 15 mL of H₂O, and potassium carbonate (290 mg, 2.1 mmol) was added. The mixture was heated and reacted at 80 ° C for 2 hours. After TLC monitoring showed that the reaction was complete, the reaction was extracted twice by adding water and ethyl acetate, washed once with saturated saline, dried over anhydrous sodium sulfate, concentrated, and purified by column chromatography using methanol/dichloromethane (1/30, v/v) to obtain a yellow compound **A99** (206 mg, yield 95%). **¹H NMR** (600 MHz, CDCl₃) δ 8.75 (s, 1H), 7.44 (dd, *J =* 8.3, 5.4 Hz, 2H), 7.28 (dd, *J =* 9.6, 2.5 Hz, 1H), 7.22 (dd, *J =* 8.8, 4.3 Hz, 1H), 7.10 (t, *J =* 8.6 Hz, 2H), 7.04 (s, 1H), 6.96 (s, 1H), 6.73 (d, *J =* 6.2 Hz, 2H), 5.05 (s, 2H), 4.02 (dd, *J =* 8.9, 3.5 Hz, 1H), 3.09 - 2.78 (m, 6H), 2.72 (dt, *J =* 16.2, 5.7 Hz, 1H), 2.27 - 2.09 (m, 2H). ESI/MS *m*/*z* 517.2 [M + H]⁺.

### Example 102: 1- (2- (5-fluoro-1H-indol-3-yl) ethyl) -6- ((4-fluorobenzyl) oxo) -2-methyl-7- (2,2,2-trifluoroethoxy) -1,2,3,4-tetrahydroisoquinoline (A100)

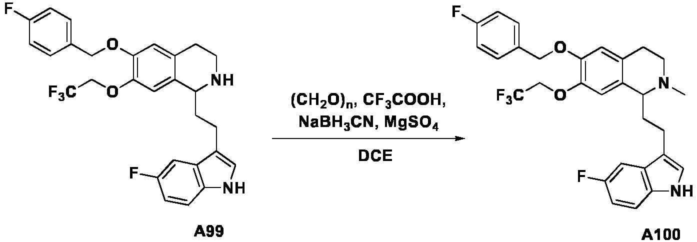

Using compound **A99** as a synthetic intermediate. **A99** (103 mg, 0.2 mmol) was dissolved in 15 mL of 1,2-dichloroethane. Polyformaldehyde (12 mg, 0.4 mmol), sodium cyanoborohydride (24 mg, 0.4 mmol), anhydrous magnesium sulfate (24 mg, 0.2 mmol), and trifluoroacetic acid (9 mg, 0.1 mmol) were sequentially added. The reaction was heated at 60 ° C for 2 hours. After TLC monitoring showed that the reaction was complete, the reaction was extracted twice by adding water and dichloromethane, washed once with saturated salt water, dried over anhydrous sodium sulfate, concentrated, and subjected to column chromatography using methanol/dichloromethane (1/30, v/v) to obtain a yellow compound **A100** (69 mg, yield 65%). **¹H NMR** (600 MHz, CDCl₃) δ 9.01 (s, 1H), 7.52 (dd, *J* = 8.2, 5.4 Hz, 2H), 7.39 (dd, *J =* 9.6, 2.5 Hz, 1H), 7.31 (dd, *J =* 8.9, 4.3 Hz, 1H), 7.26 (t, *J =* 8.7 Hz, 2H), 7.17 (s, 1H), 6.99 (s, 1H), 6.80 (d, *J =* 6.1 Hz, 2H), 5.17 (s, 2H), 4.43 - 4.08 (m, 1H), 3.31 (s, 3H), 3.09 - 2.78 (m, 6H), 2.81 - 2.60 (m, 1H), 2.27 - 2.09 (m, 2H). ESI/MS *m*/*z* 531.2 [M + H]⁺.

### Example 103: 1- (2- (1H-indol-3-yl) ethyl) -6- ((4-fluorobenzyl) oxo) -7-methoxy-2-((tetrahydro-2H-pyran-4-yl) methyl) -1,2,3,4-tetrahydroisoquinoline (A101)

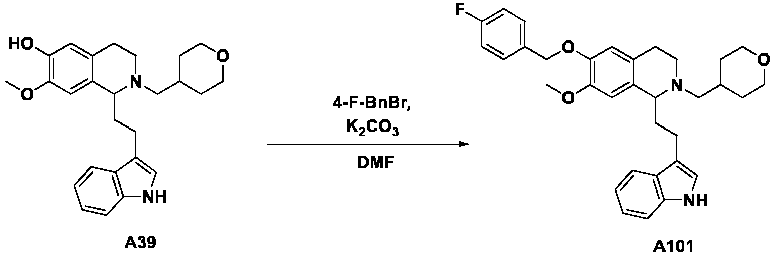

Using compound **A39** as a synthetic intermediate. **A39** (43 mg, 0.1 mmol) was dissolved in 5 mL of DMF, and 4-fluorobenzyl bromide (23 mg, 0.12 mmol), and potassium carbonate (69 mg, 0.5 mmol) were sequentially added. The reaction was heated at 60 ° C for 2 hours. After TLC monitoring showed that the reaction was complete, the reaction was extracted twice by adding water and dichloromethane, washed once with saturated salt water, dried over anhydrous sodium sulfate, concentrated, and recrystallizated using ethyl acetate/petroleum ether (1/2, v/v) to obtain a light yellow compound **A101** (46 mg, yield 87%). ESI/MS *m*/*z* 529.3 [M + H]⁺.

### Example 104: 1- (2- (5-fluoro-1H-indol-3-yl) ethyl) -6- ((4-fluorobenzyl) oxo) -7-methoxy-2- ((tetrahydro-2H-pyran-4-yl) methyl) -1,2,3,4-tetrahydroisoquinoline (A102)

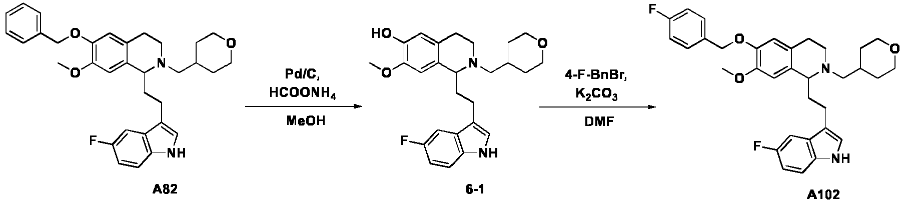

Using compound **A82** as a synthetic intermediate, **A82** (106 mg, 0.2 mmol) was dissolved in 10 mL of methonal, and 11 mg of palladium carbon (containing 10% water) and ammonium formate (126 mg, 2 mmol) were added. The reaction was heated at 60 ° C for 4 hours under argon protection. After TLC monitoring showed that the reaction was complete, the reaction was filtered to remove palladium carbon, extracted twice by adding water and dichloromethane, washed once with saturated salt water, dried over anhydrous sodium sulfate, and concentrated to obtain intermediate **6-1** which was directly subjected to the next step without passing through a column. Intermediate **6-1** was dissolved in 5 mL DMF, and 4-fluorobenzyl bromide (212 mg, 0.24 mmol) and potassium carbonate (138 mg, 1.0 mmol) were added. The mixture was heated and reacted at 60 ° C for 2 hours. After TLC monitoring showed that the reaction was complete, the reaction was extracted twice by adding water and ethyl acetate, washed once with saturated saline, dried over anhydrous sodium sulfate, concentrated, and recrystallizated with ethyl acetate/petroleum ether (1/2, v/v) to obtain a light yellow compound **A102** (67 mg, yield 61%). ESI/MS *m*/*z* 547.2 [M + H]⁺.

### Examples of Pharmacological Activity and Pharmacokinetic Experiments

### Example 1: Promoting TFEB Nuclear Translocation Activity Test Experiment

The experimental steps were as follows: 1. Cultivation of Hela cells. This experiment requires the cultivation of HeLa cell line which can stably express TFEB-EGFP fluorescent protein, and can be used to screen compounds activity. 2. Screening of small molecule compounds that induce TFEB-EGFP nuclear translocation and lysosomal generation. When the density of TFEB-EGFP stable cell line cells in a 96 well plate is about 85%, it was treated with compounds with concentrations of 30 µM and 50 µM or Torin 1 (1 µ M). After treating for 3 hours, 6 hours, and 9 hours, the nuclear translocation of TFEB was observed and the nuclear translocation rate was counted using a fluorescence microscope (Nikon). During this process, the experimenter conducted a double-blind screening.

The experimental results were shown in Table 1.

**Table 1: Activity Data List of Some Compounds^{a,b}**

| Compound number | Percentages of cells with nuclear TFEB (%) | | | | | |
|---|---|---|---|---|---|---|
| | 30 µM | | | 50 µM | | |
| | 3 h | 6 h | 9 h | 3 h | 6h | 9 h |
| **A1** | 65 | 86 | 90 | 83 | 98 | 100 |
| **(*S*)-A1** | 65 | 86 | 90 | 83 | 98 | 100 |
| **(*R*)-A1** | 65 | 86 | 90 | 83 | 98 | 100 |
| **A2** | 62 | 69 | 68 | / | / | / |
| **A3** | 100 | / | / | / | / | / |
| **A4** | 100 | / | / | / | / | / |
| **A5** | 93 | / | / | / | / | / |
| **A13** | 77 | 84 | 90 | 86 | 93 | 98 |
| **A15** | 96 | 100 | / | 100 | 100 | / |
| **A16** | 81 | / | / | / | / | / |
| **A18** | 65 | 80 | / | 90 | 100 | / |
| **A24** | 80 | 90 | / | 95 | 95 | / |
| **A26** | 81 | 85 | 89 | 100 | 100 | / |
| **(*S*)-A26** | 78 | 88 | / | / | / | / |
| **(*R*)-A26** | 76 | 88 | / | / | / | / |
| **A27** | 70 | 70 | / | 90 | 90 | / |
| **A29** | 94 | 95 | / | / | / | / |
| **A30** | 61 | / | / | / | / | / |
| **A55** | 88 | / | / | / | / | / |
| **A56** | 96 | 98 | 100 | / | / | / |
| **A57** | 96 | 97 | 100 | / | / | / |
| **A58** | 95 | / | / | / | / | / |
| **A59** | 83 | / | / | / | / | / |
| **A60** | 92 | / | / | / | / | / |
| **A61** | 95 | / | / | / | / | / |
| **A62** | 83 | / | / | / | / | / |
| **A63** | 93 | / | / | / | / | / |
| **A64** | 84 | / | / | / | / | / |
| **A65** | 75 | / | / | / | / | / |
| **A66** | 80 | / | / | / | / | / |
| **A68** | 72 | / | / | / | / | / |
| **A78** | 70 | 70 | / | 80 | 90 | / |
| **A81** | 77 | / | / | / | / | / |
| **A82** | 83 | / | / | / | / | / |
| **A83** | 82 | / | / | / | / | / |
| **A84** | 100 | / | / | / | / | / |
| **A85** | 94 | / | / | / | / | / |
| **A86** | 100 | / | / | / | / | / |
| **A88** | 83 | / | / | / | / | / |
| **A89** | 82 | / | / | / | / | / |
| **A90** | 93 | / | / | / | / | / |
| **A95** | 70 | / | / | / | / | / |
| **A98** | 90 | / | / | / | / | / |
| **A99** | 94 | / | / | / | / | / |
| **A100** | 100 | / | / | / | / | / |
| **A101** | 100 | / | | / | / | / |
| Torin 1 | 100 | 100 | 100 | 100 | 100 | 100 |
| DMSO | 0 | 0 | 0 | 0 | 0 | 0 |

*^{a}*Torin 1 (1 µM) was the positive control group, and DMSO was the blank control group. *^{b} "*/*"* indicates that the compound has already achieved a high TFEB nuclear translocation rate under the previous conditions, so there is no need to evaluate the current conditions again.

**Experimental conclusion:** From the above experimental results, it can be seen that multiple compounds have significant induction effects on TFEB nuclear translocation in cells. Compounds **A16, A24, A26, A55, A59, A62, A64, A66, A82, A83, A88, and A89** promote TFEB nuclear translocation reach a rate of 80% to 90% at a concentration of 30 µM and a time point of 3 hours. Compounds **A3, A4, A5, A15, A29, A56, A57, A58, A60, A61, A63, A84, A85, A86, A90, A98, A99, A100, and A101** promote TFEB nuclear translocation to a rate of 90% to 100% at a concentration of 30 µM and a time point of 3 hours. These compounds have the potential to induce lysosomal biosynthesis and activate the autophagy-lysosome pathway to degrade pathological protein deposits in the brain, providing a good choice for the development of therapeutic drugs for neurodegenerative diseases such as Alzheimer's and Parkinson's disease.

### Example 2: Tissue distribution data of compound A26 in mice

The drug concentrations in plasma or cerebrospinal fluid at two time points of 15 minutes and 30 minutes under three administration routes: oral (20 mg/kg), intraperitoneal injection (10 mg/kg), and intravenous injection (5 mg/kg) were measured. The experimental results were shown in Table 2.

**Table 2 Tissue distribution data of compound A26 in mice^{a}**

| Administration mode | point of time (min) | Cardiac plasma (ng/mL) | cerebrospinal fluid (ng/g) | Brain to plasma drug concentration ratio ( C_{brain}/Cₚₗₐₛₘₐ) |
|---|---|---|---|---|
| PO | 15 | 65.3 ± 94.7 | 224 ± 363 | 3.4 |
| 20 mg/kg | 30 | 20.7 ± 6.1 | 32.4 ± 11.9 | 1.6 |
| IP | 15 | 313 ± 15 | 665 ± 49 | 2.12 |
| 10 mg/kg | 30 | 186 ± 57 | 355 ± 106 | 1.91 |
| IV | 15 | 466 ± 85 | 1562 ± 245 | 3.3 |
| 5 mg/kg | 30 | 135 ± 47 | 292 ± 69 | 2.2 |

| | | | | |
|---|---|---|---|---|
| *^{a}* Each dose averaged 3 mice | | | | |

**Experimental conclusion:** At the time point of 15min, the optimal compound **A26** achieved drug concentration ratios of 3.4 times, 2.12 times, and 3.3 times in the brain and plasma through oral, intraperitoneal, and intravenous administration, respectively. This indicates that compound **A26** has a good blood-brain barrier penetration ability and brain targeting property, which demonstrates its advantages in developing drugs for treating brain diseases.

All documents mentioned in the present invention are cited as references in this application, just as each document is individually cited as a reference. In addition, it should be understood that, after reading the above teaching content of the present invention, those skilled in the art can make various changes or modifications to the present invention, and these equivalent forms also fall within the scope defined by the appended claims of the present application.

## Claims

1. A compound having a structure shown in formula (I), and a racemate, an R-isomer, an S-isomer, or a pharmaceutically acceptable salt thereof, 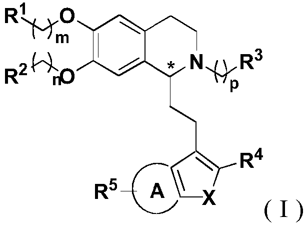 wherein,
X is selected from the group consisting of: O, S, or NR⁶;
m, n and p are each independently selected from the group consisting of: 0, 1, 2, 3, or 4;
the configuration of C* can independently be S configuration, R configuration, or racemate;
R¹ and R² are each independently selected from the group consisting of: hydrogen, deuterium, tritium, halogen, cyano, nitro, hydroxyl, sulfydryl, carboxyl, -S(O)₂OH, substituted or unsubstituted C₁-C₁₀ linear or branched alkyl, substituted or unsubstituted C₁-C₁₀ alkoxy, C₂-C₆ linear or branched alkenyl, substituted or unsubstituted C₂-C₆ alkynyl, substituted or unsubstituted saturated or partially unsaturated C₃-C₁₀ carbocyclyl, substituted or unsubstituted C₆-C₁₀ aryl, substituted or unsubstituted saturated or partially unsaturated 5-12 membered heterocyclyl, substituted or unsubstituted 5-12 membered heteroaryl, substituted or unsubstituted C₂-C₆ acyl, substituted or unsubstituted C₂-C₆ ester group, substituted or unsubstituted C₀-C₆ amino, substituted or unsubstituted C₁-C₆ amido, substituted or unsubstituted C₁-C₆ alkyl-sulfonyl, or substituted or unsubstituted C₁-C₆ alkyl-sulfinyl;
or, R¹ and R² together with the atoms to which they are attached form a substituted or unsubstituted 5-12 membered heterocyclic ring or heteroaromatic ring;
R³ is selected from the group consisting of: hydrogen, substituted or unsubstituted (-C₁-C₆ alkyl - C₆-C₁₀ aryl), substituted or unsubstituted (- C₁-C₆ alkyl - 5-12 membered heteroaryl), substituted or unsubstituted (- C₁-C₆ alkyl - C₃-C₈ carbocyclyl), substituted or unsubstituted (- C₁-C₆ alkyl - 3-12 membered heterocyclyl), substituted or unsubstituted (-C₁-C₆ alkyl - 7-20 membered heteropolycyclyl), substituted or unsubstituted C₃-C₈ saturated or partially unsaturated carbocyclyl, substituted or unsubstituted C₆-C₁₀ aryl, substituted or unsubstituted 3-12 membered heterocyclyl, and substituted or unsubstituted 7-20 membered heteropolycyclyl; wherein, the heteropolycyclyl includes fused ring, bridged ring, and spiro ring structures; 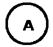
ring is selected from the group consisting of: substituted or unsubstituted 3-12 membered heterocyclyl, substituted or unsubstituted C₆-C₁₀ aryl, and substituted or unsubstituted 5-12 membered heteroaryl;
R⁴ and R⁵ are each independently selected from the group consisting of: hydrogen, deuterium, tritium, halogen, cyano, nitro, amino, C₁-C₆ amino, hydroxyl, hydroxymethyl, carboxyl, C₁-C₆ amido, sulfydryl, -S(O)₂OH, C₁-C₆ alkylsulfonyl, substituted or unsubstituted C₁-C₆ alkyl, substituted or unsubstituted C₁-C₆ alkoxy, substituted or unsubstituted C₃-C₈ cycloalkyl, substituted or unsubstituted C₆-C₁₀ aryl, and substituted or unsubstituted 3-12 membered saturated or partially unsaturated heterocyclyl;
R⁶ is selected from the group consisting of: hydrogen, C₁-C₆ alkylsulfonyl, C₁-C₆ alkyl, C₁-C₆ haloalkyl, C₁-C₆ alkyl substituted with aryl or heteroaryl, C₁-C₆ alkyl substituted with cycloalkane or heterocycloalkane, C₃-C₈ saturated or partially unsaturated carbocyclyl, C₃-C₈ halogenated saturated or partially unsaturated carbocyclyl, C₆-C₁₀ aryl, 3-12 membered heterocyclyl, and -(CH₂)_{q}YR⁷;
wherein, q is selected from the group consisting of: 0, 1, 2, 3, or 4;
Y is selected from the group consisting of: O, S, NR⁸, CO, or SO₂;
R⁷ and R⁸ are each independently selected from the group consisting of: hydrogen, hydroxyl, amino, C₁-C₆ amino, substituted or unsubstituted C₁-C₆ alkoxy, C₁-C₆ alkyl, C₁-C₆ haloalkyl, - C₁-C₆ alkyl - C₆-₁₀ aryl, - C₁-C₆ alkyl - 5-12 membered heteroaryl, - C₁-C₆ alkyl - C₃-₈ carbocyclyl, - C₁-C₆ alkyl - 3-12 membered heterocyclyl, C₃-C₈ saturated or partially unsaturated carbocyclyl, C₃-C₈ halogenated saturated or partially unsaturated carbocyclyl, C₆-C₁₀ aryl, 5-12 membered heteroaryl, and 3-12 membered saturated or partially unsaturated heterocyclyl;
wherein, the heteroaryl or heterocyclyl each independently contains 1 to 4 heteroatoms selected from oxygen, sulfur, and nitrogen;
the substituted in substituted or unsubstituted refers to, the group is substituted with 1 to 3 substituents selected from the group consisting of: halogen, cyano, nitro, amino, hydroxyl, hydroxymethyl, carboxyl, sulfydryl, C₁-C₆ alkyl, C₁-C₆ haloalkyl, C₁-C₆ alkoxy, C₁-C₆ alkoxycarbonyl, C₁-C₆ haloalkoxy, C₂-C₆ alkenyl, C₂-C₆ alkynyl, C₁-C₆ alkylsulfonyl, C₃-C₈ saturated or partially unsaturated carbocyclyl, C₆-C₁₀ aryl, 3-12 membered saturated or partially unsaturated heterocyclyl, and 5-12 membered heteroaryl;
the halogen is F, Cl, Br or I.
In another preferred embodiment, R⁴ and R⁵ are each independently selected from the group consisting of: hydrogen, deuterium, tritium, halogen, cyano, nitro, amino, NH₂, C₁-C₆ amino, hydroxyl, hydroxymethyl, carboxyl, C₂-C₆ amide, sulfydryl, -S(O)₂OH, C₁-C₆ alkylsulfonyl, C₁-C₆ alkyl, C₁-C₆ alkyl substituted with halogen, C₁-C₆ alkyl substituted with aryl or heteroaryl, C₁-C₆ alkyl substituted with cycloalkane or heterocycloalkane, C₁-C₆ alkoxy, C₁-C₆ alkoxy substituted with aryl or heteroaryl, C₁-C₆ haloalkoxy, C₃-C₈ cycloalkyl, C₃-C₈ halogenated cycloalkyl, C₆-C₁₀ aryl, 3-12 membered saturated or partially unsaturated heterocyclyl.

2. The compound according to claim 1, and the racemate, the R-isomer, the S-isomer, or the pharmaceutically acceptable salt thereof, wherein, R¹ and R² are independently selected from the group consisting of: substituted or unsubstituted C₁-C₁₀ linear or branched alkyl, substituted or unsubstituted saturated or partially unsaturated C₃-C₁₀ carbocyclyl, and substituted or unsubstituted C₅-C₁₀ aryl.
In another preferred embodiment, R¹ and R² are independently selected from the group consisting of: substituted or unsubstituted C₁-C₆ linear or branched alkyl, substituted or unsubstituted saturated or partially unsaturated C₃-C₆ carbocyclyl, and substituted or unsubstituted phenyl.

3. The compound according to claim 1, and the racemate, the R-isomer, the S-isomer, or the pharmaceutically acceptable salt thereof, wherein, R¹ and R² together with the atoms to which they are attached form a substituted or unsubstituted 5-12 membered heterocyclyl.
In another preferred embodiment, R¹ and R² together with the atoms to which they are attached form a substituted or unsubstituted 5-7 membered heterocyclyl.

4. The compound according to claim 1, and the racemate, the R-isomer, the S-isomer, or the pharmaceutically acceptable salt thereof, wherein, R³ is selected from the group consisting of: hydrogen, substituted or unsubstituted C₆-C₁₀ aryl, substituted or unsubstituted 3-12 membered heterocyclyl, or substituted or unsubstituted 7-20 membered heteropolycyclyl; wherein, the heteropolycyclyl includes fused ring, bridged ring, and spiro ring structures.

5. The compound according to claim 1, and the racemate, the R-isomer, the S-isomer, or the pharmaceutically acceptable salt thereof, wherein, 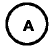 ring is selected from the group consisting of: benzene ring, thiophene ring, pyrrole ring, furan ring, pyridine ring, pyrimidine ring, or cyclohexyl.

6. The compound according to claim 1, and the racemate, the R-isomer, the S-isomer, or the pharmaceutically acceptable salt thereof, wherein, the compound is selected from the group consisting of:
| Compound Number | Structural Formula | Compound Number | Structural Formula |
|---|---|---|---|
| **A1** | 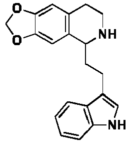 | **(*S*)-A1** | 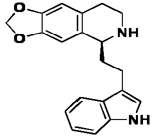 |
| **(*R*)-A1** | 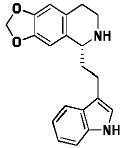 | **A2** | 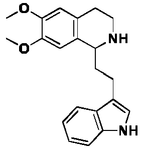 |
| **(*S*)-A2** | 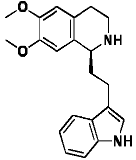 | ***(R)-A2*** | 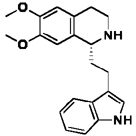 |
| **A3** | 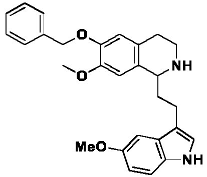 | **A4** | 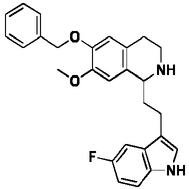 |
| **A5** | 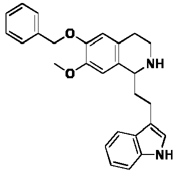 | **A12** | 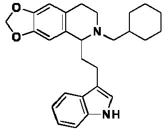 |
| **(*S*)-A12** | 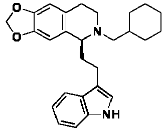 | **(*R*)-A12** | 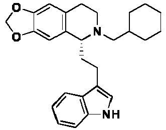 |
| **A13** | 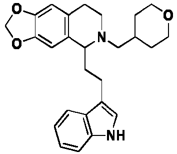 | **(*S*)-A13** | 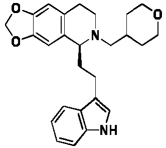 |
| **(*R*)-A13** | 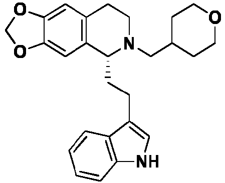 | **A14** | 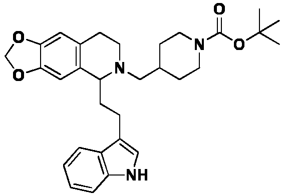 |
| **(*S*)-A14** | 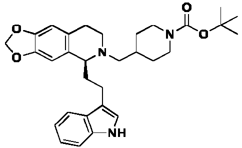 | **(*R*)-A14** | 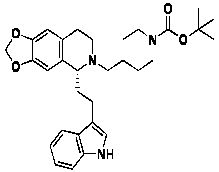 |
| **A15** | 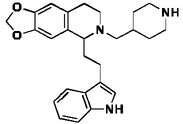 | **(*S*)-A15** | 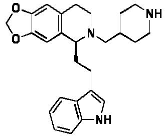 |
| **(*R*)-A15** | 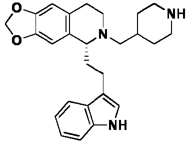 | **A16** | 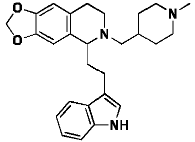 |
| **A17** | 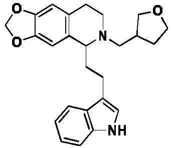 | **A18** | 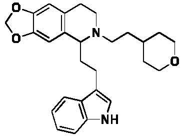 |
| **A23** | 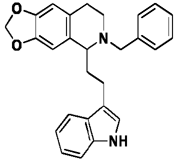 | **A24** | 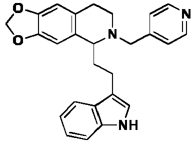 |
| **A25** | 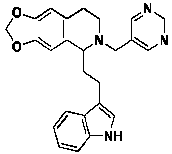 | **A26** | 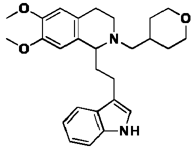 |
| **A27** | 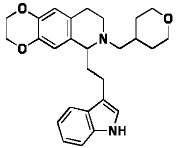 | **A28** | 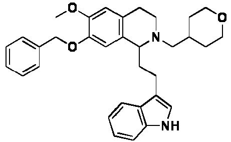 |
| **A29** | 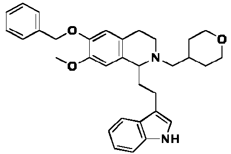 | **A30** | 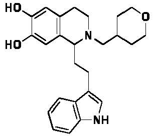 |
| **A31** | 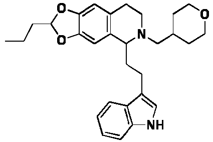 | **A32** | 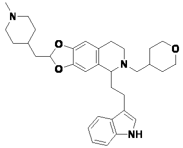 |
| **A33** | 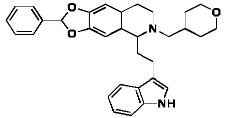 | **A34** | 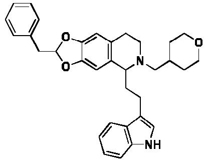 |
| **A35** | 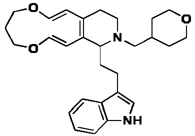 | **A36** | 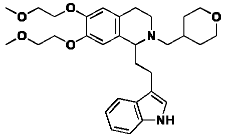 |
| **A37** | 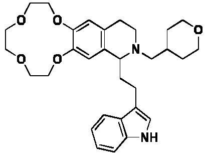 | **A38** | 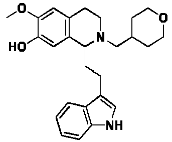 |
| **A39** | 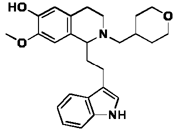 | **A40** | 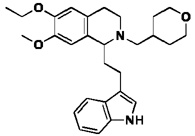 |
| **A41** | 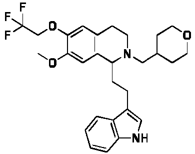 | **A42** | 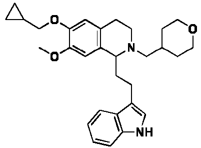 |
| **A43** | 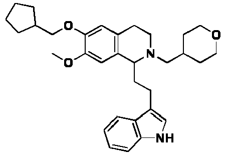 | **A44** | 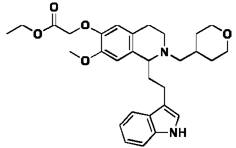 |
| **A45** | 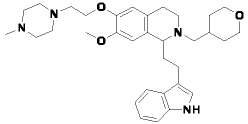 | **A46** | 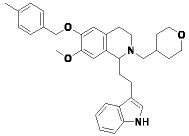 |
| **A47** | 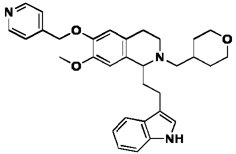 | **A48** | 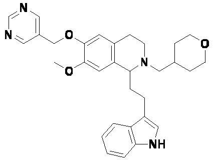 |
| **A49** | 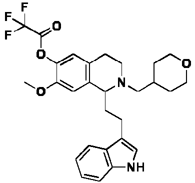 | **A50** | 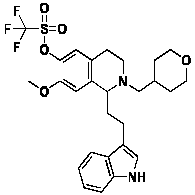 |
| **A51** | 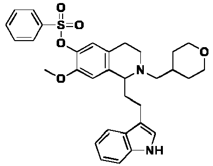 | **A52** | 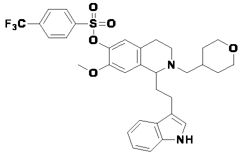 |
| **A53** | 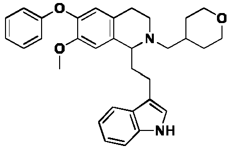 | **A54** | 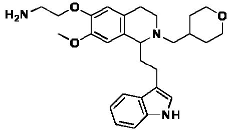 |
| **A55** | 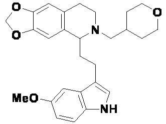 | **A56** | 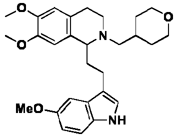 |
| **A57** | 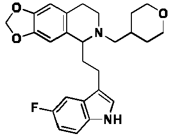 | **A58** | 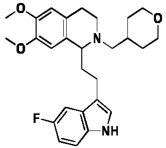 |
| **A59** | 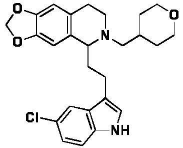 | **A60** | 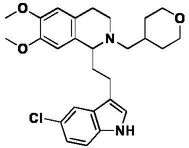 |
| **A61** | 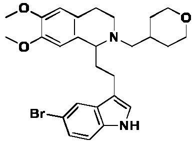 | **A62** | 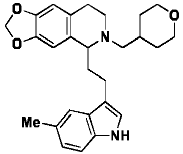 |
| **A63** | 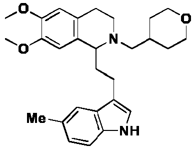 | **A64** | 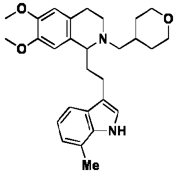 |
| **A65** | 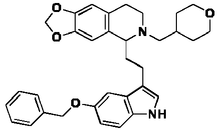 | **A66** | 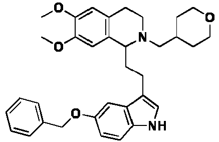 |
| **A67** | 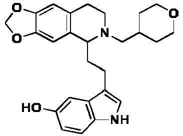 | **A68** | 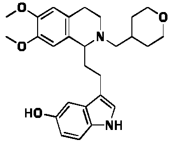 |
| **A69** | 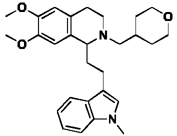 | **A70** | 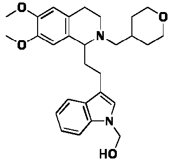 |
| **A71** | 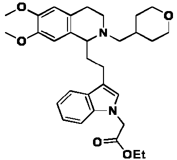 | **A72** | 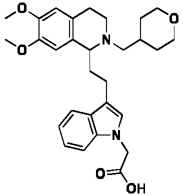 |
| **A73** | 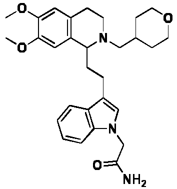 | **A74** | 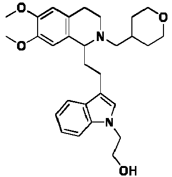 |
| **A75** | 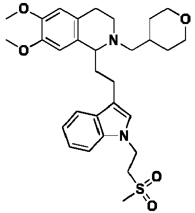 | **A76** | 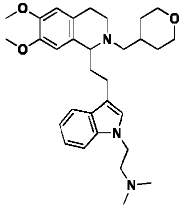 |
| **A77** | 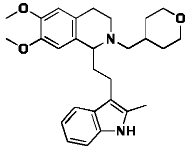 | **A78** | 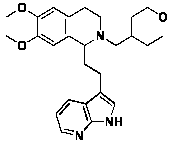 |
| **A79** | 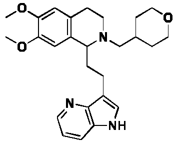 | **A80** | 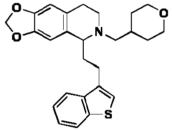 |
| **A81** | 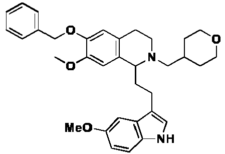 | **A82** | 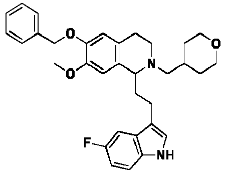 |
| **A83** | 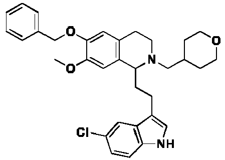 | **A84** | 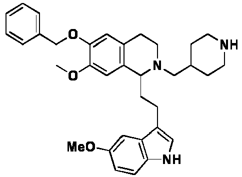 |
| **A85** | 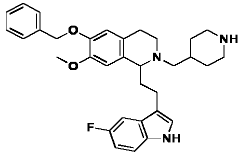 | **A86** | 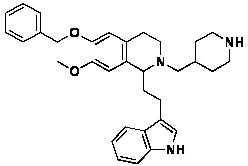 |
| **A87** | 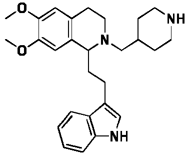 | **A88** | 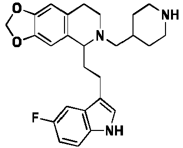 |
| **A89** | 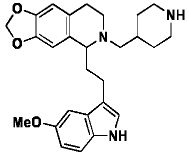 | **A90** | 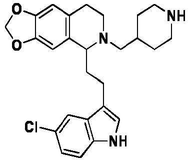 |
| **A91** | 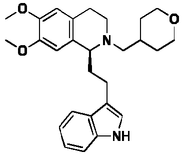 | **A92** | 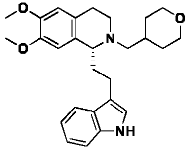 |
| **A93** | 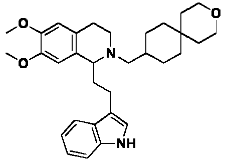 | **A94** | 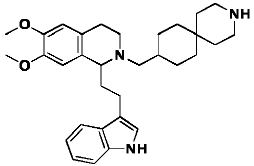 |
| **A95** | 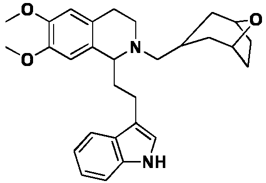 | **A96** | 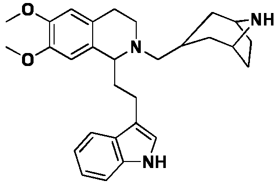 |
| **A97** | 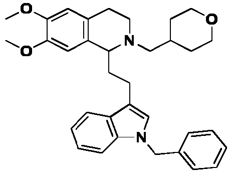 | **A98** | 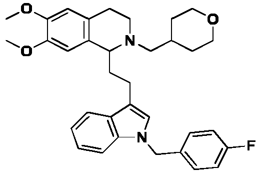 |
| **A99** | 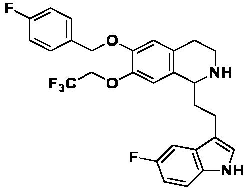 | **A100** | 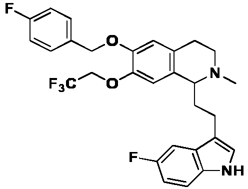 |
| **A101** | 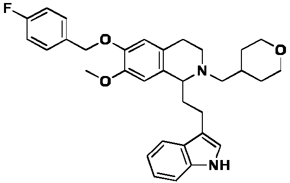 | **A102** | 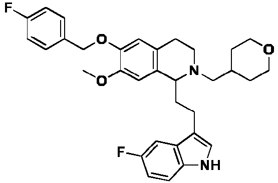 |

7. A preparation method for a compound shown in formula (I), comprising steps of:
(1) in a mixed solvent of nitromethane and organic acid, subjecting a compound of formula IIₐ to a Henry reaction with nitromethane to obtain an α,β-unsaturated nitro compound II_{b}, then subjecting the compound of formula II_{b} to a reduction reaction to obtain the compound of formula II_{c}; 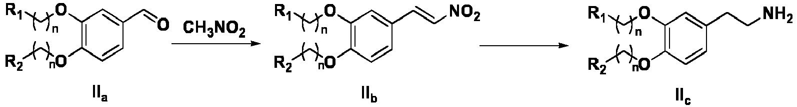
(2) in an basic solvent, in the presence of malonic acid and a base catalyst, subjecting a compound of formula Iₐ to Knoevenagel reaction with malonic acid to obtain a compound of formula I_{b}, then reducing the compound of formula I_{b} by a reducing agent to obtain a compound of formula I_{c}; 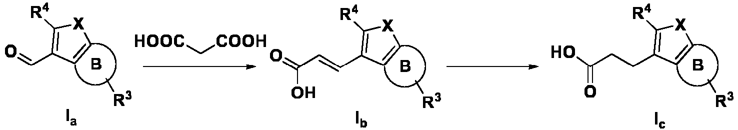
(3) in an inert solvent, in the presence of a condensing agent, reacting the compound of formula II_{c} and the compound of formula I_{c} to obtain a compound of formula Iₐ; 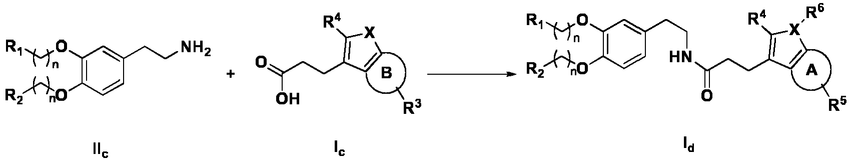
(4) in an inert solvent, performing Bischler Napieralski cyclization reaction using the compound of formula I₄ to obtain a compound of formula Iₑ; 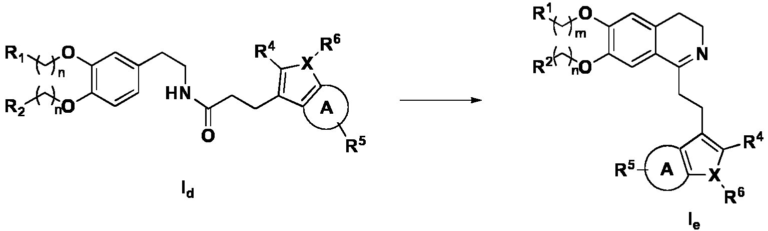
(5) in an inert solvent, performing a reduction reaction using the compound of formula Iₑ to obtain a compound of formula I_{f}; 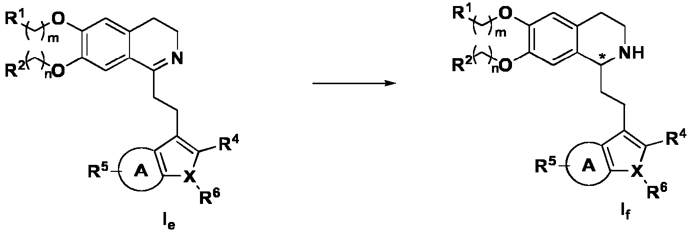
(6) in an inert solvent, performing a substitution reaction using the compound of formula I_{f} and halogenated hydrocarbon I_{g} to obtain a compound of formula (I); 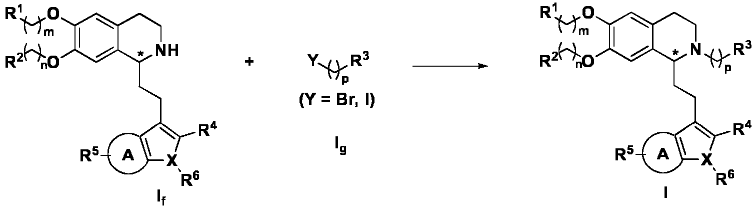 wherein, R¹, R², R³, R⁴, R⁵, R⁶, X, m, n, and p are as defined in claim 1.

8. The preparation method according to claim 7, wherein, in step (1), the organic acid is acetic acid or formic acid, and the reducing agent is LiAlH₄, sodium borohydride, or zinc powder.
In another preferred embodiment, in step (2), the basic solvent is pyridine, the base catalyst is piperidine, and the reducing agent is a combination of palladium on carbon hydrogenation catalyst and hydrogen gas, or a combination of palladium on carbon hydrogenation catalyst and ammonium formate.
In another preferred embodiment, in step (3), the condensing agent is HATU or a combination of EDCI and HOBt.
In another preferred embodiment, in step (4), in the cyclization reaction, POCl₃ phosphorus oxychloride is used as the Lewis acid.
In another preferred embodiment, in step (5), in the reduction reaction, borohydride is used as the reducing agent, or Noyori's catalyst is used as the asymmetric reduction catalyst.
In another preferred embodiment, in step (6), in the substitution reaction, potassium carbonate, cesium carbonate, or sodium carbonate is used as the base for catalyzing reaction.

9. A pharmaceutical composition comprising (1) the compound according to any one of claims 1-6, the racemate, the R-isomer, the S-isomer, or the pharmaceutically acceptable salt thereof ; and (2) pharmaceutically acceptable carriers.

10. The use of the compound according to any one of claims 1-6, the racemate, the R-isomer, the S-isomer, or the pharmaceutically acceptable salt thereof, or the pharmaceutical composition according to claim 9, in preventing and/or treating diseases related to lysosomal dysfunction and insufficient biosynthesis.
In another preferred embodiment, the disease is a neurodegenerative disease caused by the accumulation of pathological protein.
In another preferred embodiment, the disease is selected from the group consisting of: Alzheimer's disease and Parkinson's disease.
